# EUROPEAN PATENT SPECIFICATION

(11) **EP 3 942 038 B1**
(45) Date of publication and mention of the grant of the patent: **30.09.2026**
(21) Application number: 20773398.1
(22) Date of filing: 16.03.2020
(51) Int. Cl.: A61K 31/473, A61K 31/4738, A61K 31/4745, C12N 15/10, C12N 15/90, G01N 33/542, A61P 11/00, A61P 25/28, C07D 401/12, C07D 405/12, C07D 405/04, C07D 409/04, G01N 33/50, G01N 33/68, C07D 221/18, C07D 471/04

(54) **MODULATORS OF TDP-43**
MODULATOREN VON TDP-43
MODULATEURS DE TDP-43

(30) Priority: 18.03.2019 US 201962820158 P
(43) Date of publication of application: 26.01.2022
(73) Proprietor: Alteron Therapeutics, Inc., New York, NY 10019 (US)
(72) Inventor: ZHANG, Chengliang, New York, New York 10019 (US); ZHANG, Xinchun, New York, New York 10019 (US)
(74) Representative: Zacco Sweden AB
(86) International application number: PCT/US2020/022972
(87) International publication number: WO 2020/190866

(56) References cited:
- WO-A1-2010/022140
- WO-A1-2016/090317
- WO-A1-2016/191520
- WO-A2-2010/111504
- US-A1- 2009 118 135
- US-A1- 2013 252 983
- US-A1- 2013 252 983
- US-A1- 2015 065 486
- CHANG CHENG-FU ET AL: "Therapeutic effect of berberine on TDP-43-related pathogenesis in FTLD and ALS", vol. 23, no. 1, 21 October 2016 (2016-10-21), XP055978185, Retrieved from the Internet <URL:http://link.springer.com/content/pdf/10.1186/s12929-016-0290-z.pdf> DOI: 10.1186/s12929-016-0290-z
- CHEN XIAOWEI ET AL: "TDP-43 regulates cancer-associated microRNAs", vol. 9, no. 10, 26 September 2017 (2017-09-26), Beijing, CN, pages 848 - 866, XP055978375, ISSN: 1674-800X, Retrieved from the Internet <URL:http://link.springer.com/content/pdf/10.1007/s13238-017-0480-9.pdf> DOI: 10.1007/s13238-017-0480-9
- KE HAO ET AL: "Loss of TDP43 inhibits progression of triple-negative breast cancer in coordination with SRSF3", vol. 115, no. 15, 10 April 2018 (2018-04-10), XP055978379, ISSN: 0027-8424, Retrieved from the Internet <URL:https://www.pnas.org/doi/pdf/10.1073/pnas.1714573115> DOI: 10.1073/pnas.1714573115
- VICTORIA E JOHNSON ET AL: "Acute and chronically increased immunoreactivity to phosphorylation-independent but not pathological TDP-43 after a single traumatic brain injury in humans", ACTA NEUROPATHOLOGICA, SPRINGER, BERLIN, DE, vol. 122, no. 6, 19 November 2011 (2011-11-19), pages 715 - 726, XP019984653, ISSN: 1432-0533, DOI: 10.1007/S00401-011-0909-9
- DATABASE PubChem Compound 15 September 2005 (2005-09-15), "5-(Pyridin-4-yl)-1,2,3,4,5,6-hexahydrobenzo[a]phenanthridine", XP055741647, Database accession no. CID 4543610
- DATABASE PubChem Compound 12 September 2005 (2005-09-12), "2,2-Dimethyl-5-(1H-pyrrol-2-yl)-1,3,5,6-tetrahydrobenzo[a]phenanthridin-4-one", XP055741648, Database accession no. CID 3931339

## Description

### CROSS REFERENCE TO RELATED APPLICATION

This application claims the benefit of U.S. Provisional Application No. 62/820,158, filed March 18, 2019.

### REFERENCE TO SEQUENCE LISTING AND TABLES IN ELECTRONIC FORMAT

This application is filed with an electronic sequence listing entitled ALTER001WOSEQLIST.TXT, created on March 6, 2020 which is 1.9 KB in size.

### BACKGROUND

### Field

The present disclosure relates to compounds and compositions for use in methods of using the same for treating, preventing, inhibiting, ameliorating, or slowing the onset or development of a disease or condition associated with TAR DNA-binding protein 43 (TDP-43) toxicity. Such diseases include cystic fibrosis and neurodegenerative disorders, including, for example amyotrophic lateral sclerosis (ALS), frontotemporal dementia (FTD), Alzheimer's disease, hippocampal sclerosis of aging (HS-Aging), chronic traumatic encephalopathy, and Parkinson's disease.

### Description of the Related Art

TDP-43 also called TARDBP, is a DNA/RNA-binding protein that plays multiple functions with roles in transcriptional regulation (Berson et al., (2017), Curr Biol 27, 3579-3590 e3576; Ignatius et al., 1995, J Virol 69, 3584-3596), pre-mRNA splicing (Ayala et al., (2006), Febs Lett 580, 1339-1344; Bose et al., (2008), J Biol Chem 283, 28852-28859; Buratti and Baralle, (2001a), J Biol Chem 276, 36337-36343; Mercado et al., 2005, Nucleic Acids Res 33, 6000-6010), mRNA stability and transport (Alami et al., 2014, Neuron 81, 536-543; Ayala et al., (2011), Embo Journal 30, 277-288; Strong et al., 2007, Mol Cell Neurosci 35, 320-327), and translational regulation (Neelagandan et al., 2018, Nucleic Acids Res). Further, Chang Cheng-Fu et al., (2016), Journal of Biomedical Science 23:1, XP055978185, discloses berberine for us in the treatment of neurodegenerative diseases, WO2010/022140 discloses pharmaceutical compositions of (R)-pramexipole for use in the treatment of neurodegenerative diseases, WO2016/090317 discloses sulfonamide derivatives useful for the treatment of neurodegenerative diseases. Also, US2013/252983 and WO2010111504 disclose phenanthridinone derivatives for use in the treatment of cancer. WO2016/191520 discloses phenanthridinone derivates for use in the treatment of brain injury. Moreover, US2015/065486 discloses phenanthridinone derivatives for use in the treatment of polycystic kidney disease (PKD).

### SUMMARY

Described herein are compounds for use in treating or reducing onset or development of a disease or condition associated with TDP-43 toxicity. Especially, as claimed in the appended claims 1-15, the disclosure refers to a pharmaceutical composition for use in the treatment of cystic fibrosis or a neurodegenerative disease.

Some embodiments provided herein relate to pharmaceutical compositions. In some embodiments, the composition comprises a therapeutically effective amount of a compound of Formula (I), or a pharmaceutically acceptable salt thereof, for use in the treatment of cystic fibrosis or a neurogenerative disease: 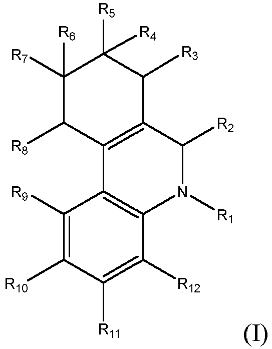 wherein R₁ is H, OH, or lower alkyl; R₂ is an optionally substituted aromatic ring of four, five, or six carbons, wherein the aromatic ring is carbocyclic or heterocyclic, and wherein each position on the aromatic ring is independently H, halo, lower alkyl, OH, lower alkoxy, NH₂, lower alkylamino, di(lower alkyl)amino, SH, lower alkylthio, NO₂, or two residues together form a heterocyclic ring; R₃ and R₈ are each independently H, lower alkyl, =O, =S, OH, NH₂, aryl, or aralkyl, where aryl and aralkyl are substituted with 0-3 moieties selected from the group consisting of halo, OH, NH₂, lower alkyl, lower alkoxy, SH, lower alkylthio, and lower alkylamino; R₄, R₅, R₆, and R₇ are each independently H, lower alkyl, OH, NH₂, aryl, or aralkyl, where aryl and aralkyl are substituted with 0-3 moieties selected from the group consisting of halo, OH, NH₂, lower alkyl, lower alkoxy, SH, lower alkylthio, and lower alkylamino; at least one of R₉ and R₁₀, R₁₀ and R₁₁, or R₁₁ and R₁₂ together form an optionally substituted benzene ring, wherein the benzene ring is optionally substituted with H, halo, lower alkyl, OH, lower alkoxy, or NO₂; and wherein any one of the carbon atoms on any one of fused rings of Formula (I) is optionally replaced with a nitrogen atom. In some embodiments, the compound of Formula (I) is ALT-212, ALT-215, ALT-308, ALT-309, ALT-408, ALT-411, ALT-59, ALT-110, ALT-201, ALT-202, ALT-204, ALT-208, ALT-207, ALT-210, ALT-211, ALT-302, ALT-306, ALT-307, ALT-311, ALT-318, ALT-322, ALT-324, ALT-402, ALT-404, ALT-406, ALT-409, ALT-410, ALT-413, ALT-414, ALT-108, ALT-317, ALT-333, ALT-403, or ALT-205. In some embodiments, compound of Formula (I) is a compound of any one of Formula (II), (III), or (IV): 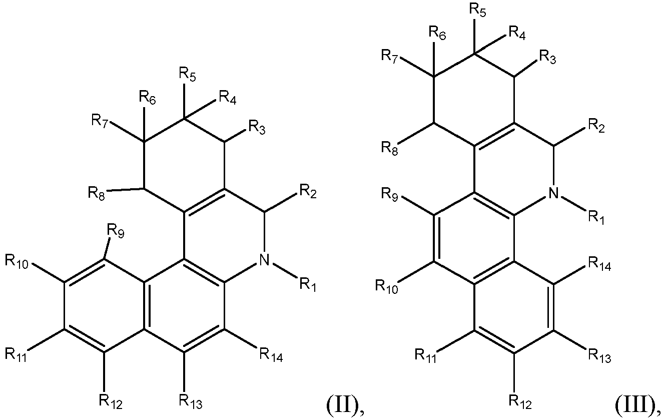 or 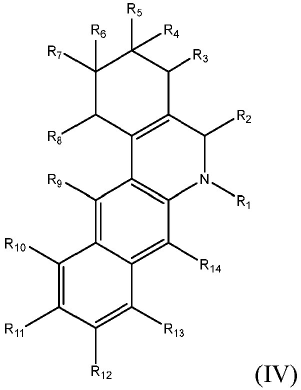 wherein R₁ is H, OH, or lower alkyl; R₂, is an optionally substituted aromatic ring of four, five, or six carbons, wherein the aromatic ring is carbocyclic or heterocyclic, and wherein each position on the aromatic ring is independently H, halo, lower alkyl, OH, lower alkoxy, NH₂, lower alkylamino, di(lower alkyl)amino, SH, lower alkylthio, NO₂, or two residues together form a heterocyclic ring; R₃ and R₈ are each independently H, lower alkyl, =O, =S, OH, NH₂, aryl, or aralkyl, where aryl and aralkyl are substituted with 0-3 moieties selected from the group consisting of halo, OH, NH₂, lower alkyl, lower alkoxy, SH, lower alkylthio, and lower alkylamino; R₄, R₅, R₆, and R₇ are each independently H, lower alkyl, OH, NH₂, aryl, or aralkyl, where aryl and aralkyl are substituted with 0-3 moieties selected from the group consisting of halo, OH, NH₂, lower alkyl, lower alkoxy, SH, lower alkylthio, and lower alkylamino; R₉, R₁₀, R₁₁, R₁₂, R₁₃, and R₁₄ are each independently H, halo, lower alkyl, OH, lower alkoxy, or NO₂; and wherein any one of the carbon atoms on any one of fused rings of Formula (II), (III), or (IV) is optionally replaced with a nitrogen atom. In some embodiments, the compound of Formula (II) is ALT-212, ALT-215, ALT-308, ALT-309, ALT-408, ALT-411, ALT-59, ALT-110, ALT-201, ALT-202, ALT-204, ALT-208, ALT-207, ALT-210, ALT-211, ALT-302, ALT-306, ALT-307, ALT-311, ALT-318, ALT-322, ALT-324, ALT-402, ALT-404, ALT-406, ALT-409, ALT-410, ALT-413, or ALT-414. In some embodiments, the compound of Formula (II) is ALT-59. In some embodiments, the compound of Formula (III) is ALT-108, ALT-317, ALT-333, or ALT-403. In some embodiments, the compound of Formula (IV) is ALT-205. In some embodiments, the pharmaceutical composition is for use in the treatment of cystic fibrosis. In some embodiments, composition inhibits the progression or development of cystic fibrosis. In some embodiments, the pharmaceutical composition is for use in the treatment of a neurodegenerative disease. In some embodiments, the composition inhibits the progression or development of the neurodegenerative disease. In some embodiments, the neurodegenerative disease is amyotrophic lateral sclerosis (ALS), frontotemporal dementia (FTD), Alzheimer's disease, hippocampal sclerosis of aging (HS-Aging), chronic traumatic encephalopathy, or Parkinson's disease. In some embodiments, the compound of Formula (I) is present in an amount of 0.01 mg to 3000 mg. In some embodiments, the composition is formulated for oral or parenteral administration.

In some embodiments, the compound of Formula (I) is a compound of any one of Formula (V), (VI), (VII), or (VIII). Some embodiments provided herein relate to pharmaceutical compositions. In some embodiments, the pharmaceutical composition is for use in the treatment of cystic fibrosis or a neurogenerative disease. In some embodiments, the composition comprises a therapeutically effective amount of a compound of Formula (V), an analogue, derivative, or a pharmaceutically acceptable salt thereof: 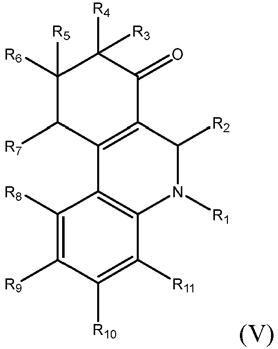 wherein R₁ is H, OH, or lower alkyl; R₂ is an optionally substituted aromatic ring of four, five, or six carbons, wherein the aromatic ring is carbocyclic or heterocyclic, and wherein each position on the aromatic ring is independently H, halo, lower alkyl, OH, lower alkoxy, NH₂, lower alkylamino, di(lower alkyl)amino, SH, lower alkylthio, NO₂, or two residues together form a heterocyclic ring; R₃, R₄, R₅, R₆, and R₇ are each independently H, lower alkyl, OH, NH₂, aryl, or aralkyl, where aryl and aralkyl are substituted with 0-3 moieties selected from the group consisting of halo, OH, NH₂, lower alkyl, lower alkoxy, SH, lower alkylthio, and lower alkylamino; at least one of R₈ and R₉, R₉ and R₁₀, or R₁₀ and R₁₁ together form an optionally substituted benzene ring, wherein the benzene ring is optionally substituted with H, halo, lower alkyl, OH, lower alkoxy, or NO₂; and wherein any one of the carbon atoms on any one of fused rings of Formula (V) is optionally replaced with a nitrogen atom. In some embodiments, the compound of Formula (V) is ALT-212, ALT-215, ALT-308, ALT-309, ALT-408, ALT-411, ALT-59, ALT-110, ALT-201, ALT-202, ALT-204, ALT-208, ALT-207, ALT-210, ALT-211, ALT-302, ALT-306, ALT-307, ALT-311, ALT-318, ALT-322, ALT-324, ALT-402, ALT-404, ALT-406, ALT-409, ALT-410, ALT-413, ALT-414, ALT-108, ALT-317, ALT-333, ALT-403, or ALT-205. In some embodiments, the compound of Formula (V) is ALT-212, ALT-215, ALT-308, ALT-309, ALT-408, ALT-411, ALT-59, ALT-110, ALT-202, ALT-204, ALT-208, ALT-207, ALT-210, ALT-211, ALT-302, ALT-306, ALT-307, ALT-311, ALT-318, ALT-322, ALT-324, ALT-402, ALT-404, ALT-406, ALT-409, ALT-410, ALT-413, ALT-414, ALT-108, ALT-317, ALT-333, ALT-403, or ALT-205. In some embodiments, compound of Formula (V) is a compound of any one of Formula (VI), (VII), or (VIII): 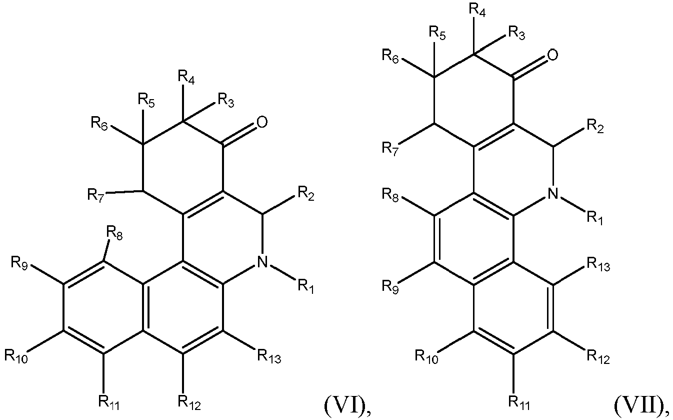 or 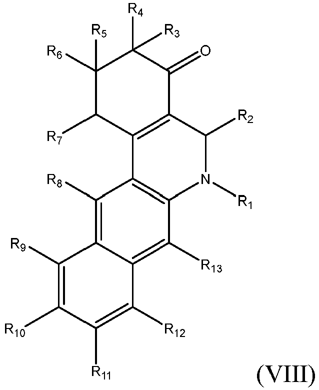 wherein R₁ is H, OH, or lower alkyl; R₂, is an optionally substituted aromatic ring of four, five, or six carbons, wherein the aromatic ring is carbocyclic or heterocyclic, and wherein each position on the aromatic ring is independently H, halo, lower alkyl, OH, lower alkoxy, NH₂, lower alkylamino, di(lower alkyl)amino, SH, lower alkylthio, NO₂, or two residues together form a heterocyclic ring; R₃, R₄, R₅, R₆, and R₇ are each independently H, lower alkyl, OH, NH₂, aryl, or aralkyl, where aryl and aralkyl are substituted with 0-3 moieties selected from the group consisting of halo, OH, NH₂, lower alkyl, lower alkoxy, SH, lower alkylthio, and lower alkylamino; R₈, R₉, R₁₀, R₁₁, R₁₂, and R₁₃ are each independently H, halo, lower alkyl, OH, lower alkoxy, or NO₂; and wherein any one of the carbon atoms on any one of fused rings of Formula (VI), (VII), or (VIII) is optionally replaced with a nitrogen atom. In some embodiments, the compound of Formula (VI) is ALT-212, ALT-215, ALT-308, ALT-309, ALT-408, ALT-411, ALT-59, ALT-110, ALT-201, ALT-202, ALT-204, ALT-208, ALT-207, ALT-210, ALT-211, ALT-302, ALT-306, ALT-307, ALT-311, ALT-318, ALT-322, ALT-324, ALT-402, ALT-404, ALT-406, ALT-409, ALT-410, ALT-413, or ALT-414. In some embodiments, the compound of Formula (VI) is ALT-212, ALT-215, ALT-308, ALT-309, ALT-408, ALT-411, ALT-59, ALT-110, ALT-202, ALT-204, ALT-208, ALT-207, ALT-210, ALT-211, ALT-302, ALT-306, ALT-307, ALT-311, ALT-318, ALT-322, ALT-324, ALT-402, ALT-404, ALT-406, ALT-409, ALT-410, ALT-413, or ALT-414. In some embodiments, the compound of Formula (VI) is ALT-59. In some embodiments, the compound of Formula (VII) is ALT-108, ALT-317, ALT-333, or ALT-403. In some embodiments, the compound of Formula (VIII) is ALT-205. In some embodiments, the pharmaceutical composition is for use in the treatment of cystic fibrosis. In some embodiments, composition inhibits the progression or development of cystic fibrosis. In some embodiments, the pharmaceutical composition is for use in the treatment of a neurodegenerative disease. In some embodiments, the composition inhibits the progression or development of the neurodegenerative disease. In some embodiments, the neurodegenerative disease is amyotrophic lateral sclerosis (ALS), frontotemporal dementia (FTD), Alzheimer's disease, hippocampal sclerosis of aging (HS-Aging), chronic traumatic encephalopathy, or Parkinson's disease. In some embodiments, the compound of Formula (V) is present in an amount of 0.01 mg to 3000 mg. In some embodiments, the composition is formulated for oral or parenteral administration.

### BRIEF DESCRIPTION OF THE DRAWINGS

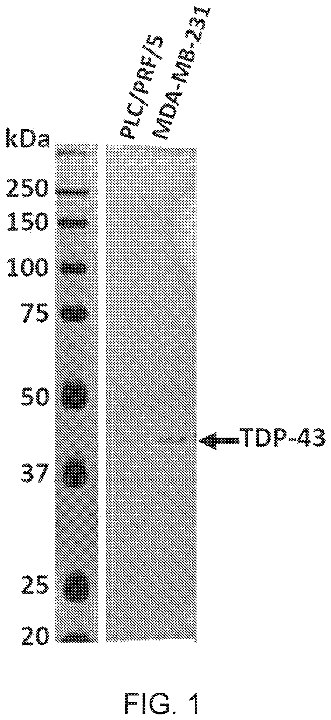

FIG. 1 depicts a blot showing isolation of ALT-59 binding proteins. Solid phase affinity precipitation was performed using cell lysates from PLC/PRF/5 and MDA-MB-231 cells. Precipitated proteins were resolved by sodium dodecyl sulfate polyacrylamide gel electrophoresis (SDS-PAGE) gel and silver stained. A major band with an apparent molecular weight of 43 kDa were visualized from both samples.

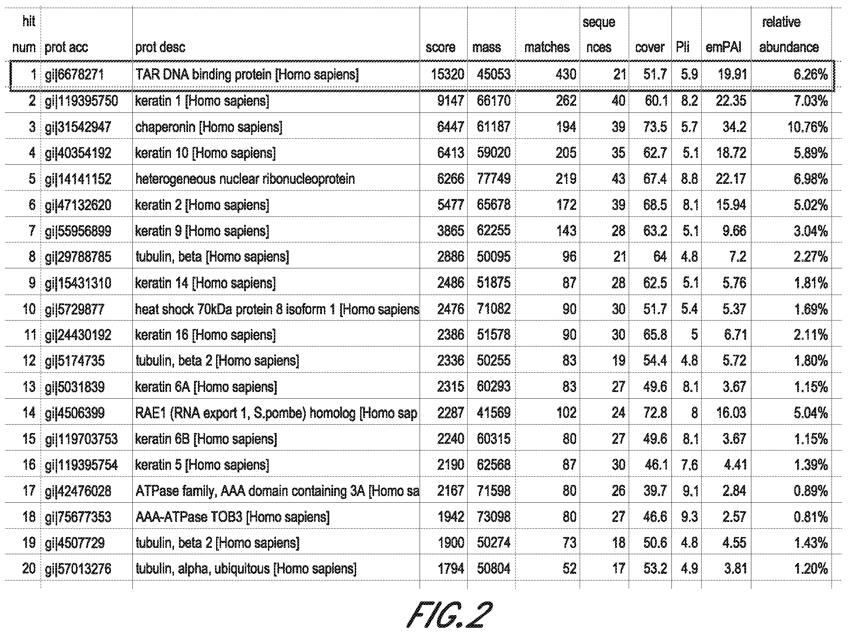

FIG. 2 is a table showing a list of potential ALT-59 binding proteins identified by mass spectrometry. ALT-59 precipitated protein samples were subjected to mass spectrometry for protein identification. TDP-43 (boxed) was ranked as the top hit and is likely the 43 kDa band visualized in FIG. 1.

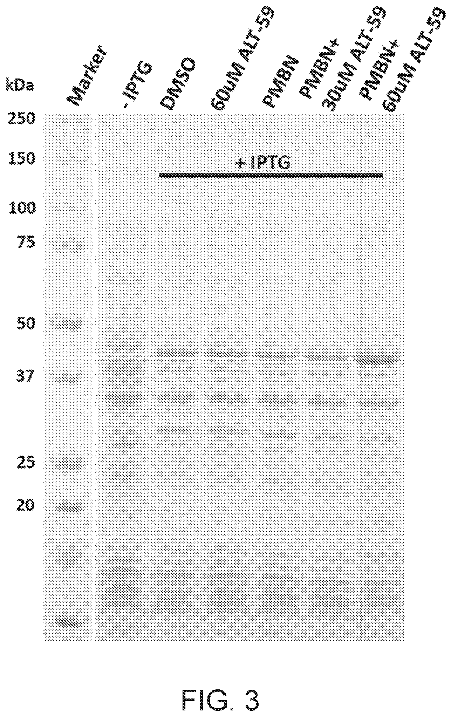

FIG. 3 depicts expression of histidine-tagged TDP-43 in *E*. *coli* whole cell lysates. Transformant Rosetta (DE3) cells with pRETb/TDP43-His were un-induced, induced with isopropyl β-D-1-thiogalactopyranoside (IPTG) alone, or together with ALT-59 (30 or 60 µM) or polymyxin B nonapeptide (PMBN) or both. PMBN increases the permeability of the outer membrane of Gram-negative bacteria for increased entry of ALT-59. In the presence of PMBN, ALT-59 at a concentration of 60 µM increased expression of TDP43-His, suggesting that ALT-59 can reduce toxicity caused by TDP-43 expression in bacteria. However, the protein is expressed at relatively low level and could not be purified due to the aggregation-prone nature of TDP-43.

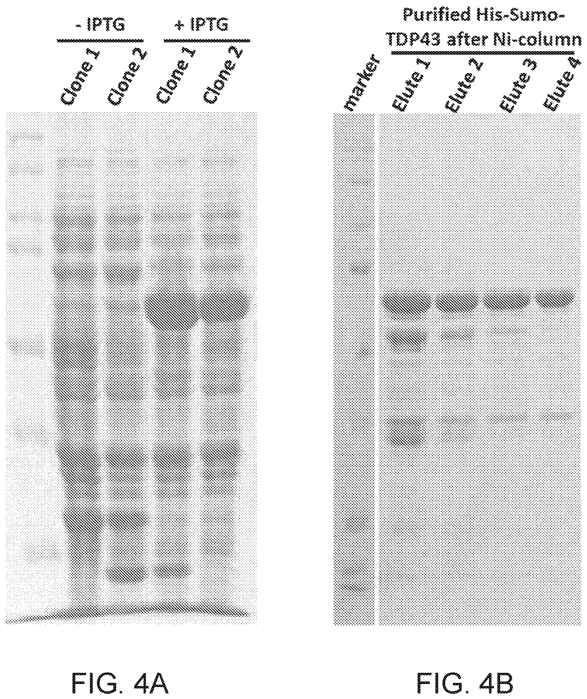

FIGs. 4A-4B depict expression and purification of His-Sumo-TDP43. A Sumo-tag was inserted at the N-terminus of TDP-43 to increase solubility. FIG. 4A shows the protein expression in whole cell lysates from two bacterial clones expressing His-Sumo-TDP43 with or without IPTG induction. FIG. 4B shows the eluted protein after purification using Ni-NTA Agarose.

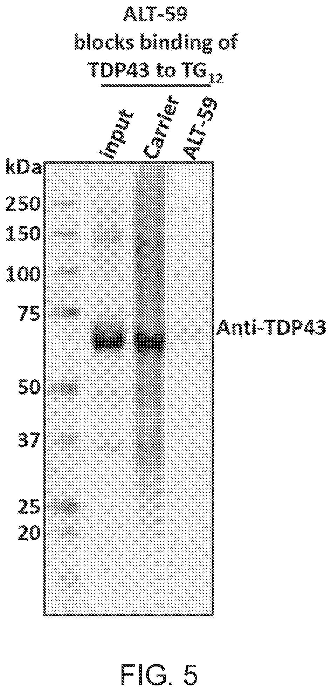

FIG. 5 depicts inhibited binding of His-Sumo-TDP43 to TG₁₂-biotin by ALT-59. Recombinant His-Sumo-TDP43 were incubated with TG₁₂-biotin (TGTGTGTGTGTGTGTGTGTGTGTG-biotin (SEQ ID NO: 1)) in the presence of carrier or ALT-59. Streptavidin magnetic beads were added to pull down the DNA-protein complex, which were then boiled and fractionated by SDS-PAGE. Western Blotting was performed to detect TDP-43 using anti-TDP-43 antibody.

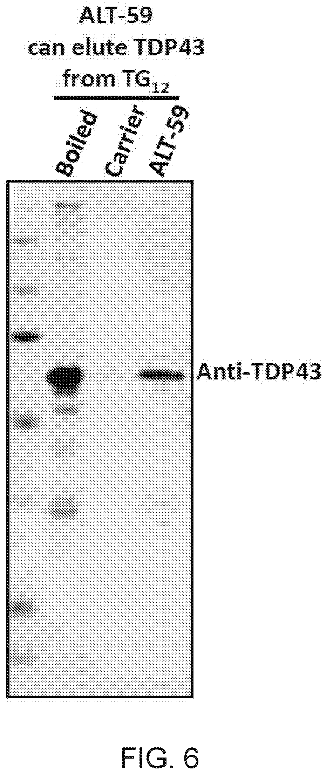

FIG. 6 depicts elution of His-Sumo-TDP43 from the DNA-protein complex by ALT-59. Recombinant His-Sumo-TDP43 were incubated with TG₁₂-biotin. Streptavidin magnetic beads were added to pull down the DNA-protein complex. After being washed three times with binding buffer to remove unbound protein, the DNA-protein complex were divided into 3 parts, one was boiled, the other two were incubated with carrier or ALT-59. The supernatants were boiled and fractionated by SDS-PAGE. Western Blotting was performed to detect TDP-43 using anti-TDP-43 antibody.

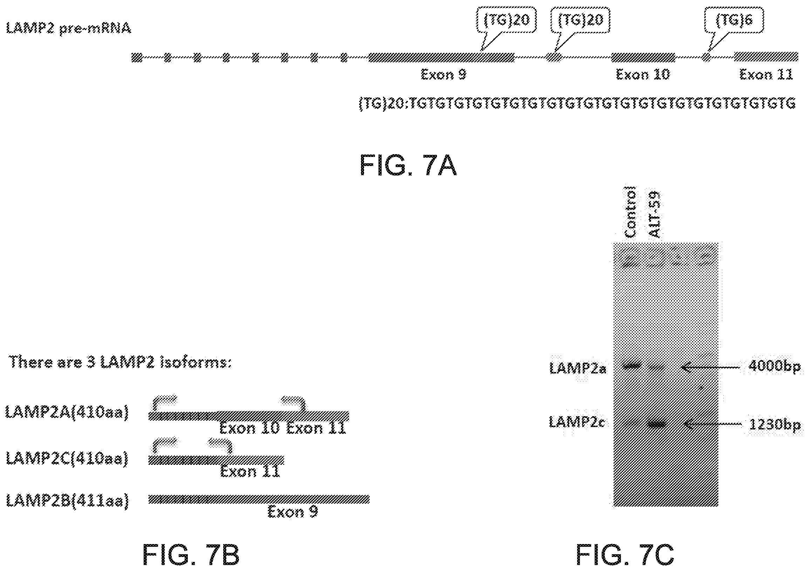

FIGs. 7A-7C depict changed lysosome-associated membrane protein 2 (LAMP2) pre-mRNA splicing following ALT-59 treatment. LAMP2 pre-mRNA contains 11 exons. Two long stretches of dinucleotide TG repeats were located in exon 9 and intron 9 (FIG. 7A). Alternative splicing of LAMP2 pre-mRNA produces three isoforms, which share the same 8 exons at the 5'-end and are translated into proteins with different C-terminal tails. Primers located on exon 1 and 11 amplify fragments with different lengths from LAMP2A and LAMP2C mRNAs (FIG. 7B). Semi-quantitative reverse transcription polymerase chain reaction (RT-PCR) experiments were performed using RNA samples purified from MDA-MB-231 cells treated with dimethyl sulfoxide (DMSO) or ALT-59. RT-PCR samples were separated using 1% agarose gel. Inhibition of TDP-43 by ALT-59 leads to exclusion of exon 10 and 11, resulting in increased LAMP2C mRNA (FIG. 7C).

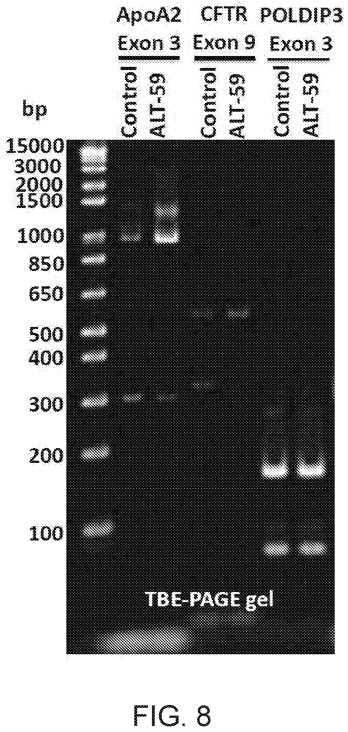

FIG. 8 depicts the effect of ALT-59 treatment on pre-mRNA splicing of apolipoprotein A2 (ApoA2), cystic fibrosis transmembrane conductance regulator (CFTR), and polymerase delta interacting protein 3 (POLDIP3). RT-PCR experiments were performed using RNA samples purified from MDA-MB-231 cells treated with DMSO or ALT-59. RT-PCR samples were separated using TBE-PAGE gel. Inhibition of TDP-43 by ALT-59 inhibited ApoA2 pre-mRNA splicing, and CFTR exon 9 skipping, but did not change the splicing of POLDIP3.

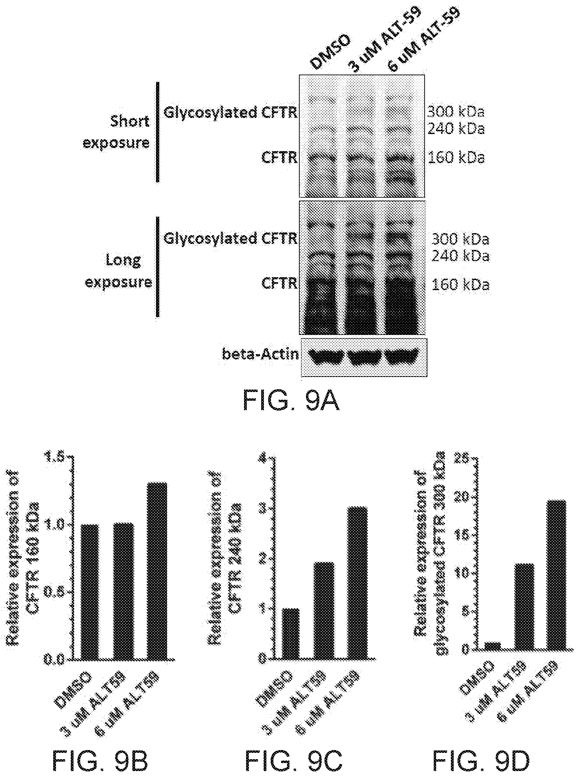

FIGs. 9A-9D depict increased expression and glycosylation of CFTR following ALT-59 treatment. U-87 MG cells were treated with DMSO, 3 µM, or 6 µM ALT-59. Western blotting was performed to detect levels of CFTR using an anti-CFTR antibody. Multiple CFTR isoforms were detected. Shorter exposure (FIG. 9A, upper panel) and longer exposure (FIG. 9A, lower panel) of the same membrane showed increased signal from CFTR bands at 160 kDa, 240 kDa, and 300 kDa, which are quantified in FIG. 9B, FIG. 9C, and FIG. 9D, respectively.

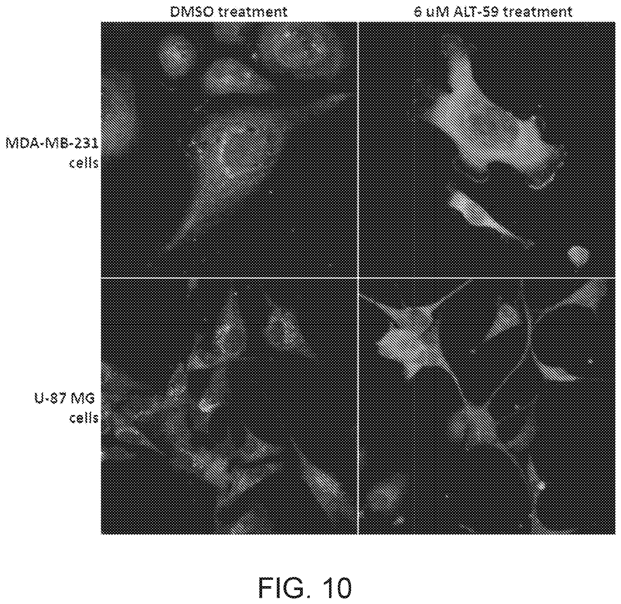

FIG. 10 depicts micrographs showing increased CFTR localization on the plasma membrane in both MDA-MB-231 and U-87 MG cells upon treatment by ALT-59. MDA-MB-231 and U-87 MG cells were cultured on cover slips and treated with DMSO or 4 µM ALT-59. Cells were fixed, permeabilized, and immunostained. Images were taken using Carl Zeiss LSM 510 Laser Scanning Microscope. Parameters were identical for all images. ALT-59 treatment caused redistribution of CFTR from perinuclear region to the plasma membrane.

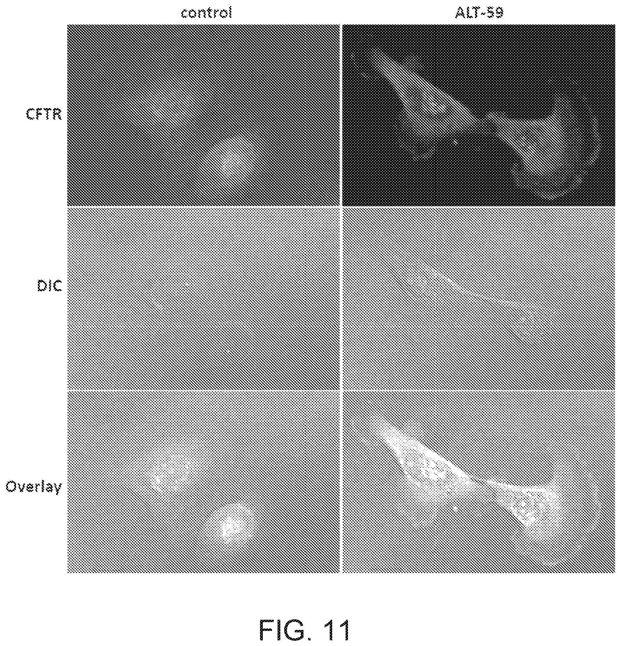

FIG. 11 depicts micrographs showing increased CFTR localization on the plasma membrane in ALT-59 treated primary bronchial epithelial cells isolated from cystic fibrosis patient homozygous for the ΔF508 mutation. Cells were cultured on cover slips and treated with carrier or 4 µM ALT-59. Cells were fixed, permeabilized, and immunostained. Images were taken using a Carl Zeiss LSM 510 Laser Scanning Microscope. Parameters were identical for all images. ALT-59 treatment caused increased localization of ΔF508-CFTR on the plasma membrane.

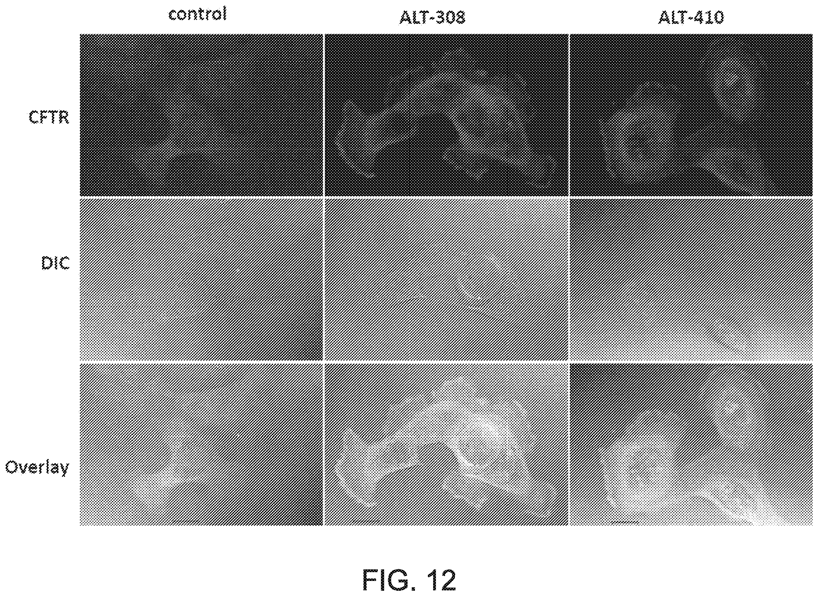

FIG. 12 depicts micrographs showing increased CFTR localization on the plasma membrane in ALT-308 and ALT-410 treated primary bronchial epithelial cells isolated from cystic fibrosis patient homozygous for the ΔF508 mutation. Cells were cultured on cover slips and treated with carrier or 2 µM ALT-59. Cells were fixed, permeabilized, and immunostained. Images were taken using a Carl Zeiss LSM 510 Laser Scanning Microscope. Parameters were identical for all images. ALT-59 treatment caused increased localization of CFTR on the plasma membrane.

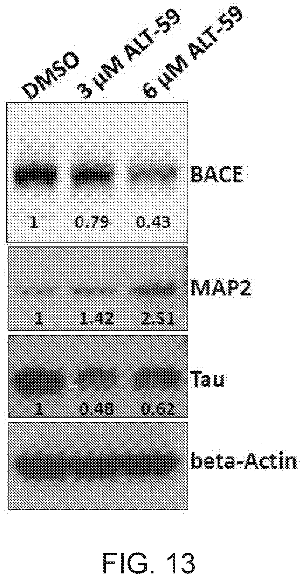

FIG. 13 depicts altered expression of proteins associated with neurodegenerative diseases following ALT-59 treatment. U-87 MG cells were treated with DMSO, 3 µM, or 6 µM ALT-59 and subjected to Western blotting analysis. Protein expression were determined using the indicated antibodies. Beta-actin was used as an internal control to indicate equal loading of samples. Numbers below bands indicate relative quantity of corresponding bands.

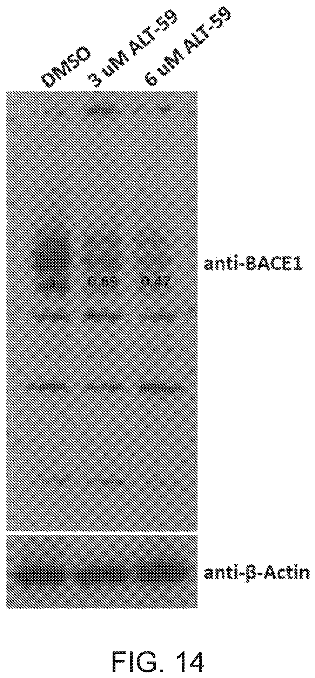

FIG. 14 depicts reduced expression of beta-secretase 1 (BACE1) upon ALT-59 treatment in T98G cells. Cells were treated with DMSO, 3 µM or 6 µM ALT-59 and subjected to Western blotting analysis. Protein expression were determined using anti-BACE1 antibody. Beta-actin was used as an internal control to indicate equal loading of samples. Numbers below bands indicate relative quantity of corresponding bands.

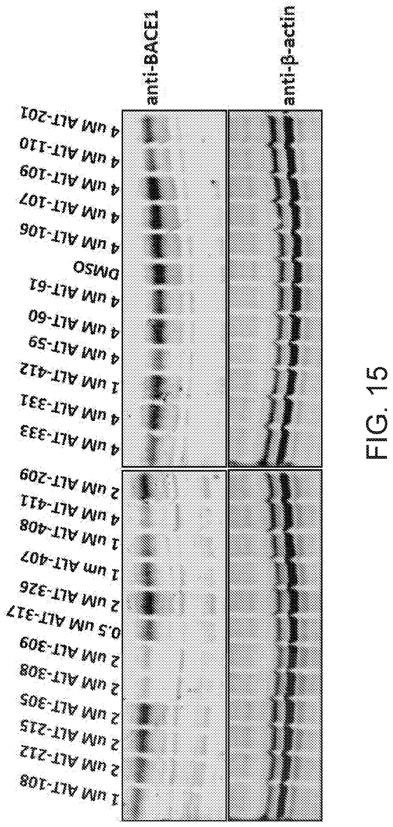

FIG. 15 depicts results from a screening for ALT-59 analogs. U-87 MG cells were treated with the indicated compounds at the indicated concentrations. BACE1 expression levels were used as screening marker to indicate TDP-43 activity.

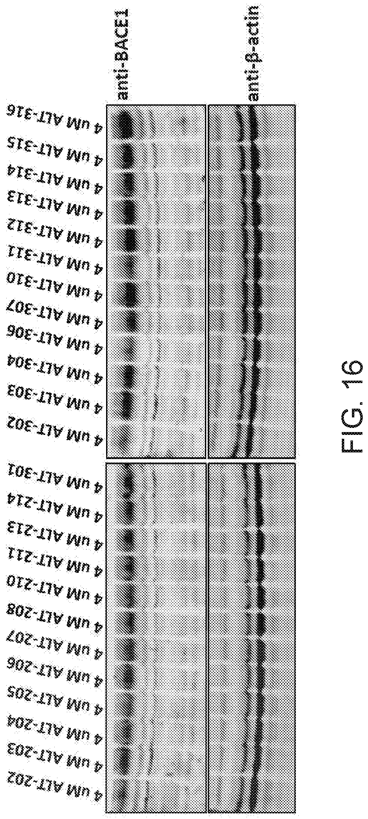

FIG. 16 depicts results from a screening for ALT-59 analogs. U-87 MG cells were treated with the indicated compounds at the indicated concentrations. BACE1 expression levels were used as screening marker to indicate TDP-43 activity.

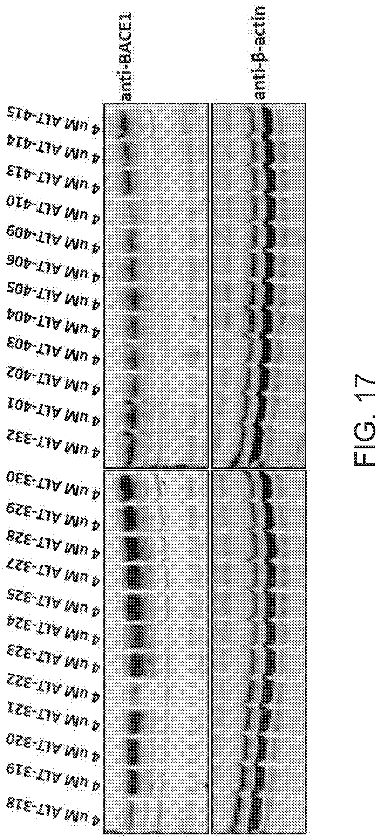

FIG. 17 depicts results from a screening for ALT-59 analogs. U-87 MG cells were treated with the indicated compounds at the indicated concentrations. BACE1 expression levels were used as screening marker to indicate TDP-43 activity.

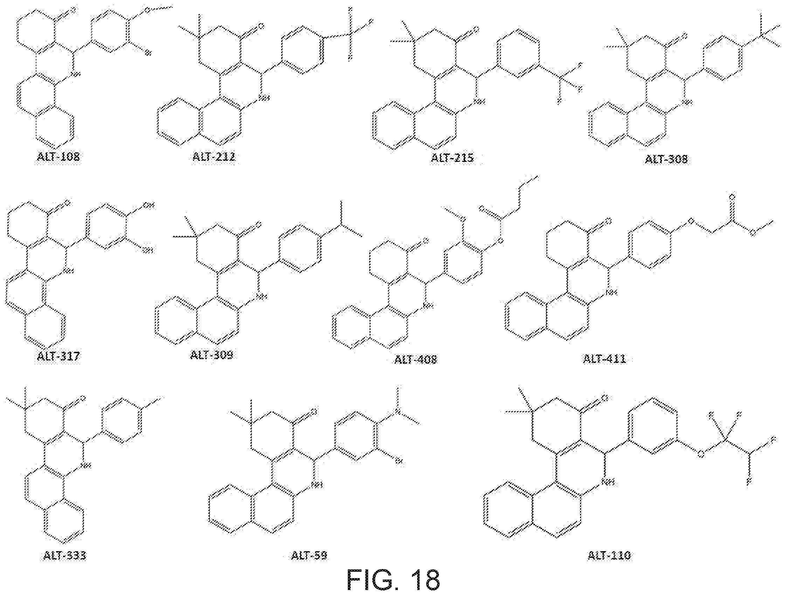

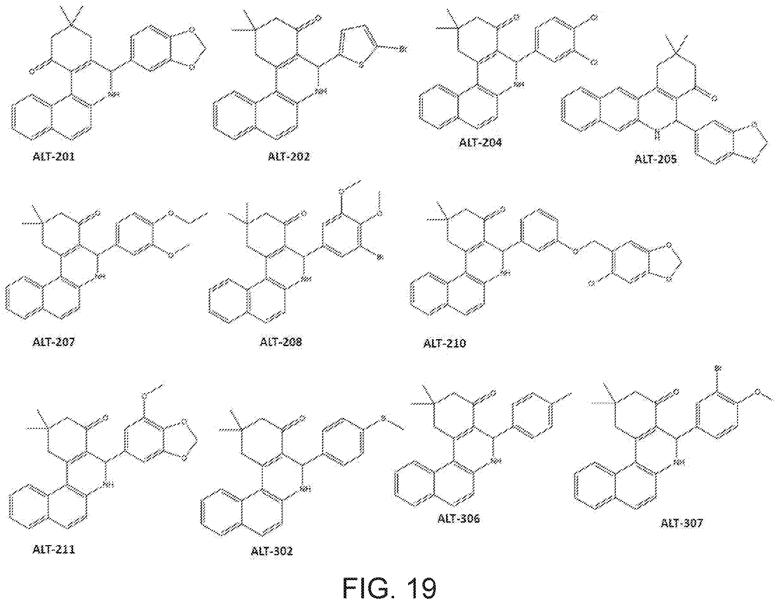

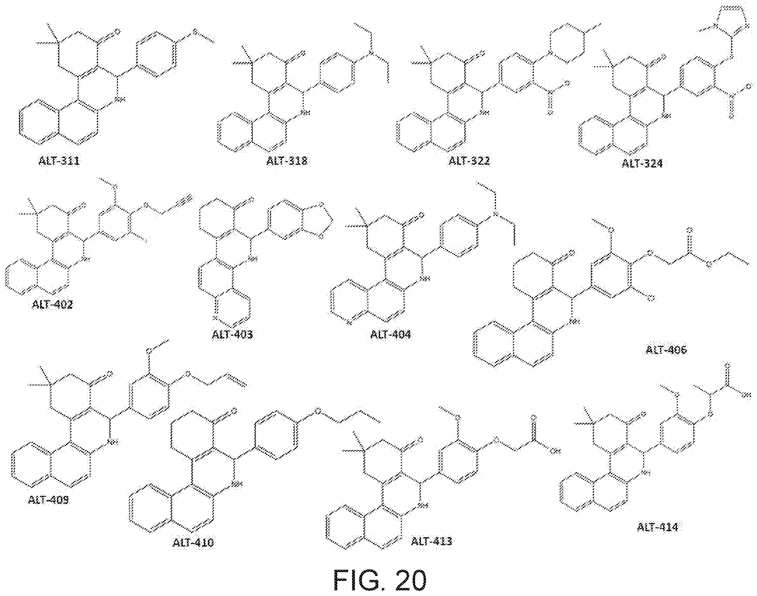

FIGs. 18, 19, and 20 depict the chemical structures of compounds that can repress BACE1 protein expression as shown in FIGS. 15-17.

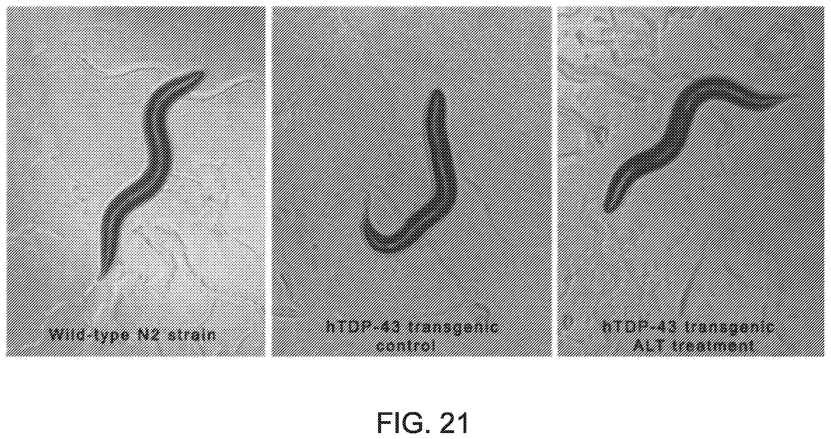

FIG. 21 depicts micrographs of *Caenorhabditis elegans.* From left to right, shows wild-type *C*. *elegans,* a transgenic *C*. *elegans* with human TDP-43 treated with control, and a transgenic *C*. *elegans* with human TDP-43 treated with the compounds described herein.

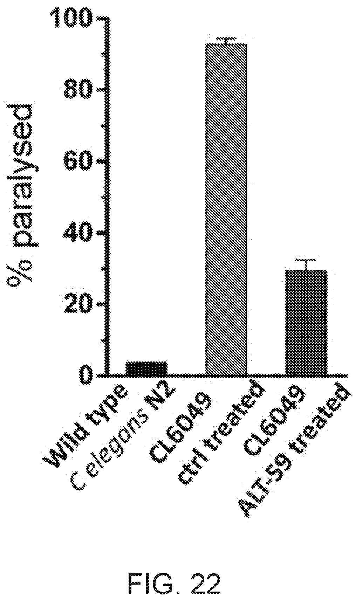

FIG. 22 graphically depicts a percentage of paralysis for the three *C*. *elegans* groups shown in FIG. 21, depicting that the transgenic *C*. *elegans* with human TDP-43 exhibits rescued phenotype upon treatment with the compounds described herein.

### DETAILED DESCRIPTION

In the following detailed description, reference is made to the accompanying drawings, which form a part hereof. In the drawings, similar symbols typically identify similar components, unless context dictates otherwise. The illustrative embodiments described in the detailed description, drawings, and claims are not meant to be limiting. Other embodiments may be utilized, and other changes may be made. It will be readily understood that the aspects of the present disclosure, as generally described herein, and illustrated in the Figures, can be arranged, substituted, combined, separated, and designed in a wide variety of different configurations.

### I. Diseases

TDP-43 preferentially binds to UG (or TG) single-stranded dinucleotide repeats through its RNA recognition motif (RRM) domains. Binding affinity increases with the number of repeats and equilibrium dissociation constant (Kd) falls below 10 nM when there are more than 8 such repeats (Buratti and Baralle, (2001b), J Biol Chem 276, 36337-36343; Kuo et al., 2009, Nucl Acids Res 37, 1799-1808). UG/TG-rich motifs can be found in various genes involved in neurodegenerative disorders and cystic fibrosis, such as microtubule-associated protein tau (MAPT) and cystic fibrosis transmembrane conductance regulator (CFTR).

Accordingly, provided herein are compositions comprising a compound of Formulas (I), (II), (III), or (IV), an analogue, derivative, or pharmaceutically acceptable salt thereof, including any of the compounds specifically disclosed herein, for use in the treatment of a disease or condition associated with TDP-43 toxicity. Diseases or conditions associated with TDP-43 toxicity include, for example, cystic fibrosis and neurodegenerative diseases.

In some embodiments, the compound of Formula (I) is a compound of any one of Formula (V), (VI), (VII), or (VIII). In some embodiments, provided herein are compositions comprising a compound of Formulas (V), (VI), (VII), or (VIII), an analogue, derivative, or pharmaceutically acceptable salt thereof, including any of the compounds specifically disclosed herein, for use in the treatment of a disease or condition associated with TDP-43 toxicity. Diseases or conditions associated with TDP-43 toxicity include, for example, cystic fibrosis and neurodegenerative diseases.

Cystic fibrosis is caused by mutations in the CFTR gene, which encodes an ABC transporter-class ion channel protein that conducts chloride and thiocyanate ions across epithelial cell membranes. Mutations affecting the chloride ion channel function of CFTR impair epithelial fluid transport in the lung, pancreas and other organs, resulting in the lethal disease cystic fibrosis. CFTR mutations also contribute to a variety of other clinical symptoms such as congenital bilateral absence of vas deferens (CBAVD) (Chillon et al., (1995), N Engl J Med 332, 1475-1480), idiopathic pancreatitis (Noone et al., 2001, Gastroenterology 121, 1310-1319), nasal polyposis (Kostuch et al., 2005, Eur Arch Oto-Rhino-L 262, 982-986), bronchiectasis (Casals et al., (2004), Clin Genet 65, 490-495), and bronchopulmonary allergic aspergillosis (Miller et al., 1996, Am J Hum Genet 59, 45-51).

To function properly, CFTR needs to be translated as full-length protein and correctly folded, glycosylated, and transported to the cell surface through the ER-Golgi secretory pathway. TDP-43 binds to the polymorphic TG repeats at the 3' splice site in CFTR intron 8 and causes skipping of exon 9. CFTR transcript missing exon 9 is not properly processed and is not capable of conducting chloride across cell membranes (Buratti and Baralle, (2001b), J Biol Chem 276, 36337-36343; Strong et al., 1993, Hum Mol Genet 2, 225-230). The numbers of contiguous thymidines immediately following the TG repeats has been correlated with exon 9 skipping. The allele with nine contiguous thymidines, named 9T, has shorter TG repeats (<12) and results in the highest expression of normal CFTR mRNA. The 5T-TG₁₂ allele with 5 thymidines and 12 TGs exacerbates skipping of exon 9, thereby resulting in reduced levels of functional CFTR protein. The 5T-TG₁₂ allele is found in approximately 10% of the general population and 91% affected individuals with male infertility or nonclassical cystic fibrosis (Groman et al., 2004, American Journal of Human Genetics 74, 176-179). Modulators that are able to block the DNA/RNA binding activity of TDP-43 will increase the level of functional full-length CFTR in individuals with longer TG repeats.

Abnormal accumulation of TDP-43 has been identified as a pathological hallmark of both amyotrophic lateral sclerosis (ALS) and frontotemporal lobar degeneration (FTLD). TDP-43-positive inclusions have been shown to be common to about 97% ALS and about 50% of FTLD cases (Arai et al., 2006, Biochem Bioph Res Co 351, 602-611; Ling et al., 2013, Neuron 79, 416-438; Mackenzie et al., 2007, Ann Neurol 61, 427-434; Neumann et al., 2006, Science 314, 130-133). This pathology is also observed in other neurodegenerative diseases, such as 52% of patients with Alzheimer's disease (James et al., 2016, Brain 139, 2983-2993), 92.4% of patients with hippocampal sclerosis of aging (HS-Aging) (Ihara et al., 2018, Ann Neurol 84, 741-753), 85% of patients with chronic traumatic encephalopathy (McKee et al., 2013, Brain 136, 43-64), and 10% of patients with Parkinson's disease (Nakashima-Yasuda et al., 2007, Acta Neuropathol 114, 221-229). TDP-43 positive individuals are 10 times more likely to be cognitively impaired at death compared to TDP-43-negative individuals. Greater TDP-43 burden and more extensive TDP-43 distribution is associated with greater cognitive impairment and medial temporal atrophy (Josephs et al., 2014, Acta Neuropathol 127, 811-824). Currently, there is an urgent unmet medical need to find effective treatments for neurodegenerative diseases.

TDP-43 is expressed at high-level during embryonic development, progressively decreases during postnatal development, and maintains at low level in adult neurons (Sephton et al., 2010, J Biol Chem 285, 38740-38740; Uchida et al., 2012, Brain 135, 833-846). High TDP-43 activity is essential for embryonic development, but has been proven toxic for mature neurons. Overexpression of TDP-43 or TDP-43 mutants leads to neurodegenerative diseases in various animal models, including mouse (Wils et al., 2010, PNAS 107, 3858-3863), zebrafish (Kabashi et al., 2010, Hum Mol Genet 19, 671-683), Drosophila (Li et al., 2010, PNAS 107, 3169-3174; Miguel et al., 2011, Neurobiol Dis 41, 398-406), and *C*. *elegans* (Ash et al., (2010), Hum Mol Genet 19, 3206-3218). Knock-in mice enable TDP-43 mutants to be expressed under control by native TDP-43 promoter to circumvent potential artifacts due to overexpression. White et al. (2018) showed that introduction of the disease causal mutation TDP-43^{Q331K} lead to a gain-of-function and cognitive dysfunction and a paucity of parvalbumin interneurons. Knock-in mice harboring TDP-43^{M323K} or TDP-43^{Q331K} generated by Fratta et al. (2018) also showed gain-of-function splicing activity and a neuromuscular and neurodegenerative phenotype. The F210I mutation in the RNA recognition motif 2 (RRM2) domain reduces TDP-43's RNA/DNA binding capacity and does not cause any motor phenotypes in the knock-in mice. Importantly, the F210I mutation can partially rescue the toxicity caused by the M323K mutation in the compound heterozygous TDP-43^{M323K} /TDP-43^{F331I} mice (Fratta et al., (2018), EMBO J 37; White et al., 2018, Nature Neuroscience 21, 1138-1138). These studies definitively demonstrated that TDP-43 neurotoxicity is caused by a gain-of-function mechanism.

Accordingly, provided herein are compounds capable of abolishing or reducing the RNA/DNA binding activity of TDP-43 for the treatment of diseases caused by TDP-43 overexpression or mutations.

In some embodiments, the neurodegenerative disease is selected from the group consisting of Alzheimer's disease, frontotemporal dementia, FTLD-U (a frontotemporal dementia caused by mutations in progranulin protein), amyotrophic lateral sclerosis (ALS), Huntington's chorea, Creutzfeld-Jacob disease, trinucleotide repeat diseases, cerebral degenerative diseases presenile dementia, senile dementia, Parkinsonism linked to chromosome 17 (FTDP-17), progressive supranuclear palsy (PSP), Huntington's disease (HD), Pick's disease, primary progressive aphasia, corticobasal dementia, Parkinson's disease, Parkinson's disease with dementia, dementia with Lewy bodies, Down's syndrome, multiple system atrophy, spinal muscular atrophy (SMA), spinocerebellar ataxia, spinal degenerative disease/motor neuron degenerative diseases, Hallervorden-Spatz syndrome, cerebral infarct, cerebral trauma, chronic traumatic encephalopathy, transient ischemic attack, and any combination thereof.

### II. Pharmaceutical Compositions of a Compound of Formulas (I) to (VIII)

Embodiments provided herein relate to compounds for use in the treatment of a disease or condition associated with TDP-43 toxicity, such as cystic fibrosis or neurodegeneration, including, amyotrophic lateral sclerosis (ALS), frontotemporal dementia (FTD), Alzheimer's disease, hippocampal sclerosis of aging (HS-Aging), chronic traumatic encephalopathy, and Parkinson's disease and methods of using the same to treat a disease or condition associated with TDP-43 toxicity.

As used herein, the terms "treating," "treatment," "therapeutic," or "therapy" have their ordinary meaning as understood in light of the specification, and do not necessarily mean total cure or abolition of the disease or condition.

As used herein, the term "inhibit" has its ordinary meaning as understood in light of the specification, and refers to the delay or prevention of a disease or condition associated with TDP-43 toxicity, such as cystic fibrosis or neurodegeneration. As used herein, the term "delay" has its ordinary meaning as understood in light of the specification, and refers to a slowing, postponement, or deferment of an event, such as the delay of a disease or condition associated with TDP-43 toxicity, such as cystic fibrosis or neurodegeneration, to a time that is later than would otherwise be expected. The delay can be a delay of 10%, 20%, 30%, 40%, 50%, 60%, 70%, 80%, 90%, 100%, or an amount within a range defined by any two of the aforementioned values. The terms inhibit and delay are not to be construed as necessarily indicating a 100% inhibition or delay. A partial inhibition or delay may be realized.

The term "therapeutically effective amount" has its ordinary meaning as understood in light of the specification, and is used to indicate an amount of an active compound, or pharmaceutical agent, that elicits the biological or medicinal response indicated. For example, a therapeutically effective amount of compound can be the amount needed to prevent, alleviate or ameliorate symptoms of disease or prolong the survival of the subject being administered the therapy. This response may occur in a tissue, system, animal, or human and includes alleviation of the signs or symptoms of the disease being treated. Determination of a therapeutically effective amount is within the capability of those skilled in the art, in view of the disclosure provided herein. The therapeutically effective amount of the compounds disclosed herein required as a dose will depend on the route of administration, the type of animal, including human, being treated, and the physical characteristics of the specific animal under consideration. The dose can be tailored to achieve a desired effect, but will depend on such factors as weight, diet, concurrent medication and other factors that those skilled in the medical arts will recognize.

As used herein, the term "derivative" has its ordinary meaning as understood in light of the specification, and refers to a chemically modified compound wherein the modification is considered routine by the ordinary skilled chemist, such as an ester or an amide of an acid, or protecting groups such as a benzyl group for an alcohol or thiol, or a tert-butoxycarbonyl group for an amine.

As used herein, the term "analogue" has its ordinary meaning as understood in light of the specification, and refers to a compound, which includes a chemically modified form of a specific compound or class thereof and which maintains the pharmaceutical and/or pharmacological activities characteristic of said compound or class.

As used herein, any "R" group(s) such as, without limitation, R, R₁, R₂, R₃, R₄, R₅, R₆, R₇, R₈, R₉, R₁₀, R₁₁, R₁₂, R₁₃, and R₁₄ represent substituents that can be attached to the indicated atom. An R group may be substituted or unsubstituted.

Whenever a group is described as being "optionally substituted" that group may be unsubstituted or substituted with one or more of the indicated substituents. Likewise, when a group is described as being "unsubstituted or substituted" if substituted, the substituent may be selected from one or more the indicated substituents. If no substituents are indicated, it is meant that the indicated "optionally substituted" or "substituted" group may be substituted with one or more group(s) individually and independently selected from alkyl, alkenyl, alkynyl, cycloalkyl, cycloalkenyl, cycloalkynyl, aryl, heteroaryl, heteroalicyclyl, aralkyl, heteroaralkyl, (heteroalicyclyl)alkyl, hydroxy, protected hydroxyl, alkoxy, aryloxy, acyl, mercapto, alkylthio, arylthio, cyano, halogen, thiocarbonyl, O-carbamyl, N-carbamyl, O-thiocarbamyl, N-thiocarbamyl, C-amido, N-amido, S-sulfonamido, N-sulfonamido, C-carboxy, protected C-carboxy, O-carboxy, isocyanato, thiocyanato, isothiocyanato, nitro, silyl, sulfenyl, sulfinyl, sulfonyl, haloalkyl, haloalkoxy, trihalomethanesulfonyl, trihalomethanesulfonamido, amino, mono-substituted amino group and di-substituted amino group, and protected derivatives thereof.

The term "alkyl" as used herein has its ordinary meaning as understood in light of the specification, and refers to a fully saturated radical consisting only of carbon and hydrogen, having from 1 to about 25 carbon atoms. The term "lower alkyl" refers to an alkyl radical having from 1 to about 6 carbon atoms, such as, for example, methyl, ethyl, propyl, isopropyl, butyl, 3-methylpentyl, hexyl, and the like.

The term "lower alkoxy" has its ordinary meaning as understood in light of the specification, and refers to a radical of the form RO-, where R is lower alkyl. Suitable examples of lower alkoxy include, without limitation, methoxy, ethoxy, propyloxy, 2-propyloxy, butoxy, t-butoxy, hexyloxy, and the like. Similarly, "lower alkylthio" refers to a radical of the form RS-, where R is lower alkyl. "Lower alkylenedioxy" refers to a diradical of the form -O-R'-O-, where R' is a lower alkyl diradical. Exemplary alkylenedioxy moieties include, without limitation, methylenedioxy, 1,2-ethylenedioxy, 2,2-propylenedioxy, and the like.

The term "aryl" has its ordinary meaning as understood in light of the specification, and refers to phenyl or naphthyl. "Aralkyl" refers to a moiety of the form Ar-R'-, where Ar is aryl and R' is lower alkylene. Exemplary aralkyl radicals include, without limitation, benzyl, phenethyl, 4-phenylhexyl, 2-naphthylmethyl, 1-naphthylethyl, and the like.

A "heterocyclic ring" as used herein has its ordinary meaning as understood in light of the specification, and refers to a closed loop of 3-7 atoms containing carbon and at least one atom of O, N, S, and/or P. Heterocyclic rings can be saturated or unsaturated. Exemplary heterocyclic rings include, without limitation, piperidine, furan, tetrahydro-furan, pyrrole, triazole, pyran, tetrahydropyran, thiazole, dioxin, 2,2-dimethyl-1,3-dioxolane, and the like. Heterocyclic rings in the context of this invention will be fused to the phenyl ring that carries R₉, R₁₀, R₁₁, R₁₂, R₁₃, and R₁₄, thus forming ring systems such as, for example, benzimidazole, benzofuran, and the like.

The term "halo" as used herein has its ordinary meaning as understood in light of the specification, and refers to fluoro, chloro, bromo, and iodo.

The term "pharmaceutically acceptable" has its ordinary meaning as understood in light of the specification, and refers to compounds and derivatives that are not unacceptably toxic to an organism or tissue to be treated.

The term "salt" has its ordinary meaning as understood in light of the specification, and refers to a derivative of a compound of the invention derived by addition of a simple acid to a basic compound of the invention, or addition of a base to an acidic compound of the invention. For example, compounds of the invention can form acid addition salts, such as hydrochlorides, hydrobromides, acetates, tartarates, citrates, malonates, phosphates, nitrates, sulfates, mesylates, and the like. The term "esters" has its ordinary meaning as understood in light of the specification, and refers to derivatives of a compound of the invention derived by condensing a compound of the invention having a free -OH group with a carboxylic acid. Exemplary esters include acetates, propionates, citrates, and the like. The term "amides" has its ordinary meaning as understood in light of the specification, and refers to derivatives of a compound of the invention derived by condensing a compound of the invention having a free -NH group with a carboxylic acid. Exemplary acids include acetic, propionic, citric, malonic, and the like.

In some embodiments, the compound for use in the treatment of a disease or condition associated with TDP-43 toxicity includes a compound of Formula (I) or a pharmaceutically acceptable salt thereof, wherein the compound of Formula (I) or a pharmaceutically acceptable salt thereof is: 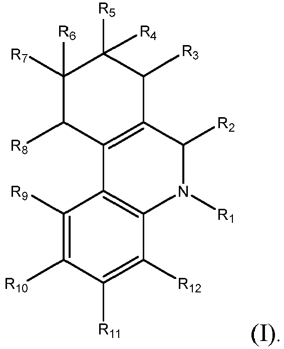 wherein R₁ is H, OH, or lower alkyl; R₂ is an optionally substituted aromatic ring of four, five, or six carbons, wherein the aromatic ring is carbocyclic or heterocyclic, and wherein each position on the aromatic ring is independently H, halo, lower alkyl, OH, lower alkoxy, NH₂, lower alkylamino, di(lower alkyl)amino, SH, lower alkylthio, NO₂, or two residues together form a heterocyclic ring; R₃, R₄, R₅, R₆, and R₇ are each independently H, lower alkyl, OH, NH₂, aryl, or aralkyl, where aryl and aralkyl are substituted with 0-3 moieties selected from the group consisting of halo, OH, NH₂, lower alkyl, lower alkoxy, SH, lower alkylthio, and lower alkylamino; wherein at least one of R₈ and R₉, R₉ and R₁₀, or R₁₀ and R₁₁ together form an optionally substituted benzene ring, wherein the benzene ring is optionally substituted with H, halo, lower alkyl, OH, lower alkoxy, or NO₂; and wherein any one of the carbon atoms on any one of fused rings of Formula (I) is optionally replaced with a nitrogen atom.

In some embodiments, the compound of Formula (I) is a compound of any one of Formulas (II), (III), or (IV). In some embodiments, the compound of Formula (I) is any one of the compounds described herein, including any one of ALT-59, ALT-212, ALT-215, ALT-308, ALT-309, ALT-408, ALT-411, ALT-110, ALT-201, ALT-202, ALT-204, ALT-207, ALT-208, ALT-210, ALT-211, ALT-302, ALT-306, ALT-307, ALT-311, ALT-318, ALT-322, ALT-324, ALT-402, ALT-404, ALT-406, ALT-409, ALT-410, ALT-413, ALT-414, ALT-108, ALT-317, ALT-333, ALT-403, or ALT-205, as described in any of the embodiments herein in further detail.

In some embodiments, the compound for use in the treatment of a disease or condition associated with TDP-43 toxicity includes a compound of Formula (II) or a pharmaceutically acceptable salt thereof, wherein the compound of Formula (II) or a pharmaceutically acceptable salt thereof is: 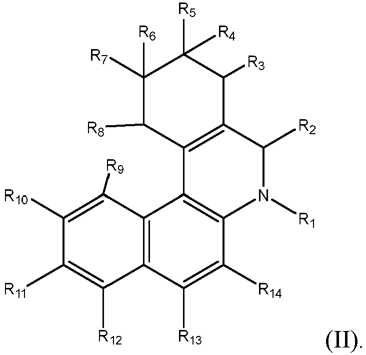 wherein R₁ is H, OH, or lower alkyl; R₂ is an optionally substituted aromatic ring of four, five, or six carbons, wherein the aromatic ring is carbocyclic or heterocyclic, and wherein each position on the aromatic ring is independently H, halo, lower alkyl, OH, lower alkoxy, NH₂, lower alkylamino, di(lower alkyl)amino, SH, lower alkylthio, NO₂, or two residues together form a heterocyclic ring; R₃ and R₈ are each independently H, lower alkyl, =O, =S, OH, NH₂, aryl, or aralkyl, where aryl and aralkyl are substituted with 0-3 moieties selected from the group consisting of halo, OH, NH₂, lower alkyl, lower alkoxy, SH, lower alkylthio, and lower alkylamino; R₄, R₅, R₆, and R₇ are each independently H, lower alkyl, OH, NH₂, aryl, or aralkyl, where aryl and aralkyl are substituted with 0-3 moieties selected from the group consisting of halo, OH, NH₂, lower alkyl, lower alkoxy, SH, lower alkylthio, and lower alkylamino; R₉, R₁₀, R₁₁, R₁₂, R₁₃, and R₁₄ are each independently H, halo, lower alkyl, OH, lower alkoxy, or NO₂; and wherein any one of the carbon atoms on any one of fused rings of Formula (II) is optionally replaced with a nitrogen atom.

Some embodiments relate to analogues or derivatives of the compound of Formula (II), wherein the compound of Formula (II), an analogue, derivative, or pharmaceutically acceptable salt thereof, including any of the compounds specifically disclosed herein, is used in the treatment of a disease or condition associated with TDP-43 toxicity, which include, for example, cystic fibrosis and a neurodegenerative disease. Examples of derivatives or analogues of the compound of Formula (II) include the following compounds:
ALT-59 or a pharmaceutically acceptable salt thereof, with the chemical name of: 5-(3-bromo-4-(dimethylamino)phenyl)-2,2-dimethyl-2,3,5,6-tetrahydrobenzo[a]phenanthridin-4(1H)-one: 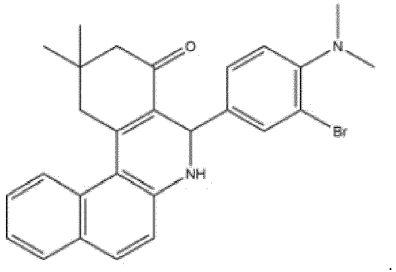
ALT-212 or a pharmaceutically acceptable salt thereof, with the chemical name of: 2,2-dimethyl-5-(4-(trifluoromethyl)phenyl)-2,3,5,6-tetrahydrobenzo[a]phenanthridin-4(1H)-one: 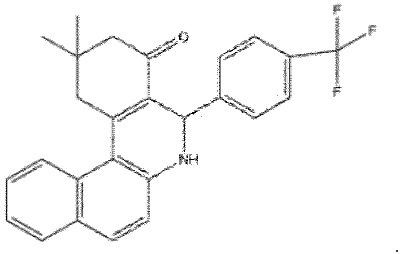
ALT-215 or a pharmaceutically acceptable salt thereof, with the chemical name of: 2,2-dimethyl-5-(3-(trifluoromethyl)phenyl)-2,3,5,6-tetrahydrobenzo[a]phenanthridin-4(1H)-one: 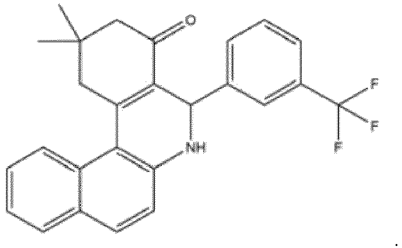
ALT-308 or a pharmaceutically acceptable salt thereof, with the chemical name of: 5-(4-(tert-butyl)phenyl)-2,2-dimethyl-2,3,5,6-tetrahydrobenzo[a]phenanthridin-4(1H)-one: 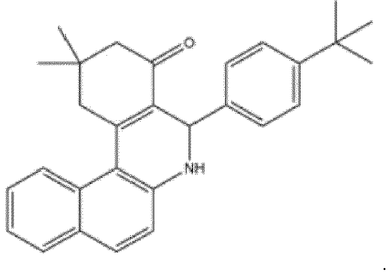
ALT-309 or a pharmaceutically acceptable salt thereof, with the chemical name of: 5-(4-isopropylphenyl)-2,2-dimethyl-2,3,5,6-tetrahydrobenzo[a]phenanthridin-4(1H)-one: 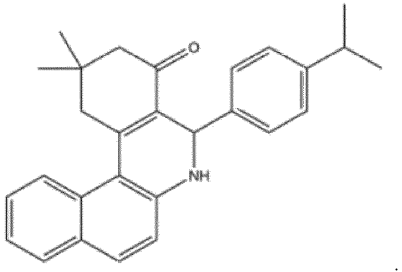
ALT-408 or a pharmaceutically acceptable salt thereof, with the chemical name of: 2-methoxy-4-(4-oxo-1,2,3,4,5,6-hexahydrobenzo[a]phenanthridin-5-yl)phenyl butyrate: 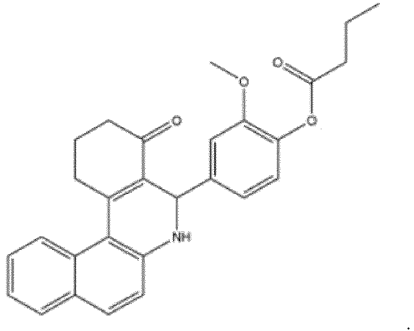
ALT-411 or a pharmaceutically acceptable salt thereof, with the chemical name of: methyl 2-(4-(4-oxo-1,2,3,4,5,6-hexahydrobenzo[a]phenanthridin-5-yl)phenoxy)acetate: 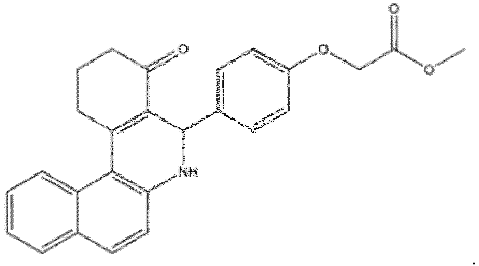
ALT-110 or a pharmaceutically acceptable salt thereof, with the chemical name of: 2,2-dimethyl-5-(3-(1,1,2,2-tetrafluoroethoxy)phenyl)-2,3,5,6-tetrahydrobenzo[a]phenanthridin-4(1H)-one: 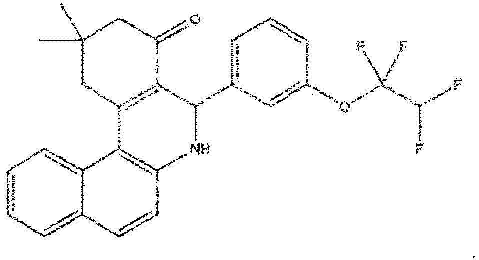
ALT-201 or a pharmaceutically acceptable salt thereof, with the chemical name of: 5-(benzo[d][1,3]dioxol-5-yl)-3,3-dimethyl-3,4,5,6-tetrahydrobenzo[a]phenanthridin-1(2H)-one: 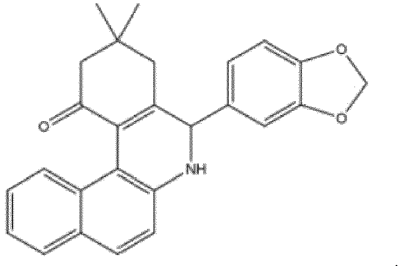
ALT-202 or a pharmaceutically acceptable salt thereof, with the chemical name of: 5-(5-bromothiophen-2-yl)-2,2-dimethyl-2,3,5,6-tetrahydrobenzo[a]phenanthridin-4(1H)-one: 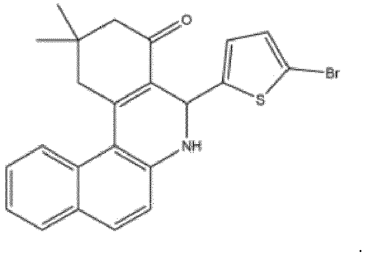
ALT-204 or a pharmaceutically acceptable salt thereof, with the chemical name of: 5-(3,4-dichlorophenyl)-2,2-dimethyl-2,3,5,6-tetrahydrobenzo[a]phenanthridin-4(1H)-one: 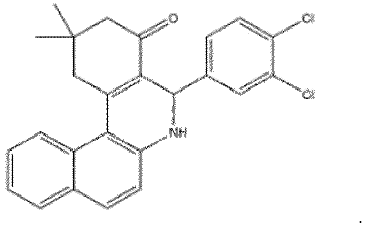
ALT-207 or a pharmaceutically acceptable salt thereof, with the chemical name of: 5-(4-ethoxy-3-methoxyphenyl)-2,2-dimethyl-2,3,5,6-tetrahydrobenzo[a]phenanthridin-4(1H)-one: 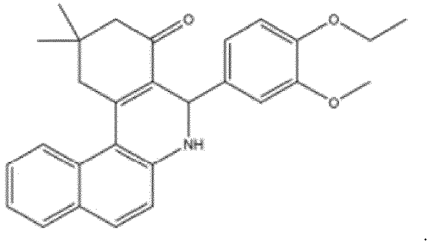
ALT-208 or a pharmaceutically acceptable salt thereof, with the chemical name of: 5-(3-bromo-4,5-dimethoxyphenyl)-2,2-dimethyl-2,3,5,6-tetrahydrobenzo[a]phenanthridin-4(1H)-one: 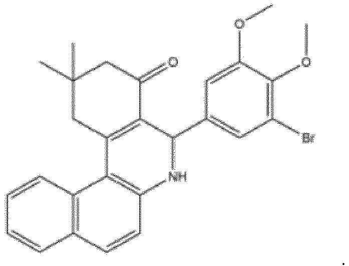
ALT-210 or a pharmaceutically acceptable salt thereof, with the chemical name of: 5-(3-((6-chlorobenzo[d][1,3]dioxol-5-yl)methoxy)phenyl)-2,2-dimethyl-2,3,5,6-tetrahydrobenzo[a]phenanthridin-4(1H)-one: 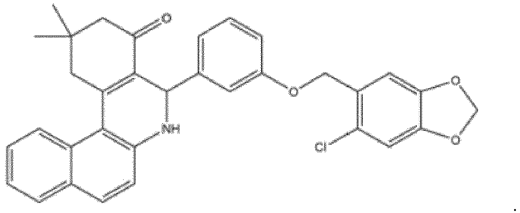
ALT-211 or a pharmaceutically acceptable salt thereof, with the chemical name of: 5-(7-methoxybenzo[d][1,3]dioxol-5-yl)-2,2-dimethyl-2,3,5,6-tetrahydrobenzo[a]phenanthridin-4(1H)-one: 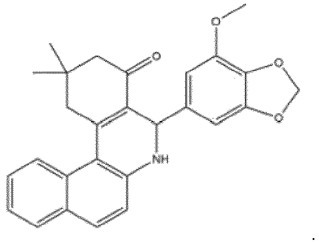
ALT-302 or a pharmaceutically acceptable salt thereof, with the chemical name of: 5-(4-methoxyphenyl)-2,2-dimethyl-2,3,5,6-tetrahydrobenzo[a]phenanthridin-4(1H)-one: 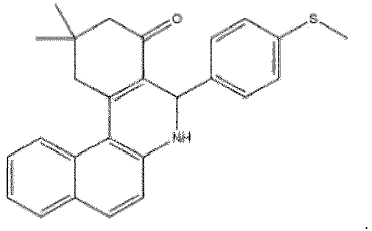
ALT-306 or a pharmaceutically acceptable salt thereof, with the chemical name of: 2,2-dimethyl-5-(p-tolyl)-2,3,5,6-tetrahydrobenzo[a]phenanthridin-4(1H)-one: 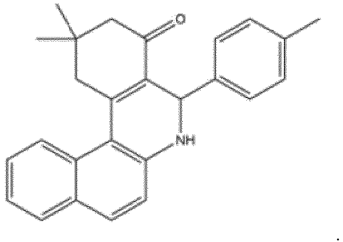
ALT-307 or a pharmaceutically acceptable salt thereof, with the chemical name of: 5-(3-bromo-4-methoxyphenyl)-2,2-dimethyl-2,3,5,6-tetrahydrobenzo[a]phenanthridin-4(1H)-one: 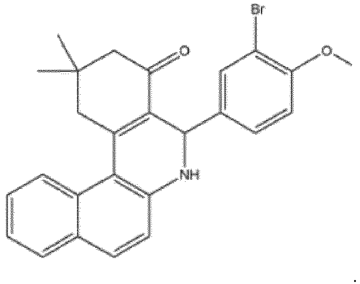
ALT-311 or a pharmaceutically acceptable salt thereof, with the chemical name of: 2,2-dimethyl-5-(4-(methylthio)phenyl)-2,3,5,6-tetrahydrobenzo[a]phenanthridin-4(1H)-one: 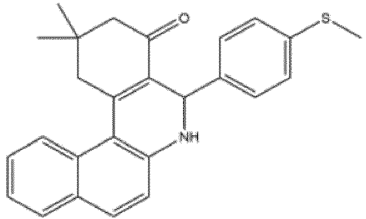
ALT-318 or a pharmaceutically acceptable salt thereof, with the chemical name of: 5-(4-(diethylamino)phenyl)-2,2-dimethyl-2,3,5,6-tetrahydrobenzo[a]phenanthridin-4(1H)-one: 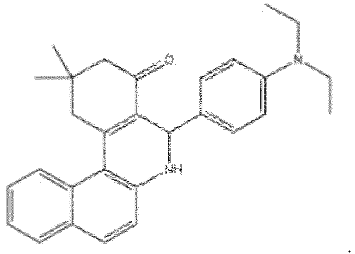
ALT-322 or a pharmaceutically acceptable salt thereof, with the chemical name of: 2,2-dimethyl-5-(4-(4-methylpiperidin-1-yl)-3-nitrophenyl)-2,3,5,6-tetrahydrobenzo[a]phenanthridin-4(1H)-one: 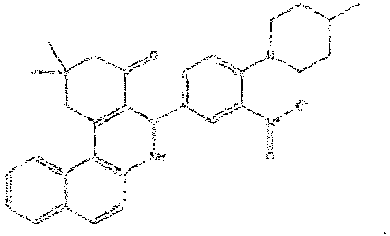
ALT-324 or a pharmaceutically acceptable salt thereof, with the chemical name of: 2,2-dimethyl-5-(4-((1-methyl-1H-imidazol-2-yl)thio)-3-nitrophenyl)-2,3,5,6-tetrahydrobenzo[a]phenanthridin-4(1H)-one: 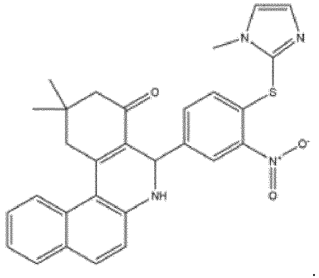
ALT-402 or a pharmaceutically acceptable salt thereof, with the chemical name of: 5-(3-iodo-5-methoxy-4-(prop-2-yn-1-yloxy)phenyl)-2,2-dimethyl-2,3,5,6-tetrahydrobenzo[a]phenanthridin-4(1H)-one: 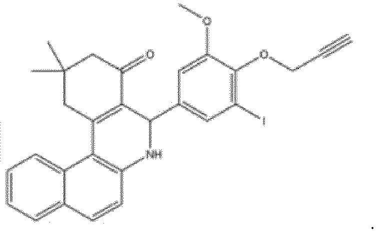
ALT-404 or a pharmaceutically acceptable salt thereof, with the chemical name of: 8-(4-(diethylamino)phenyl)-11,11-dimethyl-8,10,11,12-tetrahydrobenzo[a][4,7]phenanthrolin-9(7H)-one: 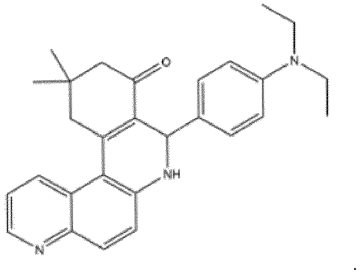
ALT-406 or a pharmaceutically acceptable salt thereof, with the chemical name of: ethyl 2-(2-chloro-6-methoxy-4-(4-oxo-1,2,3,4,5,6-hexahydrobenzo[a]phenanthridin-5-yl)phenoxy)acetate: 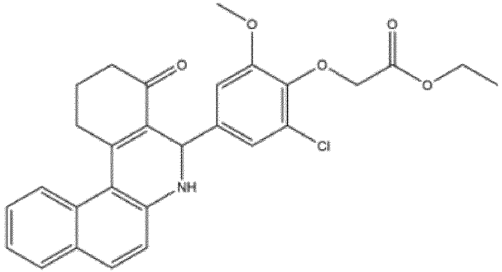
ALT-409 or a pharmaceutically acceptable salt thereof, with the chemical name of: 5-(4-(allyloxy)-3-methoxyphenyl)-2,2-dimethyl-2,3,5,6-tetrahydrobenzo[a]phenanthridin-4(1H)-one: 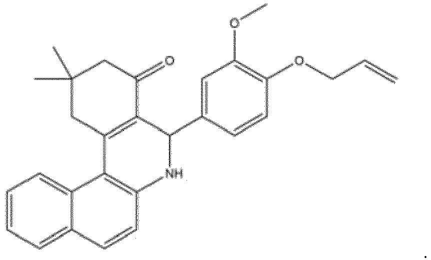
ALT-410 or a pharmaceutically acceptable salt thereof, with the chemical name of: 5-(4-propoxyphenyl)-2,3,5,6-tetrahydrobenzo[a]phenanthridin-4(1H)-one: 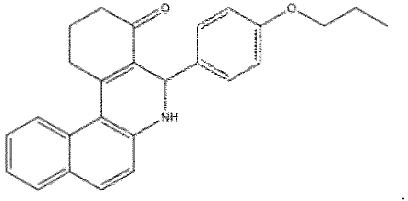
ALT-413 or a pharmaceutically acceptable salt thereof, with the chemical name of: 2-(4-(2,2-dimethyl-4-oxo-1,2,3,4,5,6-hexahydrobenzo[a]phenanthridin-5-yl)-2-methoxyphenoxy)acetic acid: 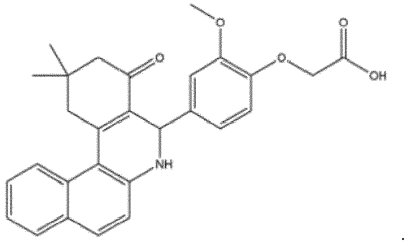
ALT-414 or a pharmaceutically acceptable salt thereof, with the chemical name of: 2-(4-(2,2-dimethyl-4-oxo-1,2,3,4,5,6-hexahydrobenzo[a]phenanthridin-5-yl)-2-methoxyphenoxy)acetic acid:

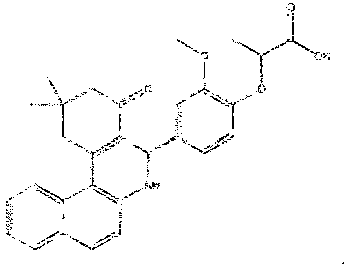

In some embodiments, the compound of Formula (II), including an analogue, derivative, or pharmaceutically acceptable salt thereof, such as a compound referred to herein as ALT-59, ALT-212, ALT-215, ALT-308, ALT-309, ALT-408, ALT-411, ALT-110, ALT-201, ALT-202, ALT-204, ALT-207, ALT-208, ALT-210, ALT-211, ALT-302, ALT-306, ALT-307, ALT-311, ALT-318, ALT-322, ALT-324, ALT-402, ALT-404, ALT-406, ALT-409, ALT-410, ALT-413, or ALT-414 is formulated as a pharmaceutical composition for use in the treatment of a disease or condition associated with TDP-43 toxicity, such as cystic fibrosis or neurodegeneration, including, amyotrophic lateral sclerosis (ALS), frontotemporal dementia (FTD), Alzheimer's disease, hippocampal sclerosis of aging (HS-Aging), chronic traumatic encephalopathy, and Parkinson's disease. In some embodiments, the pharmaceutical composition comprising a compound of Formula (II), or an analogue, derivative, or pharmaceutically acceptable salt thereof inhibits, delays, treats, prevents, or ameliorates a disease or condition associated with TDP-43 toxicity.

In some embodiments, the compound for use in the treatment of a disease or condition associated with TDP-43 toxicity includes a compound of Formula (III) or a pharmaceutically acceptable salt thereof, wherein the compound of Formula (III) or a pharmaceutically acceptable salt thereof is: 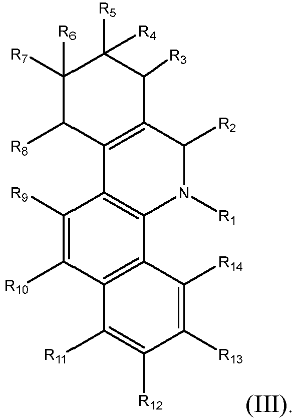 wherein R₁ is H, OH, or lower alkyl; R₂ is an optionally substituted aromatic ring of four, five, or six carbons, wherein the aromatic ring is carbocyclic or heterocyclic, and wherein each position on the aromatic ring is independently H, halo, lower alkyl, OH, lower alkoxy, NH₂, lower alkylamino, di(lower alkyl)amino, SH, lower alkylthio, NO₂, or two residues together form a heterocyclic ring; R₃ and R₈ are each independently H, lower alkyl, =O, =S, OH, NH₂, aryl, or aralkyl, where aryl and aralkyl are substituted with 0-3 moieties selected from the group consisting of halo, OH, NH₂, lower alkyl, lower alkoxy, SH, lower alkylthio, and lower alkylamino; R₄, R₅, R₆, and R₇ are each independently H, lower alkyl, OH, NH₂, aryl, or aralkyl, where aryl and aralkyl are substituted with 0-3 moieties selected from the group consisting of halo, OH, NH₂, lower alkyl, lower alkoxy, SH, lower alkylthio, and lower alkylamino; R₉, R₁₀, R₁₁, R₁₂, R₁₃, and R₁₄ are each independently H, halo, lower alkyl, OH, lower alkoxy, or NO₂; and wherein any one of the carbon atoms on any one of fused rings of Formula (III) is optionally replaced with a nitrogen atom.

Some embodiments relate to analogues or derivatives of the compound of Formula (III), wherein the compound of Formula (III), an analogue, derivative, or pharmaceutically acceptable salt thereof, including any of the compounds specifically disclosed herein, is used in the treatment of a disease or condition associated with TDP-43 toxicity, which include, for example, cystic fibrosis and a neurodegenerative disease. Examples of derivatives or analogues of the compound of Formula (III) include the following compounds:
ALT-108 or a pharmaceutically acceptable salt thereof, with the chemical name of: 6-(3-bromo-4-methoxyphenyl)-6,8,9,10-tetrahydrobenzo[c]phenanthridin-7(5H)-one: 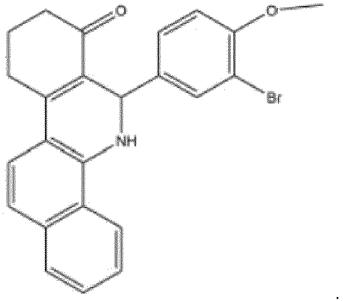
ALT-317 or a pharmaceutically acceptable salt thereof, with the chemical name of: 6-(3,4-dihydroxyphenyl)-6,8,9,10-tetrahydrobenzo[c]phenanthridin-7(5H)-one: 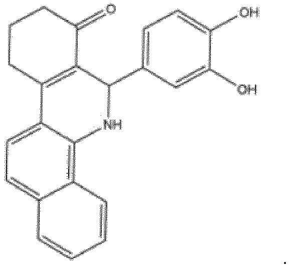
ALT-333 or a pharmaceutically acceptable salt thereof, with the chemical name of: 9,9-dimethyl-6-(p-tolyl)-6,8,9,10-tetrahydrobenzo[c]phenanthridin-7(5H)-one: 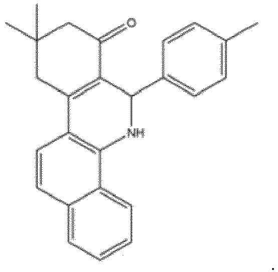
ALT-403 or a pharmaceutically acceptable salt thereof, with the chemical name of: 6-(benzo[d][1,3]dioxol-5-yl)-6,8,9,10-tetrahydrobenzo[c][1,7]phenanthrolin-7(5H)-one:

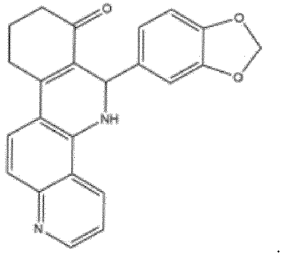

In some embodiments, the compound of Formula (III), including an analogue, derivative, or pharmaceutically acceptable salt thereof, such as a compound referred to herein as ALT-108, ALT-317, ALT-333, or ALT-403 is formulated as a pharmaceutical composition for use in the treatment of a disease or condition associated with TDP-43 toxicity, such as cystic fibrosis or neurodegeneration, including, amyotrophic lateral sclerosis (ALS), frontotemporal dementia (FTD), Alzheimer's disease, hippocampal sclerosis of aging (HS-Aging), chronic traumatic encephalopathy, and Parkinson's disease. In some embodiments, the pharmaceutical composition comprising a compound of Formula (III), or an analogue, derivative, or pharmaceutically acceptable salt thereof inhibits, delays, treats, prevents, or ameliorates a disease or condition associated with TDP-43 toxicity.

In some embodiments, the compound for use in the treatment of a disease or condition associated with TDP-43 toxicity includes a compound of Formula (IV) or a pharmaceutically acceptable salt thereof, wherein the compound of Formula (IV) or a pharmaceutically acceptable salt thereof is: 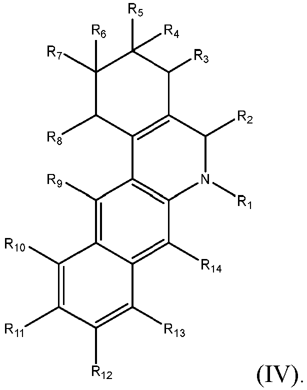 wherein R₁ is H, OH, or lower alkyl; R₂ is an optionally substituted aromatic ring of four, five, or six carbons, wherein the aromatic ring is carbocyclic or heterocyclic, and wherein each position on the aromatic ring is independently H, halo, lower alkyl, OH, lower alkoxy, NH₂, lower alkylamino, di(lower alkyl)amino, SH, lower alkylthio, NO₂, or two residues together form a heterocyclic ring; R₃ and R₈ are each independently H, lower alkyl, =O, =S, OH, NH₂, aryl, or aralkyl, where aryl and aralkyl are substituted with 0-3 moieties selected from the group consisting of halo, OH, NH₂, lower alkyl, lower alkoxy, SH, lower alkylthio, and lower alkylamino; R₄, R₅, R₆, and R₇ are each independently H, lower alkyl, OH, NH₂, aryl, or aralkyl, where aryl and aralkyl are substituted with 0-3 moieties selected from the group consisting of halo, OH, NH₂, lower alkyl, lower alkoxy, SH, lower alkylthio, and lower alkylamino; R₉, R₁₀, R₁₁, R₁₂, R₁₃, and R₁₄ are each independently H, halo, lower alkyl, OH, lower alkoxy, or NO₂; and wherein any one of the carbon atoms on any one of fused rings of Formula (IV) is optionally replaced with a nitrogen atom.

Some embodiments relate to analogues or derivatives of the compound of Formula (IV), wherein the compound of Formula (IV), an analogue, derivative, or pharmaceutically acceptable salt thereof, including any of the compounds specifically disclosed herein, is used in the treatment of a disease or condition associated with TDP-43 toxicity, which include, for example, cystic fibrosis and a neurodegenerative disease. An example of a derivative or analogue of the compound of Formula (IV) includes the following compound:
ALT-205 or a pharmaceutically acceptable salt thereof, with the chemical name of: 5-(benzo[d][1,3]dioxol-5-yl)-2,2-dimethyl-2,3,5,6-tetrahydrobenzo[b]phenanthridin-4(1H)-one:

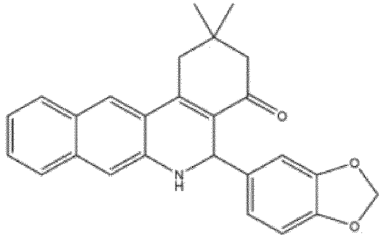

In some embodiments, the compound of Formula (IV), including an analogue, derivative, or pharmaceutically acceptable salt thereof, such as a compound referred to herein as ALT-205 is formulated as a pharmaceutical composition for use in the treatment of a disease or condition associated with TDP-43 toxicity, such as cystic fibrosis or neurodegeneration, including, amyotrophic lateral sclerosis (ALS), frontotemporal dementia (FTD), Alzheimer's disease, hippocampal sclerosis of aging (HS-Aging), chronic traumatic encephalopathy, and Parkinson's disease. In some embodiments, the pharmaceutical composition comprising a compound of Formula (IV), or an analogue, derivative, or pharmaceutically acceptable salt thereof inhibits, delays, treats, prevents, or ameliorates a disease or condition associated with TDP-43 toxicity.

In some embodiments, the compound of any one of Formulas (I), (II), (III), or (IV) or an analogue, derivative, or pharmaceutically acceptable salt thereof, includes a compound as disclosed in any one of U.S. Patent No. 6,800,634, or U.S. Patent Publication Nos. 2012/0220610 or 2017/0362221, Thus, compounds provided herein include any of the compounds described herein, any of the compounds of Formulas (I), (II), (III), and (IV) described herein, and any of the compounds described and disclosed in each of these references.

In some embodiments, the compound of any one of Formulas (I), (II), (III), or (IV) or an analogue, derivative, or pharmaceutically acceptable salt thereof, including any of the compounds specifically disclosed herein, is prepared with a pharmaceutically acceptable carrier that facilitates the incorporation of a compound into a product formulation and/or that facilitates delivery of the compound of any one of Formulas (I), (II), (III), or (IV) or an analogue, derivative, or pharmaceutically acceptable salt thereof, including any of the compounds specifically disclosed herein, to a cell or tissue for treatment. In some embodiments, the pharmaceutical composition including a compound of any one of Formulas (I), (II), (III), or (IV) including any of the compounds specifically disclosed herein, may include a compound of any one of Formulas (I), (II), (III), or (IV) at least one pharmaceutically acceptable carrier, and/or at least one excipient. In some embodiments, the at least one excipient may be a binder, a disintegrant, a surfactant, or a stabilizer.

In some embodiments, the compound of Formula (I) is a compound of any one of Formula (V), (VI), (VII), or (VIII). In some embodiments, the compound for use in the treatment of a disease or condition associated with TDP-43 toxicity includes a compound of Formula (V) or a pharmaceutically acceptable salt thereof, wherein the compound of Formula (V) or a pharmaceutically acceptable salt thereof is: 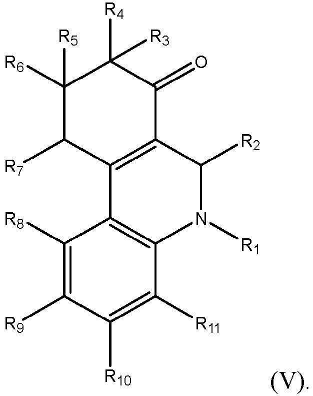 wherein R₁ is H, OH, or lower alkyl; R₂ is an optionally substituted aromatic ring of four, five, or six carbons, wherein the aromatic ring is carbocyclic or heterocyclic, and wherein each position on the aromatic ring is independently H, halo, lower alkyl, OH, lower alkoxy, NH₂, lower alkylamino, di(lower alkyl)amino, SH, lower alkylthio, NO₂, or two residues together form a heterocyclic ring; R₃, R₄, R₅, R₆, and R₇ are each independently H, lower alkyl, OH, NH₂, aryl, or aralkyl, where aryl and aralkyl are substituted with 0-3 moieties selected from the group consisting of halo, OH, NH₂, lower alkyl, lower alkoxy, SH, lower alkylthio, and lower alkylamino; wherein at least one of R₈ and R₉, R₉ and R₁₀, or R₁₀ and R₁₁ together form an optionally substituted benzene ring, wherein the benzene ring is optionally substituted with H, halo, lower alkyl, OH, lower alkoxy, or NO₂; and wherein any one of the carbon atoms on any one of fused rings of Formula (V) is optionally replaced with a nitrogen atom. In some embodiments, the oxygen at the ketone position of Formula (V) is replaced by a sulfur, forming a thioketone.

In some embodiments, the compound of Formula (V) is a compound of any one of Formulas (VI), (VII), or (VIII). In some embodiments, the compound of Formula (V) is any one of the compounds described herein, including any one of ALT-59, ALT-212, ALT-215, ALT-308, ALT-309, ALT-408, ALT-411, ALT-110, ALT-201, ALT-202, ALT-204, ALT-207, ALT-208, ALT-210, ALT-211, ALT-302, ALT-306, ALT-307, ALT-311, ALT-318, ALT-322, ALT-324, ALT-402, ALT-404, ALT-406, ALT-409, ALT-410, ALT-413, ALT-414, ALT-108, ALT-317, ALT-333, ALT-403, or ALT-205, as described in any of the embodiments herein in further detail. In some embodiments, the compound of Formula (V) is any one of the compounds described herein, including any one of ALT-59, ALT-212, ALT-215, ALT-308, ALT-309, ALT-408, ALT-411, ALT-110, ALT-202, ALT-204, ALT-207, ALT-208, ALT-210, ALT-211, ALT-302, ALT-306, ALT-307, ALT-311, ALT-318, ALT-322, ALT-324, ALT-402, ALT-404, ALT-406, ALT-409, ALT-410, ALT-413, ALT-414, ALT-108, ALT-317, ALT-333, ALT-403, or ALT-205, as described in any of the embodiments herein in further detail.

In some embodiments, the compound for use in the treatment of a disease or condition associated with TDP-43 toxicity includes a compound of Formula (VI) or a pharmaceutically acceptable salt thereof, wherein the compound of Formula (VI) or a pharmaceutically acceptable salt thereof is: 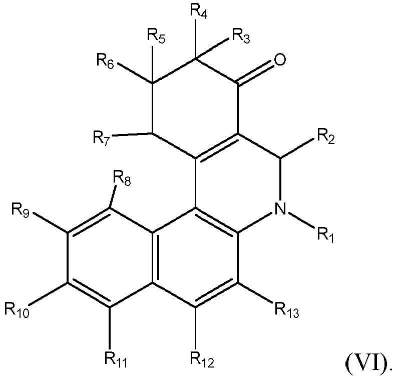 wherein R₁ is H, OH, or lower alkyl; R₂ is an optionally substituted aromatic ring of four, five, or six carbons, wherein the aromatic ring is carbocyclic or heterocyclic, and wherein each position on the aromatic ring is independently H, halo, lower alkyl, OH, lower alkoxy, NH₂, lower alkylamino, di(lower alkyl)amino, SH, lower alkylthio, NO₂, or two residues together form a heterocyclic ring; R₃, R₄, R₅, R₆, and R₇ are each independently H, lower alkyl, OH, NH₂, aryl, or aralkyl, where aryl and aralkyl are substituted with 0-3 moieties selected from the group consisting of halo, OH, NH₂, lower alkyl, lower alkoxy, SH, lower alkylthio, and lower alkylamino; R₈, R₉, R₁₀, R₁₁, R₁₂, and R₁₃ are each independently H, halo, lower alkyl, OH, lower alkoxy, or NO₂; and wherein any one of the carbon atoms on any one of fused rings of Formula (VI) is optionally replaced with a nitrogen atom. In some embodiments, the oxygen at the ketone position of Formula (VI) is replaced by a sulfur, forming a thioketone.

Some embodiments relate to analogues or derivatives of the compound of Formula (VI), wherein the compound of Formula (VI), an analogue, derivative, or pharmaceutically acceptable salt thereof, including any of the compounds specifically disclosed herein, is used in the treatment of a disease or condition associated with TDP-43 toxicity, which include, for example, cystic fibrosis and a neurodegenerative disease. Examples of derivatives or analogues of the compound of Formula (VI) include the following compounds:
ALT-59 or a pharmaceutically acceptable salt thereof, with the chemical name of: 5-(3-bromo-4-(dimethylamino)phenyl)-2,2-dimethyl-2,3,5,6-tetrahydrobenzo[a]phenanthridin-4(1H)-one: 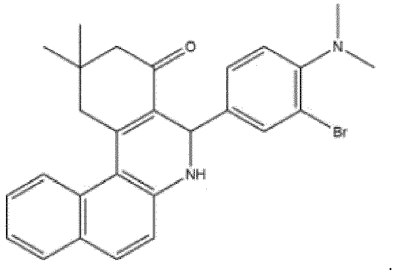
ALT-212 or a pharmaceutically acceptable salt thereof, with the chemical name of: 2,2-dimethyl-5-(4-(trifluoromethyl)phenyl)-2,3,5,6-tetrahydrobenzo[a]phenanthridin-4(1H)-one: 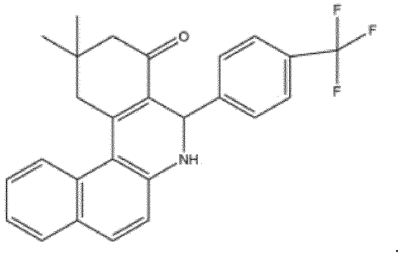
ALT-215 or a pharmaceutically acceptable salt thereof, with the chemical name of: 2,2-dimethyl-5-(3-(trifluoromethyl)phenyl)-2,3,5,6-tetrahydrobenzo[a]phenanthridin-4(1H)-one: 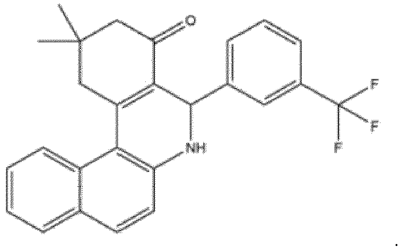
ALT-308 or a pharmaceutically acceptable salt thereof, with the chemical name of: 5-(4-(tert-butyl)phenyl)-2,2-dimethyl-2,3,5,6-tetrahydrobenzo[a]phenanthridin-4(1H)-one: 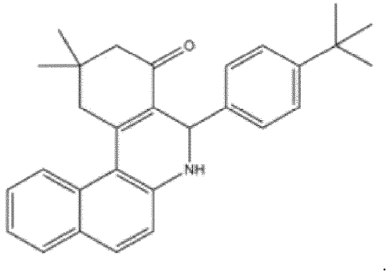
ALT-309 or a pharmaceutically acceptable salt thereof, with the chemical name of: 5-(4-isopropylphenyl)-2,2-dimethyl-2,3,5,6-tetrahydrobenzo[a]phenanthridin-4(1H)-one: 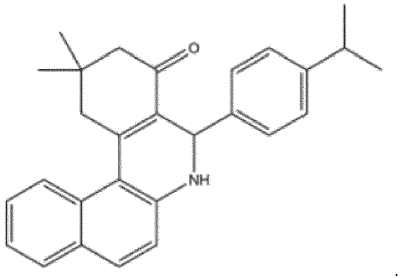
ALT-408 or a pharmaceutically acceptable salt thereof, with the chemical name of: 2-methoxy-4-(4-oxo-1,2,3,4,5,6-hexahydrobenzo[a]phenanthridin-5-yl)phenyl butyrate: 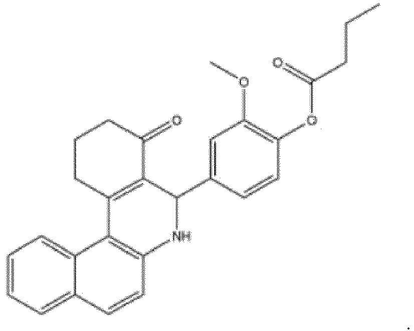
ALT-411 or a pharmaceutically acceptable salt thereof, with the chemical name of: methyl 2-(4-(4-oxo-1,2,3,4,5,6-hexahydrobenzo[a]phenanthridin-5-yl)phenoxy)acetate: 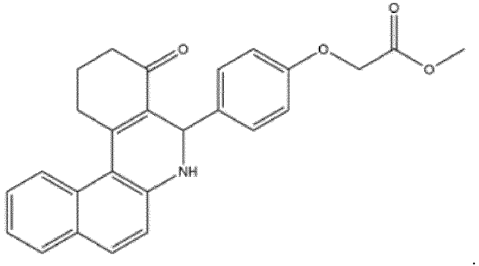
ALT-110 or a pharmaceutically acceptable salt thereof, with the chemical name of: 2,2-dimethyl-5-(3-(1,1,2,2-tetrafluoroethoxy)phenyl)-2,3,5,6-tetrahydrobenzo[a]phenanthridin-4(1H)-one: 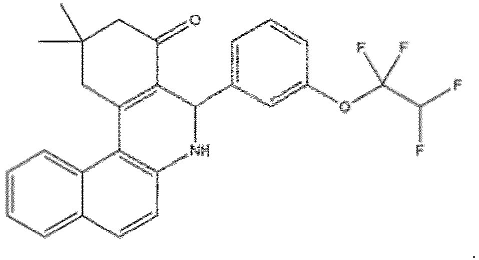
ALT-202 or a pharmaceutically acceptable salt thereof, with the chemical name of: 5-(5-bromothiophen-2-yl)-2,2-dimethyl-2,3,5,6-tetrahydrobenzo[a]phenanthridin-4(1H)-one: 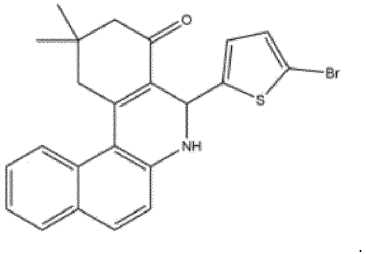
ALT-204 or a pharmaceutically acceptable salt thereof, with the chemical name of: 5-(3,4-dichlorophenyl)-2,2-dimethyl-2,3,5,6-tetrahydrobenzo[a]phenanthridin-4(1H)-one: 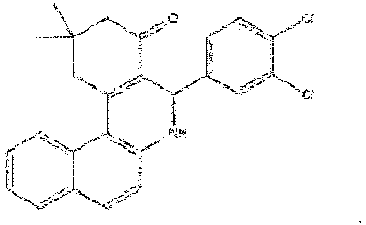
ALT-207 or a pharmaceutically acceptable salt thereof, with the chemical name of: 5-(4-ethoxy-3-methoxyphenyl)-2,2-dimethyl-2,3,5,6-tetrahydrobenzo[a]phenanthridin-4(1H)-one: 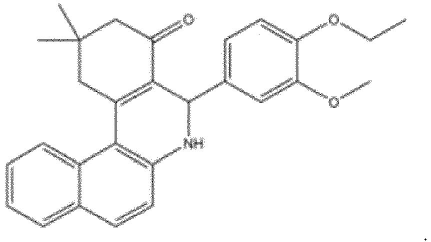
ALT-208 or a pharmaceutically acceptable salt thereof, with the chemical name of: 5-(3-bromo-4,5-dimethoxyphenyl)-2,2-dimethyl-2,3,5,6-tetrahydrobenzo[a]phenanthridin-4(1H)-one: 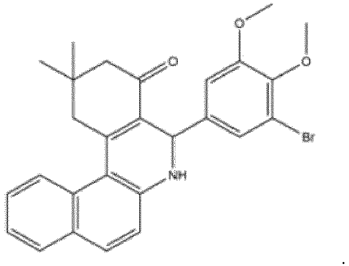
ALT-210 or a pharmaceutically acceptable salt thereof, with the chemical name of: 5-(3-((6-chlorobenzo[d][1,3]dioxol-5-yl)methoxy)phenyl)-2,2-dimethyl-2,3,5,6-tetrahydrobenzo[a]phenanthridin-4(1H)-one: 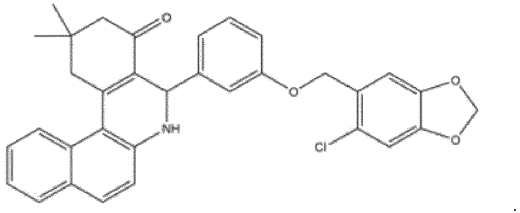
ALT-211 or a pharmaceutically acceptable salt thereof, with the chemical name of: 5-(7-methoxybenzo[d][1,3]dioxol-5-yl)-2,2-dimethyl-2,3,5,6-tetrahydrobenzo[a]phenanthridin-4(1H)-one: 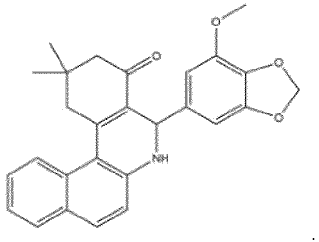
ALT-302 or a pharmaceutically acceptable salt thereof, with the chemical name of: 5-(4-methoxyphenyl)-2,2-dimethyl-2,3,5,6-tetrahydrobenzo[a]phenanthridin-4(1H)-one: 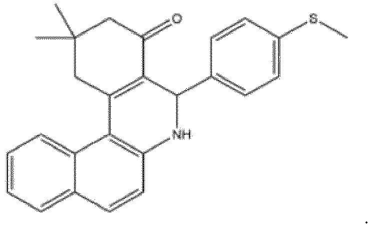
ALT-306 or a pharmaceutically acceptable salt thereof, with the chemical name of: 2,2-dimethyl-5-(p-tolyl)-2,3,5,6-tetrahydrobenzo[a]phenanthridin-4(1H)-one: 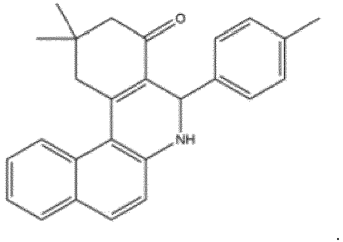
ALT-307 or a pharmaceutically acceptable salt thereof, with the chemical name of: 5-(3-bromo-4-methoxyphenyl)-2,2-dimethyl-2,3,5,6-tetrahydrobenzo[a]phenanthridin-4(1H)-one: 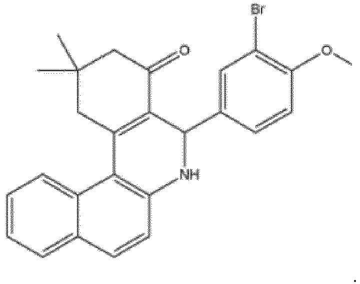
ALT-311 or a pharmaceutically acceptable salt thereof, with the chemical name of: 2,2-dimethyl-5-(4-(methylthio)phenyl)-2,3,5,6-tetrahydrobenzo[a]phenanthridin-4(1H)-one: 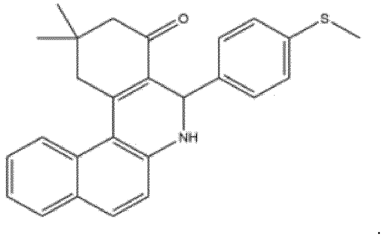
ALT-318 or a pharmaceutically acceptable salt thereof, with the chemical name of: 5-(4-(diethylamino)phenyl)-2,2-dimethyl-2,3,5,6-tetrahydrobenzo[a]phenanthridin-4(1H)-one: 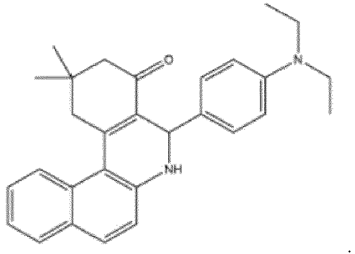
ALT-322 or a pharmaceutically acceptable salt thereof, with the chemical name of: 2,2-dimethyl-5-(4-(4-methylpiperidin-1-yl)-3-nitrophenyl)-2,3,5,6-tetrahydrobenzo[a]phenanthridin-4(1H)-one: 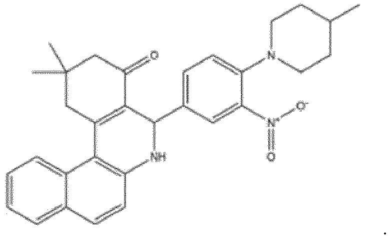
ALT-324 or a pharmaceutically acceptable salt thereof, with the chemical name of: 2,2-dimethyl-5-(4-((1-methyl-1H-imidazol-2-yl)thio)-3-nitrophenyl)-2,3,5,6-tetrahydrobenzo[a]phenanthridin-4(1H)-one: 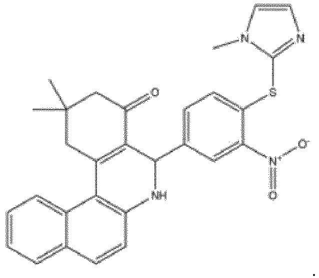
ALT-402 or a pharmaceutically acceptable salt thereof, with the chemical name of: 5-(3-iodo-5-methoxy-4-(prop-2-yn-1-yloxy)phenyl)-2,2-dimethyl-2,3,5,6-tetrahydrobenzo[a]phenanthridin-4(1H)-one: 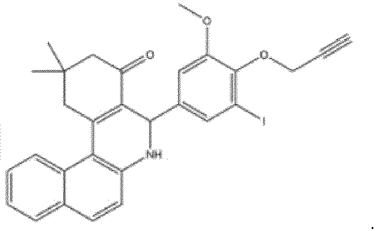
ALT-404 or a pharmaceutically acceptable salt thereof, with the chemical name of: 8-(4-(diethylamino)phenyl)-11,11-dimethyl-8,10,11,12-tetrahydrobenzo[a][4,7]phenanthrolin-9(7H)-one: 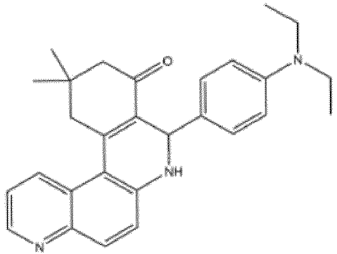
ALT-406 or a pharmaceutically acceptable salt thereof, with the chemical name of: ethyl 2-(2-chloro-6-methoxy-4-(4-oxo-1,2,3,4,5,6-hexahydrobenzo[a]phenanthridin-5-yl)phenoxy)acetate: 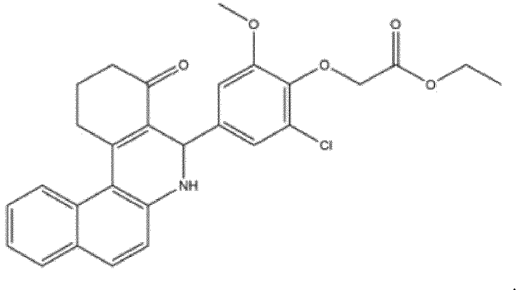
ALT-409 or a pharmaceutically acceptable salt thereof, with the chemical name of: 5-(4-(allyloxy)-3-methoxyphenyl)-2,2-dimethyl-2,3,5,6-tetrahydrobenzo[a]phenanthridin-4(1H)-one: 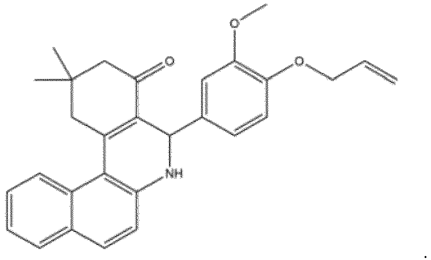
ALT-410 or a pharmaceutically acceptable salt thereof, with the chemical name of: 5-(4-propoxyphenyl)-2,3,5,6-tetrahydrobenzo[a]phenanthridin-4(1H)-one: 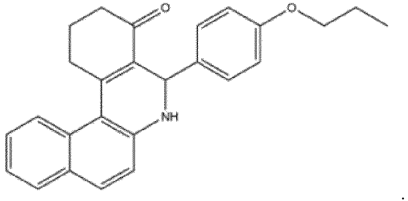
ALT-413 or a pharmaceutically acceptable salt thereof, with the chemical name of: 2-(4-(2,2-dimethyl-4-oxo-1,2,3,4,5,6-hexahydrobenzo[a]phenanthridin-5-yl)-2-methoxyphenoxy)acetic acid: 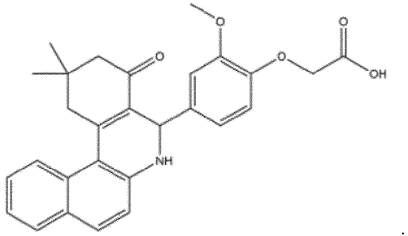
ALT-414 or a pharmaceutically acceptable salt thereof, with the chemical name of: 2-(4-(2,2-dimethyl-4-oxo-1,2,3,4,5,6-hexahydrobenzo[a]phenanthridin-5-yl)-2-methoxyphenoxy)acetic acid:

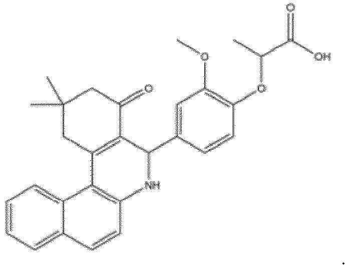

In some embodiments, the compound of Formula (VI), including an analogue, derivative, or pharmaceutically acceptable salt thereof, such as a compound referred to herein as ALT-59, ALT-212, ALT-215, ALT-308, ALT-309, ALT-408, ALT-411, ALT-110, ALT-201, ALT-202, ALT-204, ALT-207, ALT-208, ALT-210, ALT-211, ALT-302, ALT-306, ALT-307, ALT-311, ALT-318, ALT-322, ALT-324, ALT-402, ALT-404, ALT-406, ALT-409, ALT-410, ALT-413, or ALT-414 is formulated as a pharmaceutical composition for use in the treatment of a disease or condition associated with TDP-43 toxicity, such as cystic fibrosis or neurodegeneration, including, amyotrophic lateral sclerosis (ALS), frontotemporal dementia (FTD), Alzheimer's disease, hippocampal sclerosis of aging (HS-Aging), chronic traumatic encephalopathy, and Parkinson's disease. In some embodiments, the compound of Formula (VI), including an analogue, derivative, or pharmaceutically acceptable salt thereof, such as a compound referred to herein as ALT-59, ALT-212, ALT-215, ALT-308, ALT-309, ALT-408, ALT-411, ALT-110, ALT-202, ALT-204, ALT-207, ALT-208, ALT-210, ALT-211, ALT-302, ALT-306, ALT-307, ALT-311, ALT-318, ALT-322, ALT-324, ALT-402, ALT-404, ALT-406, ALT-409, ALT-410, ALT-413, or ALT-414 is formulated as a pharmaceutical composition for use in the treatment of a disease or condition associated with TDP-43 toxicity, such as cystic fibrosis or neurodegeneration, including, amyotrophic lateral sclerosis (ALS), frontotemporal dementia (FTD), Alzheimer's disease, hippocampal sclerosis of aging (HS-Aging), chronic traumatic encephalopathy, and Parkinson's disease. In some embodiments, the pharmaceutical composition comprising a compound of Formula (VI), or an analogue, derivative, or pharmaceutically acceptable salt thereof inhibits, delays, treats, prevents, or ameliorates a disease or condition associated with TDP-43 toxicity.

In some embodiments, the compound for use in the treatment of a disease or condition associated with TDP-43 toxicity includes a compound of Formula (VII) or a pharmaceutically acceptable salt thereof, wherein the compound of Formula (VII) or a pharmaceutically acceptable salt thereof is: 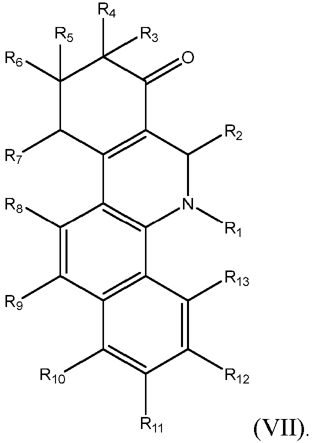 wherein R₁ is H, OH, or lower alkyl; R₂ is an optionally substituted aromatic ring of four, five, or six carbons, wherein the aromatic ring is carbocyclic or heterocyclic, and wherein each position on the aromatic ring is independently H, halo, lower alkyl, OH, lower alkoxy, NH₂, lower alkylamino, di(lower alkyl)amino, SH, lower alkylthio, NO₂, or two residues together form a heterocyclic ring; R₃, R₄, R₅, R₆, and R₇ are each independently H, lower alkyl, OH, NH₂, aryl, or aralkyl, where aryl and aralkyl are substituted with 0-3 moieties selected from the group consisting of halo, OH, NH₂, lower alkyl, lower alkoxy, SH, lower alkylthio, and lower alkylamino; R₈, R₉, R₁₀, R₁₁, R₁₂, and R₁₃ are each independently H, halo, lower alkyl, OH, lower alkoxy, or NO₂; and wherein any one of the carbon atoms on any one of fused rings of Formula (VII) is optionally replaced with a nitrogen atom. In some embodiments, the oxygen at the ketone position of Formula (VII) is replaced by a sulfur, forming a thioketone.

Some embodiments relate to analogues or derivatives of the compound of Formula (VII), wherein the compound of Formula (VII), an analogue, derivative, or pharmaceutically acceptable salt thereof, including any of the compounds specifically disclosed herein, is used in the treatment of a disease or condition associated with TDP-43 toxicity, which include, for example, cystic fibrosis and a neurodegenerative disease. Examples of derivatives or analogues of the compound of Formula (VII) include the following compounds:
ALT-108 or a pharmaceutically acceptable salt thereof, with the chemical name of: 6-(3-bromo-4-methoxyphenyl)-6,8,9,10-tetrahydrobenzo[c]phenanthridin-7(5H)-one: 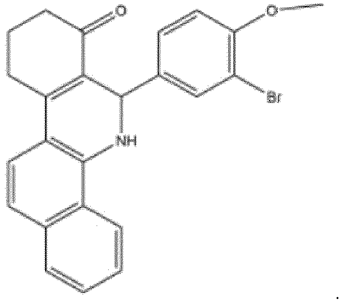
ALT-317 or a pharmaceutically acceptable salt thereof, with the chemical name of: 6-(3,4-dihydroxyphenyl)-6,8,9,10-tetrahydrobenzo[c]phenanthridin-7(5H)-one: 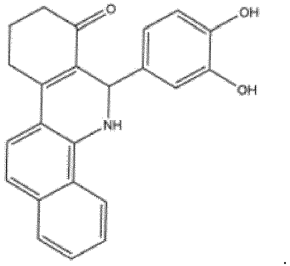
ALT-333 or a pharmaceutically acceptable salt thereof, with the chemical name of: 9,9-dimethyl-6-(p-tolyl)-6,8,9,10-tetrahydrobenzo[c]phenanthridin-7(5H)-one: 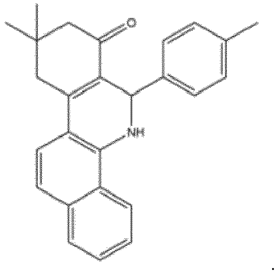
ALT-403 or a pharmaceutically acceptable salt thereof, with the chemical name of: 6-(benzo[d][1,3]dioxol-5-yl)-6,8,9,10-tetrahydrobenzo[c][1,7]phenanthrolin-7(5H)-one:

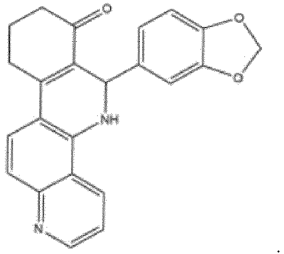

In some embodiments, the compound of Formula (VII), including an analogue, derivative, or pharmaceutically acceptable salt thereof, such as a compound referred to herein as ALT-108, ALT-317, ALT-333, or ALT-403 is formulated as a pharmaceutical composition for use in the treatment of a disease or condition associated with TDP-43 toxicity, such as cystic fibrosis or neurodegeneration, including, amyotrophic lateral sclerosis (ALS), frontotemporal dementia (FTD), Alzheimer's disease, hippocampal sclerosis of aging (HS-Aging), chronic traumatic encephalopathy, and Parkinson's disease. In some embodiments, the pharmaceutical composition comprising a compound of Formula (VII), or an analogue, derivative, or pharmaceutically acceptable salt thereof inhibits, delays, treats, prevents, or ameliorates a disease or condition associated with TDP-43 toxicity.

In some embodiments, the compound for use in the treatment of a disease or condition associated with TDP-43 toxicity includes a compound of Formula (VIII) or a pharmaceutically acceptable salt thereof, wherein the compound of Formula (VIII) or a pharmaceutically acceptable salt thereof is: 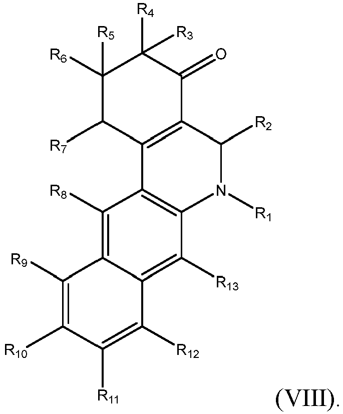 wherein R₁ is H, OH, or lower alkyl; R₂ is an optionally substituted aromatic ring of four, five, or six carbons, wherein the aromatic ring is carbocyclic or heterocyclic, and wherein each position on the aromatic ring is independently H, halo, lower alkyl, OH, lower alkoxy, NH₂, lower alkylamino, di(lower alkyl)amino, SH, lower alkylthio, NO₂, or two residues together form a heterocyclic ring; R₃, R₄, R₅, R₆, and R₇ are each independently H, lower alkyl, OH, NH₂, aryl, or aralkyl, where aryl and aralkyl are substituted with 0-3 moieties selected from the group consisting of halo, OH, NH₂, lower alkyl, lower alkoxy, SH, lower alkylthio, and lower alkylamino; R₈, R₉, R₁₀, R₁₁, R₁₂, and R₁₃ are each independently H, halo, lower alkyl, OH, lower alkoxy, or NO₂; and wherein any one of the carbon atoms on any one of fused rings of Formula (VIII) is optionally replaced with a nitrogen atom. In some embodiments, the oxygen at the ketone position of Formula (VIII) is replaced by a sulfur, forming a thioketone.

Some embodiments relate to analogues or derivatives of the compound of Formula (VIII), wherein the compound of Formula (VIII), an analogue, derivative, or pharmaceutically acceptable salt thereof, including any of the compounds specifically disclosed herein, is used in the treatment of a disease or condition associated with TDP-43 toxicity, which include, for example, cystic fibrosis and a neurodegenerative disease. An example of a derivative or analogue of the compound of Formula (VIII) includes the following compound:
ALT-205 or a pharmaceutically acceptable salt thereof, with the chemical name of: 5-(benzo[d][1,3]dioxol-5-yl)-2,2-dimethyl-2,3,5,6-tetrahydrobenzo[b]phenanthridin-4(1H)-one:

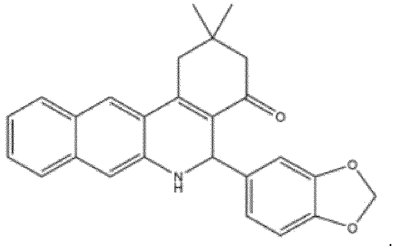

In some embodiments, the compound of Formula (VIII), including an analogue, derivative, or pharmaceutically acceptable salt thereof, such as a compound referred to herein as ALT-205 is formulated as a pharmaceutical composition for use in the treatment of a disease or condition associated with TDP-43 toxicity, such as cystic fibrosis or neurodegeneration, including, amyotrophic lateral sclerosis (ALS), frontotemporal dementia (FTD), Alzheimer's disease, hippocampal sclerosis of aging (HS-Aging), chronic traumatic encephalopathy, and Parkinson's disease. In some embodiments, the pharmaceutical composition comprising a compound of Formula (VIII), or an analogue, derivative, or pharmaceutically acceptable salt thereof inhibits, delays, treats, prevents, or ameliorates a disease or condition associated with TDP-43 toxicity.

In some embodiments, the compound of any one of Formulas (V), (VI), (VII), or (VIII) or an analogue, derivative, or pharmaceutically acceptable salt thereof, includes a compound as disclosed in any one of U.S. Patent No. 6,800,634, or U.S. Patent Publication Nos. 2012/0220610 or 2017/0362221, each of which is for the specific disclosure referenced herein. Thus, compounds provided herein include any of the compounds described herein, any of the compounds of Formulas (V), (VI), (VII), and (VIII) described herein, and any of the compounds described and disclosed in each of these references.

In some embodiments, the compound of any one of Formulas (V), (VI), (VII), or (VIII) or an analogue, derivative, or pharmaceutically acceptable salt thereof, including any of the compounds specifically disclosed herein, is prepared with a pharmaceutically acceptable carrier that facilitates the incorporation of a compound into a product formulation and/or that facilitates delivery of the compound of any one of Formulas (V), (VI), (VII), or (VIII) or an analogue, derivative, or pharmaceutically acceptable salt thereof, including any of the compounds specifically disclosed herein, to a cell or tissue for treatment. In some embodiments, the pharmaceutical composition including a compound of any one of Formulas (V), (VI), (VII), or (VIII) including any of the compounds specifically disclosed herein, may include a compound of any one of Formulas (V), (VI), (VII), or (VIII) at least one pharmaceutically acceptable carrier, and/or at least one excipient. In some embodiments, the at least one excipient may be a binder, a disintegrant, a surfactant, or a stabilizer.

The pharmaceutical compositions described herein may be formulated for oral, intranasal, or parenteral administration. Oral administration may include formulation of the compositions for administration to the oral cavity, including for administration to the digestive tract, the buccal lining, or the respiratory tract through the oral cavity, for example, formulation of the compositions as a solid or liquid formulation, such as a tablet, pill, capsule, pellet, dragee, gummy, powder, softgel, liquid, syrup, suspension, solution, or inhalable composition. Intranasal administration may include formulation for administration by the nasal cavity, and may include drops, spray, insufflation, or inhalable compositions. Parenteral administration may include, for example, intraperitoneal, infusion, intramuscular, subcutaneous, intradermal, or intravenous injection.

In some embodiments, the compositions described herein further include pharmaceutically acceptable carriers and excipients, depending on the desired delivery or mode of administration format.

### III. Methods of Treatment

Some embodiments relate to a pharmaceutical composition for use in methods of treating, ameliorating, inhibiting, preventing, or delaying a disease or condition associated with TDP-43 toxicity, such as cystic fibrosis or neurodegeneration, including, amyotrophic lateral sclerosis (ALS), frontotemporal dementia (FTD), Alzheimer's disease, hippocampal sclerosis of aging (HS-Aging), chronic traumatic encephalopathy, and Parkinson's disease. In some embodiments, the methods include administration of a compound of any one of Formula (I), (II), (III), or (IV) an analogue, derivative, or pharmaceutically acceptable salt thereof, including any of the compounds specifically disclosed herein, or a pharmaceutical composition comprising a compound of any one of Formula (I), (II), (III), or (IV) an analogue, derivative, or pharmaceutically acceptable salt thereof, including any of the compounds specifically disclosed herein, to a subject in need thereof.

In some embodiments, the compound of Formula (I) is a compound of any one of Formula (V), (VI), (VII), or (VIII). Some embodiments relate to a pharmaceutical composition for use in methods of treating, ameliorating, inhibiting, preventing, or delaying a disease or condition associated with TDP-43 toxicity, such as cystic fibrosis or neurodegeneration, including, amyotrophic lateral sclerosis (ALS), frontotemporal dementia (FTD), Alzheimer's disease, hippocampal sclerosis of aging (HS-Aging), chronic traumatic encephalopathy, and Parkinson's disease. In some embodiments, the methods include administration of a compound of any one of Formula (V), (VI), (VII), or (VIII) an analogue, derivative, or pharmaceutically acceptable salt thereof, including any of the compounds specifically disclosed herein, or a pharmaceutical composition comprising a compound of any one of Formula (V), (VI), (VII), or (VIII) an analogue, derivative, or pharmaceutically acceptable salt thereof, including any of the compounds specifically disclosed herein, to a subject in need thereof.

As used herein, a "subj ect" refers to an animal that is the object of treatment, inhibition, or amelioration, observation or experiment. "Animal" includes cold- and warm-blooded vertebrates and/or invertebrates such as fish, shellfish, or reptiles and, in particular, mammals. "Mammal" includes, without limitation, mice, rats, rabbits, guinea pigs, dogs, cats, sheep, goats, cows, horses, primates, such as monkeys, chimpanzees, and/or apes, and, in particular, humans. In some embodiments, the subject is human.

In some embodiments, the methods include a pharmaceutical composition for use in diagnosing a subject as suffering from a disease or condition associated with TDP-43 toxicity, such as cystic fibrosis or neurodegeneration, including, amyotrophic lateral sclerosis (ALS), frontotemporal dementia (FTD), Alzheimer's disease, hippocampal sclerosis of aging (HS-Aging), chronic traumatic encephalopathy, and Parkinson's disease. In some embodiments, diagnosing includes making a determination that a subject has or is likely to develop a disease or condition associated with TDP-43 toxicity. In some embodiments, diagnosing a subject equates to identifying a subject as having or likely to develop a disease or condition associated with TDP-43 toxicity.

In some embodiments, the methods include selecting the subject that has been diagnosed or that has been identified as having or as likely to develop a disease or condition associated with TDP-43 toxicity, such as cystic fibrosis or neurodegeneration, including, amyotrophic lateral sclerosis (ALS), frontotemporal dementia (FTD), Alzheimer's disease, hippocampal sclerosis of aging (HS-Aging), chronic traumatic encephalopathy, and Parkinson's disease, and administering to the subject a composition comprising a compound of any one of Formula (I), (II), (III), or (IV) an analogue, derivative, or pharmaceutically acceptable salt thereof, including any of the compounds specifically disclosed herein. In some embodiments, administration of a composition comprising a compound of any one of Formula (I), (II), (III), or (IV), an analogue, derivative, or pharmaceutically acceptable salt thereof, including any of the compounds specifically disclosed herein, to a subject in need ameliorates, treats, prevents, delays, inhibits, or slows the onset and/or development of the disease or condition associated with TDP-43 toxicity.

In some non-claimed embodiments, the methods include reducing or inhibiting TDP-43 in a subject suffering from TDP-43 toxicity. In some embodiments, the methods include selecting or identifying a subject having TDP-43 toxicity, and administering to said subject a composition comprising a compound of any one of Formula (I), (II), (III), or (IV), an analogue, derivative, or pharmaceutically acceptable salt thereof, including any of the compounds specifically disclosed herein. In some embodiments, administration of a composition comprising a compound of any one of Formula (I), (II), (III), or (IV), an analogue, derivative, or pharmaceutically acceptable salt thereof, including any of the compounds specifically disclosed herein, results in reduced levels of TDP-43 activity in the subject. Levels of TDP-43 activity may be reduced by an amount of greater than 5%, 10%, 15%, 20%, 25%, 30%, 35%, 40%, 45%, 50%, 55%, 60%, 65%, 70%, 75%, 80%, 85%, 90%, 95%, or 99% or in an amount within a range defined by any two of the aforementioned values. In some embodiments, administration of a composition comprising a compound of any one of Formula (I), (II), (III), or (IV), an analogue, derivative, or pharmaceutically acceptable salt thereof, including any of the compounds specifically disclosed herein, inhibits TDP-43 toxicity.

In some embodiments, the pharmaceutical composition comprising a compound of any one of Formula (I), (II), (III), or (IV), an analogue, derivative, or pharmaceutically acceptable salt thereof, including any of the compounds specifically disclosed herein, as disclosed herein may contain between 0.01 mg and 3000 mg of a compound of any one of Formula (I), (II), (III), or (IV), an analogue, derivative, or pharmaceutically acceptable salt thereof, including any of the compounds specifically disclosed herein.

In some embodiments, the compound of Formula (I) is a compound of any one of Formula (V), (VI), (VII), or (VIII). In some embodiments, the methods include selecting the subject that has been diagnosed or that has been identified as having or as likely to develop a disease or condition associated with TDP-43 toxicity, such as cystic fibrosis or neurodegeneration, including, amyotrophic lateral sclerosis (ALS), frontotemporal dementia (FTD), Alzheimer's disease, hippocampal sclerosis of aging (HS-Aging), chronic traumatic encephalopathy, and Parkinson's disease, and administering to the subject a composition comprising a compound of any one of Formula (V), (VI), (VII), or (VIII) an analogue, derivative, or pharmaceutically acceptable salt thereof, including any of the compounds specifically disclosed herein. In some embodiments, administration of a composition comprising a compound of any one of Formula (V), (VI), (VII), or (VIII), an analogue, derivative, or pharmaceutically acceptable salt thereof, including any of the compounds specifically disclosed herein, to a subject in need ameliorates, treats, prevents, delays, inhibits, or slows the onset and/or development of the disease or condition associated with TDP-43 toxicity.

In some non-claimed embodiments, the methods include reducing or inhibiting TDP-43 in a subject suffering from TDP-43 toxicity. In some embodiments, the methods include selecting or identifying a subject having TDP-43 toxicity, and administering to said subject a composition comprising a compound of any one of Formula (V), (VI), (VII), or (VIII), an analogue, derivative, or pharmaceutically acceptable salt thereof, including any of the compounds specifically disclosed herein. In some embodiments, administration of a composition comprising a compound of any one of Formula (V), (VI), (VII), or (VIII), an analogue, derivative, or pharmaceutically acceptable salt thereof, including any of the compounds specifically disclosed herein, results in reduced levels of TDP-43 activity in the subject. Levels of TDP-43 activity may be reduced by an amount of greater than 5%, 10%, 15%, 20%, 25%, 30%, 35%, 40%, 45%, 50%, 55%, 60%, 65%, 70%, 75%, 80%, 85%, 90%, 95%, or 99% or in an amount within a range defined by any two of the aforementioned values. In some embodiments, administration of a composition comprising a compound of any one of Formula (V), (VI), (VII), or (VIII), an analogue, derivative, or pharmaceutically acceptable salt thereof, including any of the compounds specifically disclosed herein, inhibits TDP-43 toxicity.

In some embodiments, the pharmaceutical composition comprising a compound of any one of Formula (V), (VI), (VII), or (VIII), an analogue, derivative, or pharmaceutically acceptable salt thereof, including any of the compounds specifically disclosed herein, as disclosed herein may contain between 0.01 mg and 3000 mg of a compound of any one of Formula (V), (VI), (VII), or (VIII), an analogue, derivative, or pharmaceutically acceptable salt thereof, including any of the compounds specifically disclosed herein.

As will be understood by those of skill in the art, in certain situations it may be necessary to administer the active ingredients disclosed herein in amounts that exceed, or even far exceed, the above-stated dosage ranges in order to effectively and aggressively treat particularly aggressive diseases, or it may be necessary to administer the active ingredients disclosed herein in amounts that are less than, or even significantly less than, the above-stated dosage ranges, for example for maintenance therapy.

Dosage amount and interval may be adjusted individually to provide plasma levels of the active ingredient that are sufficient to maintain the modulating effects, or minimal effective concentration (MEC). The MEC will vary for each active ingredient but can be estimated from *in vitro* data. Dosages necessary to achieve the MEC will depend on individual characteristics and route of administration. However, HPLC assays or bioassays can be used to determine plasma concentrations. Dosage intervals can also be determined using MEC value. Compositions should be administered using a regimen that maintains plasma levels above the MEC for 10-90% of the time, preferably between 30-90% and most preferably between 50-90%. In cases of local administration or selective uptake, the effective local concentration of the drug may not be related to plasma concentration.

Active ingredients disclosed herein can be evaluated for efficacy and toxicity using known methods. For example, the toxicology of a particular active ingredient, or of a subset of the active ingredients, sharing certain chemical moieties, may be established by determining *in vitro* toxicity towards a cell line, such as a mammalian, and preferably human, cell line. The results of such studies are often predictive of toxicity in animals, such as mammals, or more specifically, humans. Alternatively, the toxicity of particular compounds in an animal model, such as mice, rats, rabbits, or monkeys, may be determined using known methods. The efficacy of a particular active ingredient may be established using several recognized methods, such as *in vitro* methods, animal models, or human clinical trials. When selecting a model to determine efficacy, the skilled artisan can be guided by the state of the art to choose an appropriate model, dose, route of administration and/or regime.

The toxicology of a pharmaceutical composition including a compound of any one of Formula (I), (II), (III), or (IV), an analogue, derivative, or pharmaceutically acceptable salt thereof, including any of the compounds specifically disclosed herein, may be established by determining *in vitro* toxicity towards a cell line, such as a mammalian, and preferably human, cell line. The results of such studies are often predictive of toxicity in animals, such as mammals, or more specifically, humans. The toxicity of a pharmaceutical composition including a compound of any one of Formula (I), (II), (III), or (IV) may be established by determining *in vivo* toxicity in an animal model, such as mice, rats, rabbits, or monkeys.

In some embodiments, the compound of Formula (I) is a compound of any one of Formula (V), (VI), (VII), or (VIII). The toxicology of a pharmaceutical composition including a compound of any one of Formula (V), (VI), (VII), or (VIII), an analogue, derivative, or pharmaceutically acceptable salt thereof, including any of the compounds specifically disclosed herein, may be established by determining *in vitro* toxicity towards a cell line, such as a mammalian, and preferably human, cell line. The results of such studies are often predictive of toxicity in animals, such as mammals, or more specifically, humans. The toxicity of a pharmaceutical composition including a compound of any one of Formula (V), (VI), (VII), or (VIII) may be established by determining *in vivo* toxicity in an animal model, such as mice, rats, rabbits, or monkeys.

### EXAMPLES

Embodiments of the present invention are further defined in the following Examples. It should be understood that these Examples are given by way of illustration only.

### Example 1

### Purification of ALT-59 Binding Proteins

Experiments were performed in order to identify potential ALT-59 targeted protein. The following methods were used to perform these experiments.

### Tissue Culture and Cell Treatment

MDA-MB-231 and PLC/PRF/5 cells were maintained at 37°C in a CO₂ incubator in Dulbecco's Modified Eagle's Medium (DMEM) supplemented with 10% fetal bovine serum (FBS) and 1% of penicillin/streptomycin (Invitrogen).

### Solid phase compound-protein complex pull-down

The cells were lysed in lysis buffer containing 20 mM 4-(2-hydroxyethyl)-1-piperazineethanesulfonic acid (HEPES), pH 7.5, 0.3% 3-[(3-cholamidopropyl)dimethylammonio]-1-propanesulfonate (CHAPS), 150 mM NaCl, (Sigma-Aldrich), and protease inhibitor cocktail (Roche) with rotation at 4°C for one hour followed by centrifugation at 15000 for 20 minutes. Ten milligrams of ALT-59 dissolved in DMSO at 20 mM were added to whole cell lysate containing 5 mg protein and incubated on ice for one hour. Compound precipitates out due to high hydrophobicity. Compound-protein complex were spun down at 1000 rpm and washed 5 times with lysis buffer to remove unbound protein. The compound-protein complex were boiled in SDS-PAGE protein sample buffer (1×) (80 mM Tris-HCl, pH 6.8, 2% SDS, 10% Glycerol, 0.1% Bromophenol blue.

For silver staining, proteins were separated on a 10% SDS-PAGE gel. To prepare samples for protein identification using mass spectrometry, protein samples were run into stacking gel and the electrophoresis was stopped after samples were compressed into single bands. Gel was stained with Coomassie brilliant blue for 30 minutes and de-stained overnight with at least three changes of deionized water. Bands were excised and subjected to mass spectrometry for protein identification. The data from these experiments show that a major protein band with an apparent molecular weight of approximately 43 kDa was revealed by silver stain of samples from both cell lines (FIG. 1). Mass spectrometry analysis identified TDP-43 as the number one hit followed by the abundant chaperonins and keratins (FIG. 2).

### Example 2

### Recombinant TDP-43 Expression and Purification

Expression vector pRETb/TDP43-His was constructed and used in the following experiment for the purpose of purifying recombinant TDP-43.

Rosetta (DE3) cells were transformed with pRETb/TDP43-His according manufacture's instruction. After the culture from a single colony reached O.D 0.6 with shaking at 37°C for 5 hours, it was split into six equal cultures and treated as indicated in FIG. 3. (Lane 1) un-induced blank; (Lane 2) 0.5 mM IPTG, 60 µM DMSO; (Lane 3) 0.5 mM IPTG, 60 µM ALT-59; (Lane 4) 0.5 mM IPTG, 2 µg/ml PMBN; (Lane 5) 0.5 mM IPTG, 2 µg/ml PMBN, 30 µM ALT-59; (Lane 6) 0.5 mM IPTG, 2 µg/ml PMBN, 60 µM ALT-59. Cells were cultured overnight at 25°C on a shaker and then collected, sonicated in protein sample buffer and boiled before loading on a 10% SDS-PAGE gel. Protein were visualized by Coomassie Brilliant Blue stain.

The results show the low expression of TDP43-His in *E. coli* due to the intrinsic propensity of TDP-43 to aggregate. Treatment with 60 µM ALT-59 and PMBN, a cationic cyclic peptide capable of specifically increasing the permeability of the outer membrane of Gram-negative bacteria toward hydrophobic compounds, increased protein expression, but the expressed protein from all cultures cannot be directly purified by Nickel-NTA agarose resin (Qiagen). Increased expression of TDP43-His in the presence of ALT-59 suggests direct binding between the two (FIG. 3).

Additional experiments were performed in order to increase recombinant TDP-43 expression and solubility. TDP-43 were inserted in frame behind a Sumo-tag, which is preceded by a His-tag on pSumo-Kan vector. The resulting pSumo/TDP43 was introduced into Rosetta (DE3) cells. Two colonies were picked and cultured by shaking at 37°C until O.D. reached 0.6. Each culture was split into two, one of which receive IPTG at a final concentration of 0.5 mM. Cells were cultured overnight at 25°C on a shaker and then collected, sonicated in protein sample buffer and boiled before loading on a 10% SDS-PAGE gel. Protein purification was performed using Nickel-NTA agarose resin (Qiagen) according to manufacturer's instructions followed by fractionation of eluted protein by a 10% SDS-PAGE gel. Protein were visualized by Coomassie Brilliant Blue stain.

The data from these experiments show that inserting a Sumo-tag in front of TDP-43 drastically increases TDP-43 expression (FIG. 4A) and renders it soluble (FIG.4B).

### Example 3

### ALT-59 Inhibits Binding of TDP-43 to TG₁₂

Additional experiments were performed to confirm the direct binding of ALT-59 to TDP-43. One microgram of purified His-Sumo-TDP43 was incubated with 1 µg TG₁₂-biotin (TGTGTGTGTGTGTGTGTGTGTGTG-biotin (SEQ ID NO: 1)) in binding buffer containing 20 mM HEPES, pH 7.5, 0.3% CHAPS, 150 mM NaCl, (Sigma-Aldrich), and protease inhibitor cocktail (Roche) for 4 hours on ice in the presence of carrier (50% Cremophor EL, 50% absolute ethanol) or 30 µM ALT-59 dissolved in carrier. Streptavidin magnetic beads (20 µl settled beads, GE Healthcare Life Sciences) were added to pull down the DNA-protein complex by rotating at 4°C for 2 hours. After being washed 5 times with binding buffer, samples were boiled in protein sample buffer. In another experiment, DNA-protein complex bound to Streptavidin magnetic beads were washed and split into three parts, two of which were incubated with either carrier or ALT-59 for 4 hours with rotation at 4°C to elute TDP-43 from TG12-biotin-streptavidin. The third part and elutes were mixed with protein sample buffer and boiled. Protein samples from the above experiments were loaded onto 4-20% Tris-glycine gel (Invitrogen) for fractionation. Western blotting was performed using anti-TDP-43 antibody (Proteintech Group).

The data from these experiments show that ALT-59 can block the binding of TDP-43 to TG₁₂ (FIG. 5). It is also capable of displacing TG₁₂ from the TDP43-TG₁₂ complex (FIG. 6), suggesting that ALT-59 binds to TDP-43 with higher affinity than TG₁₂.

### Example 4

### Inhibition of TDP-43 by ALT-59 Alters LAMP2 pre-mRNA Splicing

LAMP2 pre-mRNA consists of 11 exons (FIG. 7A). Alternative splicing of the pre-mRNA results in three mature mRNA isoforms: LAMP2a, LAMP2b, and LAMP2c. The three isoforms share the first 8 exons followed by different exons containing the stop codon. Translation of the transcripts produces three protein isoforms with different c-terminal sequences, which contains 45 or 46 amino acids. It has been shown that LAMP2C, but not 2A and 2B, is the lysosomal membrane protein working as a RNA/DNA receptor for their lysosomal degradation, therefore serves as a garbage-disposal protein for removing unwanted nucleic acid (Fujiwara et al., 2015, Biochem Biophys Res Commun 460, 281-286).

Long stretches of TG repeats are found in exon 9 and intron 9 of Lamp2 gene. A shorter stretch is located in intron 10. The presence of these high affinity TDP-43 binding sequences suggests a potential regulatory role of TDP-43 in LAMP2 pre-mRNA splicing. RT-PCR analysis was performed to test if ALT-59 treatment affects LAMP2 alternative splicing. MDA-MB-231 cells were cultured as described above and treated with DMSO or 6 µM ALT-59. Total RNA was extracted using the RNeasy Mini Kit (Qiagen) and equal amount of RNA was reverse transcribed using the SuperScript III First-Strand Synthesis System (Invitrogen). PCR reactions were performed using forward primer located on exon 1 and a reverse primer located on exon 11. PCR samples were resolved in a 1.2% agarose gel and stained with ethidium bromide.

Lamp2-forward primer: ATGGTGTGCTTCCGCCTCTTC (SEQ ID NO: 2).

Lamp2-reverse primer: TTACACAGACTGATAACCAGTACG (SEQ ID NO: 3).

The resulting data show that ALT-59 treatment reduces the level of LAMP2a mRNA and increases the level of LAMP2c mRNA (FIG. 7C). Sufficient LAMP2C protein on the lysosomal membrane enables lysosome to clean up unwanted junk nucleic acids by importing them into the lumen for degradation. On the other hand, aberrant high expression of wild type TDP-43 or gain of function mutants can lead to lower expression of LAMP2C, and consequently accumulation of junk nucleic acids together with nucleic acid binding proteins in the cytoplasm. This mechanism provides an explanation for the TDP-43 aggregates found in patients with neurodegenerative disorders.

### Example 5

### Inhibition of TDP-43 by ALT-59 Induces CFTR Exon 9 Inclusion

It has been reported that TDP-43 causes CFTR exon 9 skipping, ApoA2 exon 3 skipping, and POLDIP3 exon 3 inclusion. Additional experiments were performed to further confirm that TDP-43 is the target of ALT-59. RT-PCR analysis was performed as described above using the following primers for the three genes. PCR samples were analyzed by polyacrylamide gel electrophoresis and stained with ethidium bromide.

CFTR-forward primer: CAGAAGTAGTGATGGAGAATGTAAC (SEQ ID NO: 4).

CFTR-reverse primer: GTTGACCTCCACTCAGTGTGATTC (SEQ ID NO: 5).

ApoA2-forward primer: ATGAAGCTGCTCGCAGCAAC (SEQ ID NO: 6).

ApoA2-reverse primer: TCACTGGGTGGCAGGCTGTG (SEQ ID NO: 7).

POLDIP3-forward primer: TGCTCTGAAGCTCACCAAAA (SEQ ID NO: 8).

POLDIP3-reverse primer: GGAACGGAAGCTATACCATCAT (SEQ ID NO: 9).

The data from these experiments confirmed that by inhibiting TDP-43, ALT-59 indeed is capable of promoting CFTR exon 9 inclusion and blocking ApoA2 splicing (FIG. 8). In the case of ApoA2, inhibited TDP-43 nucleic acid binding by ALT-59 leads to less mature mRNA and more ApoA2 pre-mRNA, which due to retaining not only exon 3 (Mercado et al., 2015, Nucleic Acids Res 33, 6000-6010) but also all of the other exons and introns. The different observation could be explained by the different experiment setups. Endogenous ApoA2 gene expression was determined in this study, whereas Mercado et al (2015) determined the splicing of an artificial minigenes, which only include exon 3 (133 nt) with its flanking intronic regions (174 nt of intron 2 and 116 nt of intron 3). Splicing of POLDIP3 exon 3 remain unchanged, which is inconsistent with previous report.

### Example 6

### Inhibition of TDP-43 by ALT-59 Increases CFTR Protein Expression

Additional experiments were performed to determine how ALT-59 treatment affects CFTR protein levels. U-87 MG cells were maintained at 37°C in a CO2 incubator in Dulbecco's Modified Eagle's Medium (DMEM) supplemented with 10% fetal bovine serum (FBS) and 1% of penicillin/streptomycin (Invitrogen), and were treated with DMSO, 3 µM, or 6 µM ALT-59. Cells were lysed using RIPA buffer (25mM Tris, pH 7.4, 150 mM NaCl, 0.1% SDS, 0.5% sodium deoxycholate, 1% Triton X-100, Complete protease inhibitor cocktail). Western blotting was performed using anti-CFTR (Novus Biologicals) and anti-beta-actin (Sigma-Aldrich) antibodies. Images were obtained using Odyssey imager and quantified using the Odyssey software (LI-COR).

The resulting data confirmed the increased full-length CFTR expression by ALT-59 (FIG. 9A). Multiple CFTR isoforms with molecular weight of approximately 160, 240, and 300 kDa were detected. Treatment with 3 µM ALT-59 did not affect the level of CFTR160, but increased CFTR240 level by one fold and CFTR300 by 10 fold. Treatment with 6 µM ALT-59 increased the levels of CFTR160, CFTR240, and CFTR300 by 30%, 3 fold, and 18 fold, respectively (FIGS. 9B-9D). CFTR300 likely represents glycosylated CFTR.

### Example 7

### Inhibition of TDP-43 by ALT-59 Increases CFTR Localization on the Plasma Membrane

Immunofluorescence microscopy was performed in order to determine how ALT-59 treatment affects CFTR localization.

MDA-MB-231 and U-87 MG cells were cultured on cover slips under the conditions as described above. Cells were treated with DMSO or 4 µM ALT-59 for 3 days. Primary human bronchial epithelial cells isolated from cystic fibrosis patient homozygous for Phe508del-CFTR (Lifeline Cell Technology) were cultured on cover slips in BronchiaLife Cell Culture Medium supplemented with BrochiaLife LifeFactors Kit (Lifeline Cell Technology) and maintained at 37°C in a CO2 incubator. Cells were treated with DMSO, 4 µM ALT-59, 2 µM ALT-308, or 4 µM ALT-410 for 3 days.

Cells were fixed in 4% paraformaldehyde for 15 minutes and permeabilized in 0.1% Triton X-100 for 2 minutes. After being blocked in 5% BSA (Sigma-Aldrich), cells were incubated with anti-CFTR antibody (Alomone Labs) followed by Alexa Fluor 488 anti-rabbit secondary antibody. Reagents were buffered in GIBCO PBS (Phosphate-Buffered Saline). Images were taken using Carl Zeiss LSM 510 Laser Scanning Microscope. Parameters were set identical for all images.

The data from these experiments show that ALT-59 causes the redistribution of CFTR from the perinuclear region to the plasma membrane in MDA-MB-231 and U-87 MG cells (FIG. 10). ALT-59 treatment caused redistribution of CFTR from perinuclear region to the plasma membrane. Treatment of primary bronchial epithelial cells isolated from cystic fibrosis patient homozygous for Phe508del-CFTR by ALT-59 and its derivatives, ALT-308 and ALT-410, also leads to increased localization of Phe508del-CFTR on the plasma membrane (FIGS. 11-12).

### Example 8

### Inhibition of TDP-43 by ALT-59 Changes Expression of Proteins Associated with Neurodegenerative Diseases

U-87 MG and T98G cells were maintained at 37°C in a CO2 incubator in Dulbecco's Modified Eagle's Medium (DMEM) supplemented with 10% fetal bovine serum (FBS) and 1% of penicillin/streptomycin (Invitrogen). Cells were treated with DMSO, 3 µM, or 6 µM ALT-59 for 2 days and subjected to Western blotting analysis using the indicated antibodies. Protein bands were quantified using the ImageJ software.

The resulting data show that in U-87 MG cells, inhibition of TDP-43 by ALT-59 at 3 µM and 6 µM reduces Beta-secretase 1 (BACE1) protein levels by 20% and 50%, respectively. ALT-59 at 3 µM and 6 µM reduced Tau expression by 52% and 38%, respectively. In contrast, the treatments increased MAP2 expression by 1.42 and 2.51 folds, respectively (FIG. 13). The regulation of BACE1 expression by ALT-59 treatment was confirmed in T98G cells, in which 3 µM and 6 µM ALT-59 reduced BACE1 expression by 31% and 53%, respectively (FIG. 14).

BACE1 is responsible for the proteolytic processing of the amyloid precursor protein (APP) into the 40 or 42 amino acid-long amyloid-β peptides, which are the components of amyloid plaques found in patients with neurodegenerative diseases. The microtubule-associated protein tau (MAPT) forms insoluble filaments that accumulate as neurofibrillary tangles in many diseases, including neurodegenerative diseases. It has been shown that MAP2 is crucial traffic regulator that controls the axonal growth potential of sensory neurons by directing axonal cargo transport (Gumy et al., 2017, Neuron 94, 347-362 e347). Together, these data show that inhibition of TDP-43 by ALT-59 treatment can decrease the expression of pathological proteins associated with neurodegenerative diseases and at the same time increase beneficial protein expression.

### Example 9

### Screening for ALT-59 Derivatives

Additional experiments were performed to screen for compounds with higher potency and the ability to penetrate blood brain barrier using BACE1 protein levels as an indicator. U-87 MG cells were cultured as described previously and treated for 2 days with compounds at concentrations as indicated in FIGS 15-17. Cells were then subjected to Western blotting analysis using anti-BACE1 and anti-beta-actin antibodies.

The data from these experiments lead to the identification of compounds capable of lowering BACE1 protein expression. Chemical structures of compounds capable of lowering BACE1 expression are described herein and are shown in FIGs. 18-20. These compounds are referred to herein as ALT-108, ALT-212, ALT-215, ALT-308, ALT-317, ALT-309, ALT-408, ALT-411, ALT-333, ALT-59, ALT-110, ALT-201, ALT-202, ALT-204, ALT-205, ALT-207, ALT-208, ALT-210, ALT-211, ALT-302, ALT-306, ALT-307, ALT-311, ALT-318, ALT-322, ALT-324, ALT-402, ALT-403, ALT-404, ALT-406, ALT-409, ALT-410, ALT-413, and ALT-414.

### Example 10

### ALT-59 Treatment Rescues the Paralysis Caused by hTDP-43 expression in C. elegans

Wild-type N2 strain or transgenic C. *elegans* expressing full-length (wild-type) human TDP-43 (strain number, CL6049, Caenorhabditis Genetics Center, Minneapolis, MN) were placed on freshly prepared Nematode Growth Medium (NGM) petri plates with or without 10 µM ALT-59.

Uncoordinated phenotypes were imaged, and movement deficits were quantified by counting body thrashes in liquid after recording under an inverted microscope.

Neuronal expression of full-length human TDP-43 in C. *elegans* leads to motorneuron dysfunction and causes the animal to have a coiled body posture, uncoordinated movement, and reduced body thrashes in liquid (Ash et al., 2010). Thus, the C. *elegans* model used for this example is an appropriate model for in vivo testing of the effects of the compositions provided herein on human TDP-43, which has known correlations to neurodegenerative disorders. Treatment with ALT-59 rescued the coiled body posture and uncoordinated phenotype (FIG. 21), and reduced the percentage of paralysis from 93% to 29.5% (FIG. 22). These results demonstrate the in vivo therapeutic benefits upon blocking TDP-43's nucleic acid binding activity using the compositions provided herein.

### Example 11

### Treatment of Neurodegenerative Disorders in Mice

A pharmaceutical composition comprising a compound of any one of Formula (I), (II), (III), (IV), (V), (VI), (VII), or (VIII), an analogue, derivative, or pharmaceutically acceptable salt thereof, including any of the compounds specifically disclosed herein, is prepared. The pharmaceutical composition includes a compound of any one of Formula (I), (II), (III), (IV), (V), (VI), (VII), or (VIII) present in a therapeutically effective amount.

TDP-43 toxicity originates from its nucleic acid binding activity (Ihara et al., 2013, Hum Mol Genet, 22(22), 4474-4484; Voigt et al., 2010, PLoS One, 5(8), e12247; Wils et al., 2010, PNAS 107, 3858-3863). Expression of the human TDP-43 gene in neurons throughout the mouse central nervous system causes degeneration of cortical and spinal motor neurons and spastic paralysis (Wils et al., 2010, PNAS 107, 3858-3863). A mouse model of TDP-43 neurodegeneration is obtained, mouse strain B6;SJL-Tg(Thy1-TARDBP)4Singh/J from the Jackson Laboratory (Stock No: 012836). The mouse model is an accepted animal model for neurodegeneration and TDP-43 toxicity, because it has been established for such, and is screened using the compounds provided herein. The mouse strain expresses the human TDP-43 gene and is used to test the in vivo efficacy of the compositions provided herein. The compounds are administered to homozygous mice at 10 mg/kg body weight by Intraperitoneal injection 3 times per week for 4 weeks. Motor phenotypes of the treated groups are compared to vehicle-treated control groups.

The mice treated with the compounds exhibit ameliorated paralysis caused by TDP-43 expression in neurons, and improved survival.

### Example 12

### Treatment of Neurodegenerative Disorders using a Pharmaceutical Composition

A pharmaceutical composition comprising a compound of any one of Formula (I), (II), (III), (IV), (V), (VI), (VII), or (VIII), an analogue, derivative, or pharmaceutically acceptable salt thereof, including any of the compounds specifically disclosed herein, is prepared. The pharmaceutical composition includes a compound of any one of Formula (I), (II), (III), (IV), (V), (VI), (VII), or (VIII) present in a therapeutically effective amount, for example, in an amount ranging from 0.01 mg to 3000 mg.

A pharmaceutical composition comprising a compound of any one of Formula (V), (VI), (VII), or (VIII), an analogue, derivative, or pharmaceutically acceptable salt thereof, including any of the compounds specifically disclosed herein, is prepared. The pharmaceutical composition includes a compound of any one of Formula (V), (VI), (VII), or (VIII) present in a therapeutically effective amount, for example, in an amount ranging from 0.01 mg to 3000 mg.

Subjects suffering from neurodegenerative disorders are identified by diagnosing the subjects as having a neurodegenerative disorder. Subjects are selected based on the degree of neurodegenerative disorder. The severity of the neurodegenerative disorder is assessed prior to, during, and following treatment. Assessment includes subject responses, subject neurological testing, and biomarker assessment.

Subjects are instructed to discontinue use of any neurodegenerative treatments and to avoid any new treatments. The identified subjects are grouped into control and treatment groups. At the start of the study, all subjects are assessed for disease severity. On the same day, each subject is administered a composition, wherein the control group is administered a placebo composition and the treatment group is administered the pharmaceutical composition. The treatment is repeated as necessary during the course of treatment.

Outcomes of treatment are assessed during the course of the study and following the final treatment. The outcomes of treatment group is compared to the outcomes of control group. The efficacy of the pharmaceutical composition is evaluated based on the assessment during the course of the treatment and on the assessment after treatment, as compared to the control group. Subjects receiving the pharmaceutical composition exhibit improvement in neurological assessment as compared to the control group.

As used herein, the section headings are for organizational purposes only and are not to be construed as limiting the described subject matter in any way. It will be appreciated that there is an implied "about" prior to the temperatures, concentrations, times, etc. discussed in the present teachings, such that slight and insubstantial deviations are within the scope of the present teachings herein.

In this application, the use of the singular includes the plural unless specifically stated otherwise. Also, the use of "comprise", "comprises", "comprising", "contain", "contains", "containing", "include", "includes", and "including" are not intended to be limiting.

As used in this specification and claims, the singular forms "a," "an" and "the" include plural references unless the content clearly dictates otherwise.

It should be understood, however, that this detailed description, while indicating preferred embodiments of the invention, is given by way of illustration only.

The terminology used in the description presented herein is not intended to be interpreted in any limited or restrictive manner. Rather, the terminology is simply being utilized in conjunction with a detailed description of embodiments of the systems, methods and related components. Furthermore, embodiments may comprise several novel features, no single one of which is solely responsible for its desirable attributes or is believed to be essential to practicing the inventions herein described.

## Claims

1. A pharmaceutical composition comprising a therapeutically effective amount of a compound of Formula (I), or a pharmaceutically acceptable salt thereof, for use in the treatment of cystic fibrosis or a neurodegenerative disease, wherein the compound of Formula (I) is: 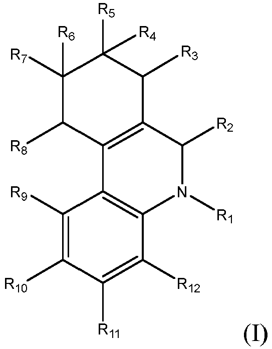 wherein:
R₁ is H, OH, or lower alkyl;
R₂ is an optionally substituted aromatic ring of four, five, or six carbons, wherein the aromatic ring is carbocyclic or heterocyclic, and wherein each position on the aromatic ring is independently H, halo, lower alkyl, OH, lower alkoxy, NH₂, lower alkylamino, di(lower alkyl)amino, SH, lower alkylthio, NO₂, or two residues together form a heterocyclic ring;
R₃ and R₈ are each independently H, lower alkyl, =O, =S, OH, NH₂, aryl, or aralkyl, where aryl and aralkyl are substituted with 0-3 moieties selected from the group consisting of halo, OH, NH₂, lower alkyl, lower alkoxy, SH, lower alkylthio, and lower alkylamino
R₄, R₅, R₆, and R₇ are each independently H, lower alkyl, OH, NH₂, aryl, or aralkyl, where aryl and aralkyl are substituted with 0-3 moieties selected from the group consisting of halo, OH, NH₂, lower alkyl, lower alkoxy, SH, lower alkylthio, and lower alkylamino;
at least one of R₉ and R₁₀, R₁₀ and R₁₁, or R₁₁ and R₁₂, together form an optionally substituted benzene ring, wherein the benzene ring is optionally substituted with H, halo, lower alkyl, OH, lower alkoxy, or NO₂;
wherein any one of the carbon atoms on any one of fused rings of Formula (I) is optionally replaced with a nitrogen atom; and
wherein the alkyl part of a lower alkyl, lower alkoxy, lower alkylthio and lower alkylamino is an alkyl radical having from 1 to about 6 carbon atoms.

2. The pharmaceutical composition for use in the treatment of cystic fibrosis or a neurodegenerative disease according to claim 1, wherein the compound of Formula (I) is ALT-212, ALT-215, ALT-308, ALT-309, ALT-408, ALT-411, ALT-59, ALT-110, ALT-201, ALT-202, ALT-204, ALT-208, ALT-207, ALT-210, ALT-211, ALT-302, ALT-306, ALT-307, ALT-311, ALT-318, ALT-322, ALT-324, ALT-402, ALT-404, ALT-406, ALT-409, ALT-410, ALT-413, ALT-414, ALT-108, ALT-317, ALT-333, ALT-403, or ALT-205.

3. The pharmaceutical composition for use in the treatment of cystic fibrosis or a neurodegenerative disease according to claim 1, wherein the compound of Formula (I) is a compound of any one of Formula (II), (III), or (IV): 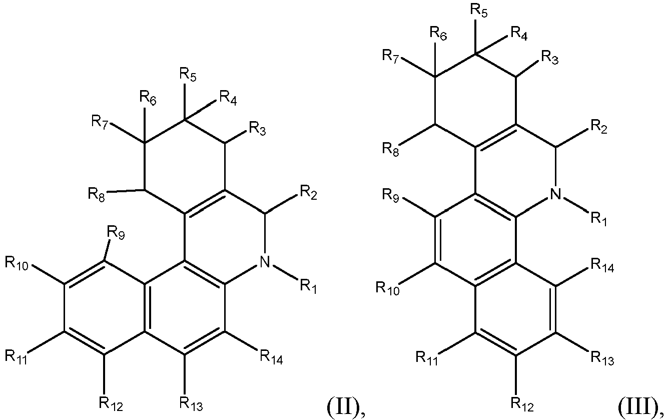 or 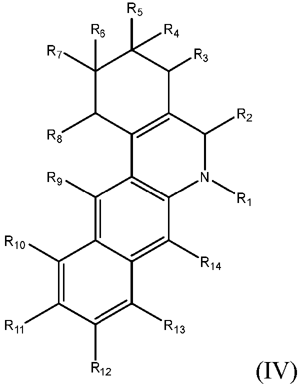 wherein:
R₁ is H, OH, or lower alkyl;
R₂, is an optionally substituted aromatic ring of four, five, or six carbons, wherein the aromatic ring is carbocyclic or heterocyclic, and wherein each position on the aromatic ring is independently H, halo, lower alkyl, OH, lower alkoxy, NH₂, lower alkylamino, di(lower alkyl)amino, SH, lower alkylthio, NO₂, or two residues together form a heterocyclic ring;
R₃ and R₈ are each independently H, lower alkyl, =O, =S, OH, NH₂, aryl, or aralkyl, where aryl and aralkyl are substituted with 0-3 moieties selected from the group consisting of halo, OH, NH₂, lower alkyl, lower alkoxy, SH, lower alkylthio, and lower alkylamino;
R₄, R₅, R₆, and R₇ are each independently H, lower alkyl, OH, NH₂, aryl, or aralkyl, where aryl and aralkyl are substituted with 0-3 moieties selected from the group consisting of halo, OH, NH₂, lower alkyl, lower alkoxy, SH, lower alkylthio, and lower alkylamino;
R₈, R₉, R₁₀, R₁₁, R₁₂, R₁₃, R₁₄ are each independently H, halo, lower alkyl, OH, lower alkoxy, or NO₂; and
wherein any one of the carbon atoms on any one of fused rings of Formula (II), (III), or (IV) is optionally replaced with a nitrogen atom.

4. The pharmaceutical composition for use in the treatment of cystic fibrosis or a neurodegenerative disease according to claim 3, wherein the compound of Formula (II) is ALT-212, ALT-215, ALT-308, ALT-309, ALT-408, ALT-411, ALT-59, ALT-110, ALT-201, ALT-202, ALT-204, ALT-208, ALT-207, ALT-210, ALT-211, ALT-302, ALT-306, ALT-307, ALT-311, ALT-318, ALT-322, ALT-324, ALT-402, ALT-404, ALT-406, ALT-409, ALT-410, ALT-413, or ALT-414; or
wherein the compound of Formula (III) is ALT-108, ALT-317, ALT-333, or ALT-403; or
wherein the compound of Formula (IV) is ALT-205.

5. The pharmaceutical composition for use in the treatment of cystic fibrosis or a neurodegenerative disease according to claim 1, wherein the compound of Formula (I) is a compound of Formula (V): 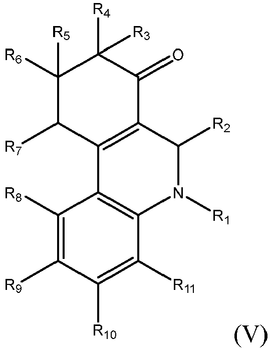 wherein:
R₁ is H, OH, or lower alkyl;
R₂ is an optionally substituted aromatic ring of four, five, or six carbons, wherein the aromatic ring is carbocyclic or heterocyclic, and wherein each position on the aromatic ring is independently H, halo, lower alkyl, OH, lower alkoxy, NH₂, lower alkylamino, di(lower alkyl)amino, SH, lower alkylthio, NO₂, or two residues together form a heterocyclic ring;
R₃, R₄, R₅, R₆, and R₇ are each independently H, lower alkyl, OH, NH₂, aryl, or aralkyl, where aryl and aralkyl are substituted with 0-3 moieties selected from the group consisting of halo, OH, NH₂, lower alkyl, lower alkoxy, SH, lower alkylthio, and lower alkylamino;
at least one of R₈ and R₉, R₉ and R₁₀, or R₁₀ and R₁₁ together form an optionally substituted benzene ring, wherein the benzene ring is optionally substituted with H, halo, lower alkyl, OH, lower alkoxy, or NO₂; and
wherein any one of the carbon atoms on any one of fused rings of Formula (V) is optionally replaced with a nitrogen atom.

6. The pharmaceutical composition for use in the treatment of cystic fibrosis or a neurodegenerative disease according to claim 5, wherein the compound of Formula (V) is a compound of any one of Formula (VI), (VII), or (VIII): 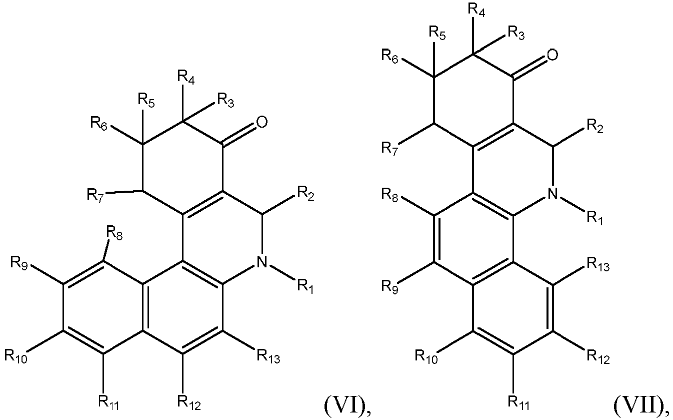 or 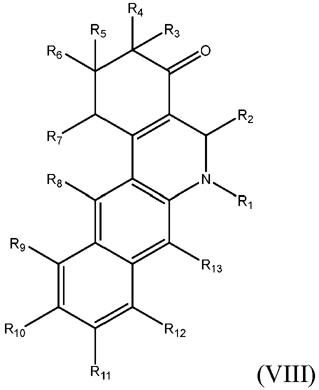 wherein:
R₁ is H, OH, or lower alkyl;
R₂ is an optionally substituted aromatic ring of four, five, or six carbons, wherein the aromatic ring is carbocyclic or heterocyclic, and wherein each position on the aromatic ring is independently H, halo, lower alkyl, OH, lower alkoxy, NH₂, lower alkylamino, di(lower alkyl)amino, SH, lower alkylthio, NO₂, or two residues together form a heterocyclic ring;
R₃, R₄, R₅, R₆, and R₇ are each independently H, lower alkyl, OH, NH₂, aryl, or aralkyl, where aryl and aralkyl are substituted with 0-3 moieties selected from the group consisting of halo, OH, NH₂, lower alkyl, lower alkoxy, SH, lower alkylthio, and lower alkylamino;
R₈, R₉, R₁₀, R₁₁, R₁₂, and R₁₃ are each independently H, halo, lower alkyl, OH, lower alkoxy, or NO₂; and
wherein any one of the carbon atoms on any one of fused rings of Formula (VI), (VII), or (VIII) is optionally replaced with a nitrogen atom.

7. The pharmaceutical composition for use in the treatment of cystic fibrosis or a neurodegenerative disease according to claim 6, wherein the compound of Formula (VI) is ALT-212, ALT-215, ALT-308, ALT-309, ALT-408, ALT-411, ALT-59, ALT-110, ALT-202, ALT-204, ALT-208, ALT-207, ALT-210, ALT-211, ALT-302, ALT-306, ALT-307, ALT-311, ALT-318, ALT-322, ALT-324, ALT-402, ALT-404, ALT-406, ALT-409, ALT-410, ALT-413, or ALT-414; or
wherein the compound of Formula (VII) is ALT-108, ALT-317, ALT-333, or ALT-403; or
wherein the compound of Formula (VIII) is ALT-205.

8. The pharmaceutical composition for use in the treatment of cystic fibrosis or a neurodegenerative disease according to any one of claims 1-7, wherein the composition inhibits the progression or development of cystic fibrosis.

9. The pharmaceutical composition for use in the treatment of cystic fibrosis or a neurodegenerative disease according to any one of claims 1-8, wherein the composition inhibits the progression or development of the neurodegenerative disease; optionally, wherein the neurodegenerative disease is amyotrophic lateral sclerosis (ALS), frontotemporal dementia (FTD), Alzheimer's disease, hippocampal sclerosis of aging (HS-Aging), chronic traumatic encephalopathy, or Parkinson's disease.

10. The pharmaceutical composition for use in the treatment of cystic fibrosis or a neurodegenerative disease according to any one of claims 1-9, wherein the compound of Formula (I) is present in an amount of 0.01 mg to 3000 mg.

11. The pharmaceutical composition for use in the treatment of cystic fibrosis or a neurodegenerative disease according to any one of claims 1-10, wherein the composition is formulated for oral or parenteral administration.

12. The pharmaceutical composition for use in the treatment of cystic fibrosis or a neurodegenerative disease according to any one of claims 1-11, further comprising a pharmaceutically acceptable carrier or excipient.

13. The pharmaceutical composition for use in the treatment of cystic fibrosis or a neurodegenerative disease according to claim 4, wherein the compound of Formula (II) is ALT-302, ALT-306, ALT-308 or ALT-309.

14. The pharmaceutical composition for use in the treatment of cystic fibrosis or a neurodegenerative disease according to claim 4, wherein the compound of Formula (II) is ALT-302.

15. The pharmaceutical composition for use in the treatment of cystic fibrosis or a neurodegenerative disease according to claim 7, wherein the compound of Formula (VI) is ALT-059, ALT-302, ALT-306, ALT-308 or ALT-309.

## Patentansprüche

1. Pharmazeutische Zusammensetzung, umfassend eine therapeutisch wirksame Menge einer Verbindung nach Formel (I) oder eines pharmazeutisch verträglichen Salzes davon zur Verwendung bei der Behandlung von Mukoviszidose oder einer neurodegenerativen Erkrankung, wobei die Verbindung nach Formel (I) wie folgt lautet: 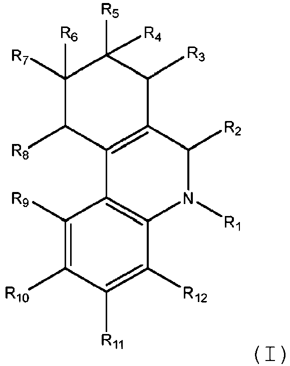 wobei:
R₁ H, OH oder niederes Alkyl ist;
R₂ ein optional substituierter aromatischer Ring mit vier, fünf oder sechs Kohlenstoffen ist, wobei der aromatische Ring carbocyclisch oder heterocyclisch ist und wobei jede Position auf dem aromatischen Ring unabhängig voneinander H, Halogen, niederes Alkyl, OH, niederes Alkoxy, NH₂, niederes Alkylamino, di(niederes Alkyl)amino, SH, niederes Alkylthio, NO₂ ist oder zwei Reste zusammen einen heterocyclischen Ring bilden;
R₃ und R₈ jeweils unabhängig voneinander H, niederes Alkyl, =O, =S, OH, NH₂, Aryl oder Aralkyl sind, wobei Aryl und Aralkyl mit 0-3 Teilen substituiert sind, die aus der Gruppe bestehend aus Halogen, OH, NH₂, niedrigem Alkyl, niedrigem Alkoxy, SH, niedrigem Alkylthio und niedrigem Alkylamino ausgewählt sind
R₄, R₅, R₆ und R₇ jeweils unabhängig voneinander H, niederes Alkyl, OH, NH₂, Aryl oder Aralkyl sind, wobei Aryl und Aralkyl mit 0-3 Teilen substituiert sind, die aus der Gruppe bestehend aus Halogen, OH, NH₂, niedrigem Alkyl, niedrigem Alkoxy, SH, niedrigem Alkylthio und niedrigem Alkylamino ausgewählt sind;
mindestens einer aus R₉ und R₁₀, R₁₀ und R₁₁ oder R₁₁ und R₁₂ zusammen einen optional substituierten Benzolring bilden, wobei der Benzolring optional mit H, Halogen, niedrigem Alkyl, OH, niedrigem Alkoxy oder NO₂ substituiert ist;
wobei ein beliebiges der Kohlenstoffatome auf einem beliebigen aus anellierten Ringen nach Formel (I) optional durch ein Stickstoffatom ersetzt ist; und
wobei der Alkylanteil eines niedrigen Alkyls, niedrigen Alkoxys, niedrigen Alkylthios und niedrigen Alkylaminos ein Alkylradikal ist, das 1 bis etwa 6 Kohlenstoffatome aufweist.

2. Pharmazeutische Zusammensetzung zur Verwendung bei der Behandlung von Mukoviszidose oder einer neurodegenerativen Erkrankung nach Anspruch 1, wobei die Verbindung nach Formel (I) ALT-212, ALT-215, ALT-308, ALT-309, ALT-408, ALT-411, ALT-59, ALT-110, ALT-201, ALT-202, ALT-204, ALT-208, ALT-207, ALT-210, ALT-211, ALT-302, ALT-306, ALT-307, ALT-311, ALT-318, ALT-322, ALT-324, ALT-402, ALT-404, ALT-406, ALT-409, ALT-410, ALT-413, ALT-414, ALT-108, ALT-317, ALT-333, ALT-403 oder ALT-205 ist.

3. Pharmazeutische Zusammensetzung zur Verwendung bei der Behandlung von Mukoviszidose oder einer neurodegenerativen Erkrankung nach Anspruch 1, wobei die Verbindung nach Formel (I) eine Verbindung nach einer beliebigen der Formeln (II), (III) oder (IV) ist: 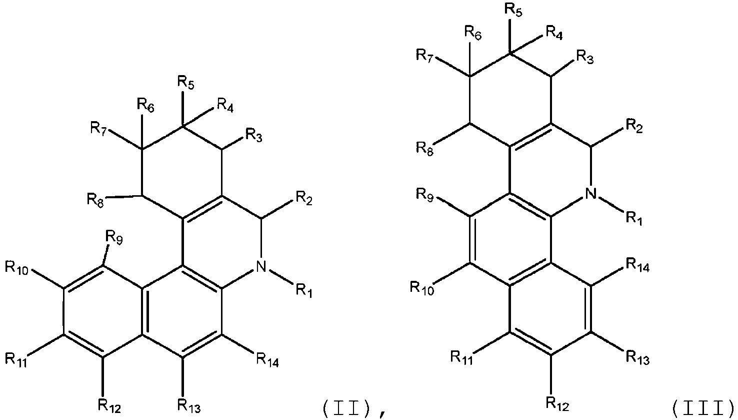 oder 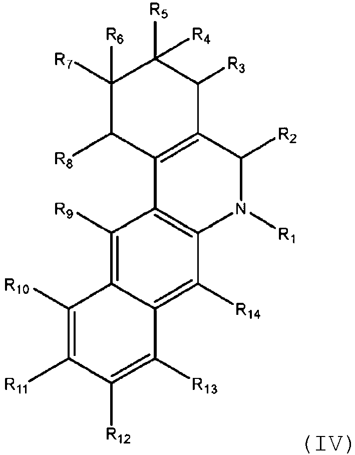 wobei:
R₁ H, OH oder niederes Alkyl ist;
R₂ ein optional substituierter aromatischer Ring mit vier, fünf oder sechs Kohlenstoffen ist, wobei der aromatische Ring carbocyclisch oder heterocyclisch ist und wobei jede Position auf dem aromatischen Ring unabhängig voneinander H, Halogen, niederes Alkyl, OH, niederes Alkoxy, NH₂, niederes Alkylamino, di(niederes Alkyl)amino, SH, niederes Alkylthio, NO₂ ist oder zwei Reste zusammen einen heterocyclischen Ring bilden;
R₃ und R₈ jeweils unabhängig voneinander H, niederes Alkyl, =O, =S, OH, NH₂, Aryl oder Aralkyl sind, wobei Aryl und Aralkyl mit 0-3 Teilen substituiert sind, die aus der Gruppe bestehend aus Halogen, OH, NH₂, niedrigem Alkyl, niedrigem Alkoxy, SH, niedrigem Alkylthio und niedrigem Alkylamino ausgewählt sind;
R₄, R₅, R₆ und R₇ jeweils unabhängig voneinander H, niederes Alkyl, OH, NH₂, Aryl oder Aralkyl sind, wobei Aryl und Aralkyl mit 0-3 Teilen substituiert sind, die aus der Gruppe bestehend aus Halogen, OH, NH₂, niedrigem Alkyl, niedrigem Alkoxy, SH, niedrigem Alkylthio und niedrigem Alkylamino ausgewählt sind;
R₈, R₉, R₁₀, R₁₁, R₁₂, R₁₃, R₁₄ jeweils unabhängig voneinander H, Halogen, niedriges Alkyl, OH, niedriges Alkoxy oder NO₂ sind; und
wobei ein beliebiges der Kohlenstoffatome auf einem beliebigen aus anellierten Ringen nach Formel (II), (III) oder (IV) optional durch ein Stickstoffatom ersetzt ist.

4. Pharmazeutische Zusammensetzung zur Verwendung bei der Behandlung von Mukoviszidose oder einer neurodegenerativen Erkrankung nach Anspruch 3, wobei die Verbindung nach Formel (II) ALT-212, ALT-215, ALT-308, ALT-309, ALT-408, ALT-411, ALT-59, ALT-110, ALT-201, ALT-202, ALT-204, ALT-208, ALT-207, ALT-210, ALT-211, ALT-302, ALT-306, ALT-307, ALT-311, ALT-318, ALT-322, ALT-324, ALT-402, ALT-404, ALT-406, ALT-409, ALT-410, ALT-413 oder ALT-414 ist; oder
wobei die Verbindung nach Formel (III) ALT-108, ALT-317, ALT-333 oder ALT-403 ist; oder
wobei die Verbindung nach Formel (IV) ALT-205 ist.

5. Pharmazeutische Zusammensetzung zur Verwendung bei der Behandlung von Mukoviszidose oder einer neurodegenerativen Erkrankung nach Anspruch 1, wobei die Verbindung nach Formel (I) eine Verbindung nach Formel (V) ist: 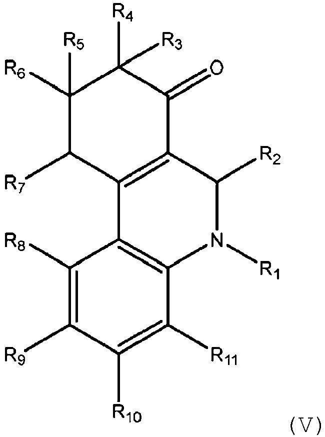 wobei:
R₁ H, OH oder niederes Alkyl ist;
R₂ ein optional substituierter aromatischer Ring mit vier, fünf oder sechs Kohlenstoffen ist, wobei der aromatische Ring carbocyclisch oder heterocyclisch ist und wobei jede Position auf dem aromatischen Ring unabhängig voneinander H, Halogen, niederes Alkyl, OH, niederes Alkoxy, NH₂, niederes Alkylamino, di(niederes Alkyl)amino, SH, niederes Alkylthio, NO₂ ist oder zwei Reste zusammen einen heterocyclischen Ring bilden;
R₃, R₄, R₅, R₆ und R₇ jeweils unabhängig voneinander H, niederes Alkyl, OH, NH₂, Aryl oder Aralkyl sind, wobei Aryl und Aralkyl mit 0-3 Teilen substituiert sind, die aus der Gruppe bestehend aus Halogen, OH, NH₂, niedrigem Alkyl, niedrigem Alkoxy, SH, niedrigem Alkylthio und niedrigem Alkylamino ausgewählt sind;
mindestens einer aus R₈ und R₉, R₉ und R₁₀ oder R₁₀ und R₁₁ zusammen einen optional substituierten Benzolring bilden, wobei der Benzolring optional mit H, Halogen, niedrigem Alkyl, OH, niedrigem Alkoxy oder NO₂ substituiert ist; und
wobei ein beliebiges der Kohlenstoffatome auf einem beliebigen aus anellierten Ringen nach Formel (V) optional durch ein Stickstoffatom ersetzt ist.

6. Pharmazeutische Zusammensetzung zur Verwendung bei der Behandlung von Mukoviszidose oder einer neurodegenerativen Erkrankung nach Anspruch 5, wobei die Verbindung nach Formel (V) eine Verbindung nach einer beliebigen der Formeln (VI), (VII) oder (VIII) ist: 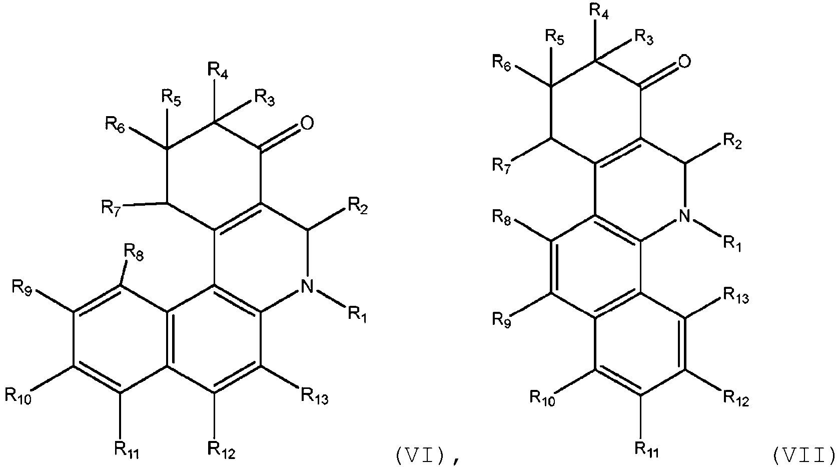 oder 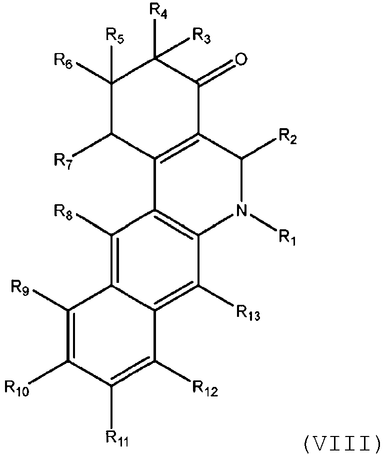 wobei:
R₁ H, OH oder niederes Alkyl ist;
R₂ ein optional substituierter aromatischer Ring mit vier, fünf oder sechs Kohlenstoffen ist, wobei der aromatische Ring carbocyclisch oder heterocyclisch ist und wobei jede Position auf dem aromatischen Ring unabhängig voneinander H, Halogen, niederes Alkyl, OH, niederes Alkoxy, NH₂, niederes Alkylamino, di(niederes Alkyl)amino, SH, niederes Alkylthio, NO₂ ist oder zwei Reste zusammen einen heterocyclischen Ring bilden;
R₃, R₄, R₅, R₆ und R₇ jeweils unabhängig voneinander H, niederes Alkyl, OH, NH₂, Aryl oder Aralkyl sind, wobei Aryl und Aralkyl mit 0-3 Teilen substituiert sind, die aus der Gruppe bestehend aus Halogen, OH, NH₂, niedrigem Alkyl, niedrigem Alkoxy, SH, niedrigem Alkylthio und niedrigem Alkylamino ausgewählt sind;
R₈, R₉, R₁₀, R₁₁, R₁₂, und R₁₃ jeweils unabhängig voneinander H, Halogen, niedriges Alkyl, OH, niedriges Alkoxy oder NO₂ sind; und
wobei ein beliebiges der Kohlenstoffatome auf einem beliebigen aus anellierten Ringen nach Formel (VI), (VII) oder (VIII) optional durch ein Stickstoffatom ersetzt ist.

7. Pharmazeutische Zusammensetzung zur Verwendung bei der Behandlung von Mukoviszidose oder einer neurodegenerativen Erkrankung nach Anspruch 6, wobei die Verbindung nach Formel (VI) ALT-212, ALT-215, ALT-308, ALT-309, ALT-408, ALT-411, ALT-59, ALT-110, ALT-202, ALT-204, ALT-208, ALT-207, ALT-210, ALT-211, ALT-302, ALT-306, ALT-307, ALT-311, ALT-318, ALT-322, ALT-324, ALT-402, ALT-404, ALT-406, ALT-409, ALT-410, ALT-413 oder ALT-414 ist; oder
wobei die Verbindung nach Formel (VII) ALT-108, ALT-317, ALT-333 oder ALT-403 ist; oder
wobei die Verbindung nach Formel (VIII) ALT-205 ist.

8. Pharmazeutische Zusammensetzung zur Verwendung bei der Behandlung von Mukoviszidose oder einer neurodegenerativen Erkrankung nach einem der Ansprüche 1-7, wobei die Zusammensetzung das Voranschreiten oder die Entwicklung von Mukoviszidose verhindert.

9. Pharmazeutische Zusammensetzung zur Verwendung bei der Behandlung von Mukoviszidose oder einer neurodegenerativen Erkrankung nach einem der Ansprüche 1-8, wobei die Zusammensetzung das Voranschreiten oder die Entwicklung der neurodegenerativen Erkrankung verhindert; optional, wobei es sich bei der neurodegenerativen Erkrankung um amyotrophe Lateralsklerose (ALS), frontotemporale Demenz (FTD), Alzheimer, Hippocampus-Sklerose im Alter (HS-Aging), chronische traumatische Enzephalopathie oder Parkinson handelt.

10. Pharmazeutische Zusammensetzung zur Verwendung bei der Behandlung von Mukoviszidose oder einer neurodegenerativen Erkrankung nach einem der Ansprüche 1-9, wobei die Verbindung nach Formel (I) in einer Menge von 0,01 mg bis 3000 mg vorhanden ist.

11. Pharmazeutische Zusammensetzung zur Verwendung bei der Behandlung von Mukoviszidose oder einer neurodegenerativen Erkrankung nach einem der Ansprüche 1-10, wobei die Zusammensetzung zur oralen oder parenteralen Verabreichung formuliert ist.

12. Pharmazeutische Zusammensetzung zur Verwendung bei der Behandlung von Mukoviszidose oder einer neurodegenerativen Erkrankung nach einem der Ansprüche 1-11, ferner umfassend einen pharmazeutisch verträglichen Träger oder Hilfsstoff.

13. Pharmazeutische Zusammensetzung zur Verwendung bei der Behandlung von Mukoviszidose oder einer neurodegenerativen Erkrankung nach Anspruch 4, wobei die Verbindung nach Formel (II) ALT-302, ALT-306, ALT-308 oder ALT-309 ist.

14. Pharmazeutische Zusammensetzung zur Verwendung bei der Behandlung von Mukoviszidose oder einer neurodegenerativen Erkrankung nach Anspruch 4, wobei die Verbindung nach Formel (II) ALT-302 ist.

15. Pharmazeutische Zusammensetzung zur Verwendung bei der Behandlung von Mukoviszidose oder einer neurodegenerativen Erkrankung nach Anspruch 7, wobei die Verbindung nach Formel (VI) ALT-059, ALT-302, ALT-306, ALT-308 oder ALT-309 ist.

## Revendications

1. Composition pharmaceutique comprenant une quantité thérapeutiquement efficace d'un composé de formule (I), ou d'un sel pharmaceutiquement acceptable de celui-ci, pour une utilisation dans le traitement de la mucoviscidose ou d'une maladie neurodégénérative, dans laquelle le composé de formule (I) est : 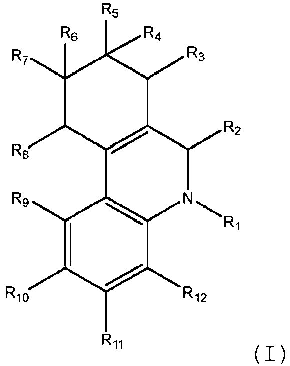 dans laquelle :
R₁ est H, OH ou un alkyle inférieur ;
R₂ est un cycle aromatique éventuellement substitué de quatre, cinq ou six carbones, dans lequel le cycle aromatique est carbocyclique ou hétérocyclique, et dans lequel chaque position sur le cycle aromatique est indépendamment occupée par H, un halo, un alkyle inférieur, OH, un alcoxy inférieur, NH₂, un alkylamino inférieur, un di(alkyle inférieur)amino, SH, un alkylthio inférieur, NO₂, ou deux résidus forment ensemble un cycle hétérocyclique ;
R₃ et R₈ sont chacun indépendamment H, un alkyle inférieur, =O, =S, OH, NH₂, un aryle ou un aralkyle, où l'aryle et l'aralkyle sont substitués par 0 à 3 groupements choisis dans le groupe constitué par un halo, OH, NH₂, un alkyle inférieur, un alcoxy inférieur, SH, un alkylthio inférieur et un alkylamino inférieur ;
R₄, R₅, R₆ et R₇ sont chacun indépendamment H, un alkyle inférieur, OH, NH₂, un aryle ou un aralkyle, où l'aryle et l'aralkyle sont substitués par 0 à 3 groupements choisis dans le groupe constitué par un halo, OH, NH₂, un alkyle inférieur, un alcoxy inférieur, SH, un alkylthio inférieur et un alkylamino inférieur ;
au moins un couple parmi R₉ et R₁₀, R₁₀ et R₁₁, ou R₁₁ et R₁₂, forme ensemble un cycle benzénique éventuellement substitué, dans lequel le cycle benzénique est éventuellement substitué par H, un halo, un alkyle inférieur, OH, un alcoxy inférieur ou NO₂ ;
dans laquelle tout atome de carbone sur l'un quelconque des cycles condensés de la formule (I) est éventuellement remplacé par un atome d'azote ; et
dans laquelle la partie alkyle d'un alkyle inférieur, d'un alcoxy inférieur, d'un alkylthio inférieur et d'un alkylamino inférieur est un radical alkyle ayant de 1 à environ 6 atomes de carbone.

2. Composition pharmaceutique pour une utilisation dans le traitement de la mucoviscidose ou d'une maladie neurodégénérative selon la revendication 1, dans laquelle le composé de formule (I) est ALT-212, ALT-215, ALT-308, ALT-309, ALT-408, ALT-411, ALT-59, ALT-110, ALT-201, ALT-202, ALT-204, ALT-208, ALT-207, ALT-210, ALT-211, ALT-302, ALT-306, ALT-307, ALT-311, ALT-318, ALT-322, ALT-324, ALT-402, ALT-404, ALT-406, ALT-409, ALT-410, ALT-413, ALT-414, ALT-108, ALT-317, ALT-333, ALT-403 ou ALT-205.

3. Composition pharmaceutique pour une utilisation dans le traitement de la mucoviscidose ou d'une maladie neurodégénérative selon la revendication 1, dans laquelle le composé de formule (I) est un composé de l'une quelconque des formules (II), (III) ou (IV) : 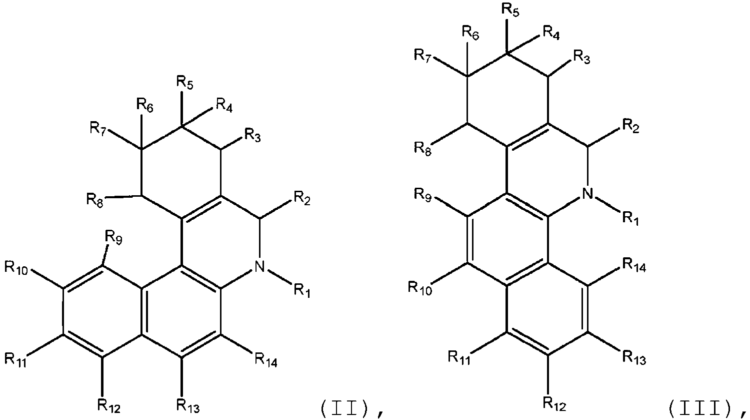 ou 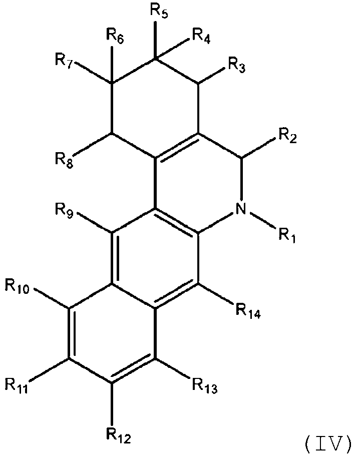 dans laquelle :
R₁ est H, OH ou un alkyle inférieur ;
R₂ est un cycle aromatique éventuellement substitué de quatre, cinq ou six carbones, dans lequel le cycle aromatique est carbocyclique ou hétérocyclique, et dans lequel chaque position sur le cycle aromatique est indépendamment occupée par H, un halo, un alkyle inférieur, OH, un alcoxy inférieur, NH₂, un alkylamino inférieur, un di(alkyle inférieur)amino, SH, un alkylthio inférieur, NO₂, ou deux résidus forment ensemble un cycle hétérocyclique ;
R₃ et R₈ sont chacun indépendamment H, un alkyle inférieur, =O, =S, OH, NH₂, un aryle ou un aralkyle, où l'aryle et l'aralkyle sont substitués par 0 à 3 groupements choisis dans le groupe constitué par un halo, OH, NH₂, un alkyle inférieur, un alcoxy inférieur, SH, un alkylthio inférieur et un alkylamino inférieur ;
R₄, R₅, R₆ et R₇ sont chacun indépendamment H, un alkyle inférieur, OH, NH₂, un aryle ou un aralkyle, où l'aryle et l'aralkyle sont substitués par 0 à 3 groupements choisis dans le groupe constitué par un halo, OH, NH₂, un alkyle inférieur, un alcoxy inférieur, SH, un alkylthio inférieur et un alkylamino inférieur ;
R₈, R₉, R₁₀, R₁₁, R₁₂, R₁₃, R₁₄ sont chacun indépendamment H, un halo, un alkyle inférieur, OH, un alcoxy inférieur ou NO₂ ; et
dans laquelle tout atome de carbone sur l'un quelconque des cycles condensés des formules (II), (III) ou (IV) est éventuellement remplacé par un atome d'azote.

4. Composition pharmaceutique pour une utilisation dans le traitement de la mucoviscidose ou d'une maladie neurodégénérative selon la revendication 3, dans laquelle le composé de formule (II) est ALT-212, ALT-215, ALT-308, ALT-309, ALT-408, ALT-411, ALT-59, ALT-110, ALT-201, ALT-202, ALT-204, ALT-208, ALT-207, ALT-210, ALT-211, ALT-302, ALT-306, ALT-307, ALT-311, ALT-318, ALT-322, ALT-324, ALT-402, ALT-404, ALT-406, ALT-409, ALT-410, ALT-413 ou ALT-414 ; ou
dans laquelle le composé de formule (III) est ALT-108, ALT-317, ALT-333 ou ALT-403 ; ou
dans laquelle le composé de formule (IV) est ALT-205.

5. Composition pharmaceutique pour une utilisation dans le traitement de la mucoviscidose ou d'une maladie neurodégénérative selon la revendication 1, dans laquelle le composé de formule (I) est un composé de formule (V) : 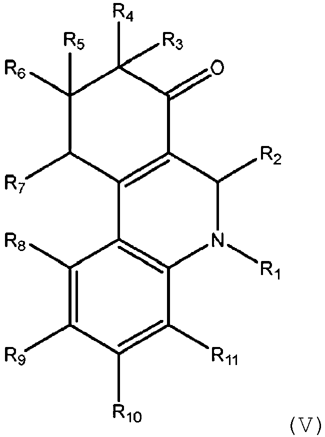 dans laquelle :
R₁ est H, OH ou un alkyle inférieur ;
R₂ est un cycle aromatique éventuellement substitué de quatre, cinq ou six carbones, dans lequel le cycle aromatique est carbocyclique ou hétérocyclique, et dans lequel chaque position sur le cycle aromatique est indépendamment occupée par H, un halo, un alkyle inférieur, OH, un alcoxy inférieur, NH₂, un alkylamino inférieur, un di(alkyle inférieur)amino, SH, un alkylthio inférieur, NO₂, ou deux résidus forment ensemble un cycle hétérocyclique ;
R₃, R₄, R₅, R₆, et R₇ sont chacun indépendamment H, un alkyle inférieur, OH, NH₂, un aryle ou un aralkyle, où l'aryle et l'aralkyle sont substitués par 0 à 3 groupements choisis dans le groupe constitué par un halo, OH, NH₂, un alkyle inférieur, un alcoxy inférieur, SH, un alkylthio inférieur et un alkylamino inférieur ;
au moins un couple parmi R₈ et R₉, R₉ et R₁₀, ou R₁₀ et R₁₁ forme ensemble un cycle benzénique éventuellement substitué, dans lequel le cycle benzénique est éventuellement substitué par H, un halo, un alkyle inférieur, OH, un alcoxy inférieur ou NO₂ ; et
dans laquelle tout atome de carbone sur l'un quelconque des cycles condensés de la formule (V) est éventuellement remplacé par un atome d'azote.

6. Composition pharmaceutique pour une utilisation dans le traitement de la mucoviscidose ou d'une maladie neurodégénérative selon la revendication 5, dans laquelle le composé de formule (V) est un composé de l'une quelconque des formules (VI), (VII) ou (VIII) : 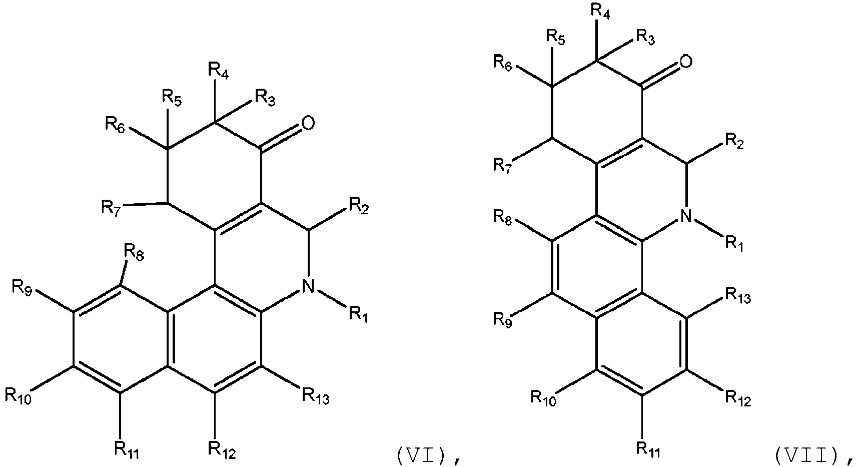 ou 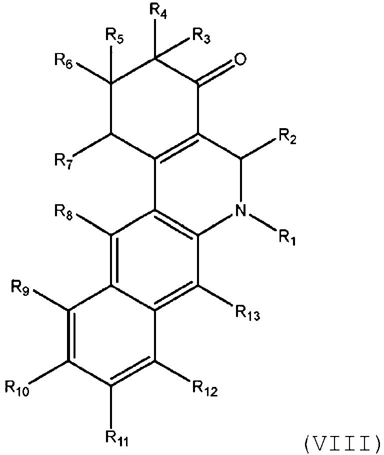 dans laquelle :
R₁ est H, OH ou un alkyle inférieur ;
R₂ est un cycle aromatique éventuellement substitué de quatre, cinq ou six carbones, dans lequel le cycle aromatique est carbocyclique ou hétérocyclique, et dans lequel chaque position sur le cycle aromatique est indépendamment occupée par H, un halo, un alkyle inférieur, OH, un alcoxy inférieur, NH₂, un alkylamino inférieur, un di(alkyle inférieur)amino, SH, un alkylthio inférieur, NO₂, ou deux résidus forment ensemble un cycle hétérocyclique ;
R₃, R₄, R₅, R₆, et R₇ sont chacun indépendamment H, un alkyle inférieur, OH, NH₂, un aryle ou un aralkyle, où l'aryle et l'aralkyle sont substitués par 0 à 3 groupements choisis dans le groupe constitué par un halo, OH, NH₂, un alkyle inférieur, un alcoxy inférieur, SH, un alkylthio inférieur et un alkylamino inférieur ;
R₈, R₉, R₁₀, R₁₁, R₁₂, et R₁₃ sont chacun indépendamment H, un halo, un alkyle inférieur, OH, un alcoxy inférieur ou NO₂ ; et
dans laquelle tout atome de carbone sur l'un quelconque des cycles condensés des formules (VI), (VII) ou (VIII) est éventuellement remplacé par un atome d'azote.

7. Composition pharmaceutique pour une utilisation dans le traitement de la mucoviscidose ou d'une maladie neurodégénérative selon la revendication 6, dans laquelle le composé de formule (VI) est ALT-212, ALT-215, ALT-308, ALT-309, ALT-408, ALT-411, ALT-59, ALT-110, ALT-202, ALT-204, ALT-208, ALT-207, ALT-210, ALT-211, ALT-302, ALT-306, ALT-307, ALT-311, ALT-318, ALT-322, ALT-324, ALT-402, ALT-404, ALT-406, ALT-409, ALT-410, ALT-413 ou ALT-414 ; ou
dans laquelle le composé de formule (VII) est ALT-108, ALT-317, ALT-333 ou ALT-403 ; ou
dans laquelle le composé de formule (VIII) est ALT-205.

8. Composition pharmaceutique pour une utilisation dans le traitement de la mucoviscidose ou d'une maladie neurodégénérative selon l'une quelconque des revendications 1 à 7, dans laquelle la composition inhibe la progression ou le développement de la mucoviscidose.

9. Composition pharmaceutique pour une utilisation dans le traitement de la mucoviscidose ou d'une maladie neurodégénérative selon l'une quelconque des revendications 1 à 8, dans laquelle la composition inhibe la progression ou le développement de la maladie neurodégénérative ; éventuellement, dans laquelle la maladie neurodégénérative est la sclérose latérale amyotrophique (SLA), la démence frontotemporale (DFT), la maladie d'Alzheimer, la sclérose hippocampique liée à l'âge (HS-Aging), l'encéphalopathie traumatique chronique ou la maladie de Parkinson.

10. Composition pharmaceutique pour une utilisation dans le traitement de la mucoviscidose ou d'une maladie neurodégénérative selon l'une quelconque des revendications 1 à 9, dans laquelle le composé de formule (I) est présent en une quantité de 0,01 mg à 3 000 mg.

11. Composition pharmaceutique pour une utilisation dans le traitement de la mucoviscidose ou d'une maladie neurodégénérative selon l'une quelconque des revendications 1 à 10, dans laquelle la composition est formulée pour une administration orale ou parentérale.

12. Composition pharmaceutique pour une utilisation dans le traitement de la mucoviscidose ou d'une maladie neurodégénérative selon l'une quelconque des revendications 1 à 11, comprenant en outre un support ou un excipient pharmaceutiquement acceptable.

13. Composition pharmaceutique pour une utilisation dans le traitement de la mucoviscidose ou d'une maladie neurodégénérative selon la revendication 4, dans laquelle le composé de formule (II) est ALT-302, ALT-306, ALT-308 ou ALT-309.

14. Composition pharmaceutique pour une utilisation dans le traitement de la mucoviscidose ou d'une maladie neurodégénérative selon la revendication 4, dans laquelle le composé de formule (II) est ALT-302.

15. Composition pharmaceutique pour une utilisation dans le traitement de la mucoviscidose ou d'une maladie neurodégénérative selon la revendication 7, dans laquelle le composé de formule (VI) est ALT-059, ALT-302, ALT-306, ALT-308 ou ALT-309.
